# EUROPEAN PATENT SPECIFICATION

(11) **EP 1 786 792 B1**
(45) Date of publication and mention of the grant of the patent: **08.04.2009**
(21) Application number: 05768525.7
(22) Date of filing: 27.07.2005
(51) Int. Cl.: C07D 277/00

(54) **THIAZOLE DERIVATIVES HAVING VAP-1 INHIBITORY ACTIVITY**
THIAZOLDERIVATE MIT VAP-1-HEMMENDER WIRKUNG
DERIVES DE THIAZOLE PRESENTANT UNE ACTIVITE D'INHIBITION DE LA VAP-1

(30) Priority: 27.07.2004 AU 2004904196
(43) Date of publication of application: 23.05.2007
(73) Proprietor: Astellas Pharma Inc., Tokyo 103-8411 (JP)
(72) Inventor: INOUE, Takayuki, 2-chome, Chuo-ku, Tokyo 1038411; (JP); TOJO, Takashi, 2-chome, Chuo-ku, Tokyo 1038411; (JP); MORITA, Masataka, 2-chome, Chuo-ku, Tokyo 1038411; (JP)
(74) Representative: Gille Hrabal Struck Neidlein Prop Roos
(86) International application number: PCT/JP2005/014136
(87) International publication number: WO 2006/011631

(56) References cited:
- WO-A-96/30350
- WO-A-20/04067521
- WO-A-20/04087138
- US-A- 4 511 574
- MATTAMMAL M B ET AL: "MASS SPECTROMETRY OF 2,4-SUBSTITUTED CARCINOGENIC THIAZOLES AND THEIR METABOLITES" September 1985 (1985-09), JOURNAL OF HETEROCYCLIC CHEMISTRY, HETEROCORPORATION. PROVO, US, PAGE(S) 1157-1163 , XP009010468 ISSN: 0022-152X the whole document compound 8

## Description

### TECHNICAL FIELD

The present invention relates to a compound or a pharmaceutically acceptable salt thereof useful as a vascular adhesion protein-1 inhibitor, a pharmaceutical composition comprising the compound or salt thereof as an active ingredient, a method for preventing or treating a vascular adhesion protein-1 associated disease, especially macular edema, use of the compound, salt thereof or composition, and the like.

### BACKGROUND ART

Vascular adhesion protein-1 (hereinafter to be abbreviated as VAP-1) is an amine oxidase (semicarbazide sensitive amine oxidase, SSAO) which is abundant in human plasma, and shows remarkably increased expression in vascular endothelium and vascular smooth muscle of the inflammatory region. While the physiological role of VAP-1 has not been clarified until recently, VAP-1 gene was cloned in 1998, and VAP-1 has been reported to be a membrane protein that regulates rolling and migration of lymphocyte and NK cell as an adhesion molecule under regulation of expression by inflammatory cytokine. Although the amine to be a substrate is unknown, it is considered to be methylamine generated in any part of living organisms. It is also known that hydrogen peroxide and aldehydes produced due to the amine oxidase activity in the molecule are important factors of adhesion activity.

A recent report has documented that VAP-1 enzyme activity in plasma increases in diabetic patients, whether type I or type II, and the increase is particularly remarkable in diabetic patients suffering from retinopathy complications (Diabetologia, 42 (1999) 233-237 and Diabetic Medicine, 16 (1999) 514-521).

In addition, it has been reported that VAP-1 is associated with the following diseases:
(1) cirrhosis, essential stabilized hypertension, diabetes, arthrosis (see JP-A-61-239891 and USP 4,888,283);
(2) endothelium damage (in diabetes, atherosclerosis and hypertension), a cardiovascular disorder associated with diabetes and uremia, pain associated with gout and arthritis, retinopathy (in diabetes patients) (see WO 93/23023);
(3) an (connective tissue) inflammatory disease or condition (rheumatoid arthritis, ankylosing spondylitis, psoriatic arthritis and osteoarthritis or degenerative joint disease, Reiter's syndrome, Sjögren's syndrome, Behcet's syndrome, relapsing polychondritis, systemic lupus erythematosus, discoid lupus erythematosus, systemic sclerosis, eosinophilic fasciitis, polymyositis, dermatomyositis, polymyalgia rheumatica, vasculitis, temporal arteritis, polyarteritis nodosa, Wegener's granulomatosis, mixed connective tissue disease, and juvenile rheumatoid arthritis); a gastrointestinal inflammatory disease or condition [Crohn's disease, ulcerative colitis, irritable bowel syndrome (spastic colon), fibrotic conditions of the liver, inflammation of the oral mucosa (stomatitis), and recurrent aphtous stomatitis]; a central nervous system inflammatory disease or condition (multiple sclerosis, Alzheimer's disease, and ischemia-reperfusion injury associated with ischemic stroke); a pulmonary inflammatory disease or condition (asthma, adult respiratory distress syndrome, and chronic obstructive pulmonary disease); a (chronic) skin inflammatory disease or condition (psoriasis, allergic lesions, lichen planus, pityriasis rosea, contact dermatitis, atopic dermatitis, and pityriasis rubra pilaris); a disease related to carbohydrate metabolism (diabetes and complications from diabetes) including microvascular and macrovascular disease (atherosclerosis, vascular retinopathies, retinopathy, nephropathy, nephrotic syndrome and neuropathy (polyneuropathy, mononeuropathies and autonomic neuropathy), foot ulcers, joint problems, and increased risk of infection); a disease related to aberrations in adipocyte differentiation or function or smooth muscle cell function (atherosclerosis and obesity); a vascular disease [atheromatous ateriosclerosis, nonatheromatous ateriosclerosis, ischemic heart disease including myocardial infarction and peripheral arterial occlusion, Raynaud's disease and phenomenon, and thromboangiitis obliterans (Buerger's disease)]; chronic arthritis; inflammatory bowel diseases; skin dermatoses (see WO 02/02090, WO 02/02541 and US patent application publication No. 2002/0173521 A1);
(4) diabetes mellitus (see WO 02/38152);
(5) SSAO-mediated complication [diabetes (insulin dependent diabetes mellitus (IDDM) and non-insulin dependent diabetes mellitus (NIDDM)) and vascular complication (heart attack, angina, strokes, amputations, blindness and renal failure)] (see WO 02/38153);
(6) hepatitis, transplantation, and the like.

Under the present circumstances, a drug treatment or prophylaxis of the above diseases has been demanded.

In addition, macular edema is a common ocular abnormality resulting from a vast etiology and characterized by perturbation of the integrity of the blood-retinal barrier of the perifoveal capillaries and the optic nerve head. Macular edema is known to include diabetic and non-diabetic macular edema. Macular edema as a diabetic complication is a disease state that can occur in any stage of diabetic retinopathy, emerges before the onset of neovascularization and causes serious visual disorders.

Macular area is a highly evolved part in retina and plays a key role in controlling the eyesight. Once the macular area suffers from edema, how mild the change may be, it causes a significant failure of eyesight, and when left unattended, the edema causes irreversible changes of macular tissue, and it is considered to encourage progress of retinopathy.

At present, for macular edema, laser beam photocoagulation and vitreous surgery have been tried as a symptomatic therapy. However, irradiation of laser on the macular area is not easy and unnecessary laser treatments may produce side effects (e.g., possible encouragement of edema by causing inflammation). The vitreous surgery is considered to provide efficacy in 70 percent of macular edema, but physical and economical burden on patients is high, and the incidence of recurrence is also high. These treatment methods are not usually employed in the initial stage of macular edema, particularly so in the stages when the decrease of vision is comparatively small. Accordingly, a drug treatment comparatively easily applicable from the early stages of the disease has been also demanded under the present circumstances.

Compounds which may be used for the treatment of macular edema are described in WO 2004-067521. The document which is only relevant for novelty under Art. 54(3) EPC discloses compounds which are different from those of the present invention. The same compounds are described for the treatment of vascular hyperpermeable diseases except macular edema in WO 2004-087138.

Products which are inhibitors of the production of nitric oxide are described in WO 96-30350.

### DISCLOSURE OF INVENTION

The present inventors have intensively worked on the problem of the drug treatment of a VAP-1 associated disease and found that a VAP-1 inhibitor is useful for the prophylaxis or treatment of the disease, particularly macular edema, and completed the present invention. Thus, the present invention provides the following.
[1] A compound of the formula (I), (II), (III) or (IV) [hereinafter sometimes referred to as Compound (I), (II), (III) or (IV), or VAP-1 inhibitor]: 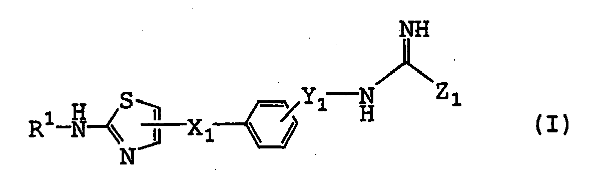 wherein
   R¹ is alkylcarbonyl;
   X₁ is a bond or lower alkylene;
   Y₁ is a bond, lower alkylene, -CH₂-CO-, -CH₂-CH₂-CO-, -CH₂-CH₂-CO-CH₂- or -NH-CH₂-CH₂-; and
   Z₁ is -NH₂, -NH(lower alkyl) or lower alkyl;
   provided that
   when X₁ is ethylene, then Y₁ should be C₂-C₆ alkylene, -CH₂-CO-, -CH₂-CH₂-CO-, -CH₂-CH₂-CO-CH₂- or -NH-CH₂-CH₂-,
   when X₁ is a bond, then Y₁ should be a bond, methylene, C₃-C₆ alkylene, -CH₂-CO-, -CH₂-CH₂-CO-, -CH₂-CH₂-CO-CH₂- or -NH-CH₂-CH₂-, and
   when R¹ is acetyl, X₁ is ethylene, Y₁ is ethylene and Z₁ is -NH₂, then Y₁ should be attached to *ortho* or *meta* position of the phenyl group; 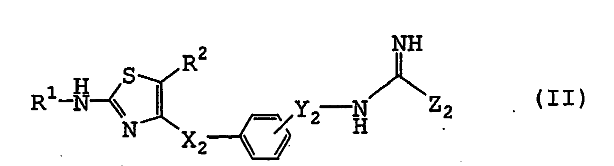 wherein
   R¹ is alkylcarbonyl;
   R² is 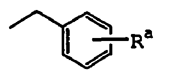 wherein R^{a} is (lower alkyl)sulfonyl, aminosulfonyl or di(lower alkyl)aminosulfonyl, 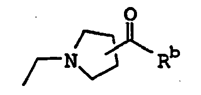 wherein R^{b} is mono- or di-(lower alkyl)amino, 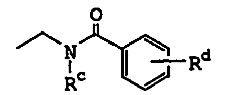 wherein R^{c} is lower alkyl and R^{d} is (lower alkyl)sulfonyl, di(lower alkyl)aminocarbonyl, alkylcarbonyl or nitro, or -CH=CH-CO-di(lower alkyl)amino;
   X₂ is a bond or lower alkylene;
   Y₂ is a bond, lower alkylene, -CH₂-CO- or -NH-CO-CH₂-; and
   Z₂ is -NH₂;
   provided that
   when R¹ is acetyl, X₂ is ethylene, Y₂ is a bond and Z₂ is -NH₂, then R² should not be 3-(methanesulfonyl)benzyl, 4-(methanesulfonyl)benzyl, 4-(ethanesulfonyl)benzyl and 2-(dimethylaminocarbonyl)pyrrolidin-1-ylmethyl; 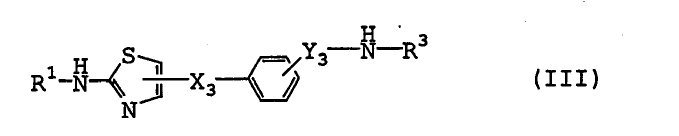 wherein
   R¹ is alkylcarbonyl:
   R³ is 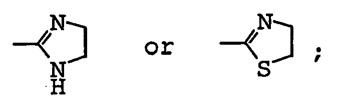 X₃ is lower alkylene; and
   Y₃ is lower alkylene; 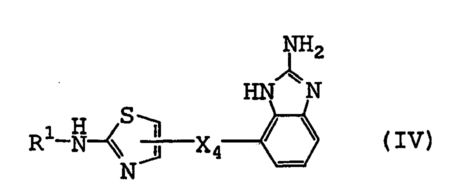 wherein
   R¹ is alkylcarbonyl; and
   X₄ is lower alkylene;
   or a pharmaceutically acceptable salt thereof.
[2] The compound of [1], wherein R¹ is acetyl, or a pharmaceutically acceptable salt thereof.
[3] The compound of [1], wherein Z₁ is -NH₂, or a pharmaceutically acceptable salt thereof.
[4] The compound of [1], wherein the compound is N-{4-[2-(4-{[amino(imino)methyl]amino}phenyl)ethyl]-5-[4-(aminosulfonyl)benzyl]-1,3-thiazol-2-yl}acetamide, N-{4-[4-(4-{[amino(imino)methyl]amino}butyl)phenyl]-1,3-thiazol-2-yl}acetamide, 2-(4-{2-[2-(acetylamino)-1,3-thiazol-4-yl]ethyl}phenyl)-N-[amino(imino)methyl]acetamide, (3R)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, (3S)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, N-({2-(acetylamino)-4-[2-(4-{[amino(imino)methyl]amino}-phenyl)ethyl]-1,3-thiazol-5-yl}methyl)-N-methyl-4-(methylsulfonyl)benzamide, or N-({2-(acetylamino)-4-[2-(4-{[amino(imino)methyl]amino}-phenyl)ethyl]-1,3-thiazol-5-yl}methyl)-N-methyl-4-nitrobenzamide,
   or a pharmaceutically acceptable salt thereof.
[5] The compound of [1] or a pharmaceutically acceptable salt thereof for use as a medicament.
[6] A pharmaceutical composition, which comprises, as an active ingredient, the compound of [1] or a pharmaceutically acceptable salt thereof.
[7] A use of the compound of [1] or a pharmaceutically acceptable salt thereof for preparing a medicament as a VAP-1 inhibitor.
[8] The use of [7], wherein the compound is N-{4-[2-(4-{[amino(imino)methyl]amino}phenyl)ethyl]-5-[4-(aminosulfonyl)benzyl]-1,3-thiazol-2-yl}acetamide, N-{4-[4-(4-{[amino(imino)methyl]amino}butyl)phenyl]-1,3-thiazol-2-yl}acetamide, 2-(4-{2-[2-(acetylamino)-1,3-thiazol-4-yl]ethyl}phenyl)-N-[amino(imino)methyl]acetamide, (3R)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, (3S)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, N-({2-(acetylamino)-4-[2-(4-{ [amino(imino)methyl]amino}-phenyl)ethyl]-1,3-thiazol-5-yl}methyl)-N-methyl-4-(methylsulfonyl)benzamide, or N-({2-(acetylamino)-4-[2-(4-{[amino(imino)methyl]amino}-phenyl)ethyl]-1,3-thiazol-5-yl}methyl)-N-methyl-4-nitrobenzamide, or a pharmaceutically acceptable salt thereof.
[9] A use of the compound of [1] or a pharmaceutically acceptable salt thereof for preparing a medicament for the prophylaxis or treatment of a VAP-1 associated disease.
[10] The use of [9], wherein said VAP-1 associated disease is selected from the group consisting of cirrhosis, essential stabilized hypertension, diabetes, arthrosis, endothelium damage (in diabetes, atherosclerosis and hypertension), a cardiovascular disorder associated with diabetes and uremia, pain associated with gout and arthritis, retinopathy (in diabetes patients), an (connective tissue) inflammatory disease or condition (rheumatoid arthritis, ankylosing spondylitis, psoriatic arthritis and osteoarthritis or degenerative joint disease, Reiter's syndrome, Sjogren's syndrome, Behçet's syndrome, relapsing polychondritis, systemic lupus erythematosus, discoid lupus erythematosus, systemic sclerosis, eosinophilic fasciitis, polymyositis, dermatomyositis, polymyalgia rheumatica, vasculitis, temporal arteritis, polyarteritis nodosa, Wegener's granulomatosis, mixed connective tissue disease, and juvenile rheumatoid arthritis), a gastrointestinal inflammatory disease or condition [Crohn's disease, ulcerative colitis, irritable bowel syndrome (spastic colon), fibrotic conditions of the liver, inflammation of the oral mucosa (stomatitis), and recurrent aphtous stomatitis], a central nervous system inflammatory disease or condition (multiple sclerosis, Alzheimer's disease, and ischemia-reperfusion injury associated with ischemic stroke), a pulmonary inflammatory disease or condition (asthma, adult respiratory distress syndrome, and chronic obstructive pulmonary disease), a (chronic) skin inflammatory disease or condition (psoriasis, allergic lesions, lichen planus, pityriasis rosea, contact dermatitis, atopic dermatitis, and pityriasis rubra pilaris), a disease related to carbohydrate metabolism (diabetes and complications from diabetes) including microvascular and macrovascular disease (atherosclerosis, vascular retinopathies, retinopathy, nephropathy, nephrotic syndrome and neuropathy (polyneuropathy, mononeuropathies and autonomic neuropathy), foot ulcers, joint problems, and increased risk of infection), a disease related to aberrations in adipocyte differentiation or function or smooth muscle cell function (atherosclerosis and obesity), a vascular disease [atheromatous ateriosclerosis, nonatheromatous ateriosclerosis, ischemic heart disease including myocardial infarction and peripheral arterial occlusion, Raynaud's disease and phenomenon, and thromboangiitis obliterans (Buerger's disease)], chronic arthritis, inflammatory bowel diseases, skin dermatoses, diabetes mellitus, SSAO-mediated complication [diabetes (insulin dependent diabetes mellitus (IDDM) and non-insulin dependent diabetes mellitus (NIDDM)) and vascular complication (heart attack, angina, strokes, amputations, blindness and renal failure)], macular edema (diabetic and non-diabetic macular edema), hepatitis and transplantation.
[11] The use of [10], wherein said VAP-1 associated disease is macular edema.
[12] The use of [11], wherein said macular edema is diabetic macular edema.
[13] The use of [11], wherein said macular edema is non-diabetic macular edema.
[14] A VAP-1 inhibitor, which comprises the compound of [1] or a pharmaceutically acceptable salt thereof.
[15] A method for preventing or treating macular edema, which method comprises administering to a subject in need thereof a VAP-1 inhibitor in an amount sufficient to treat said subject for macular edema.
[16] The method of [15], wherein the VAP-1 inhibitor is N-{4-[2-(4-{[amino(imino)methyl]amino}phenyl)ethyl]-5-[4-(aminosulfonyl)benzyl]-1,3-thiazol-2-yl}acetamide, N-{4-[4-(4-{[amino(imino)methyl]amino}butyl)phenyl]-1,3-thiazol-2-yl}acetamide, 2-(4-{2-[2-(acetylamino)-1,3-thiazol-4-yl]ethyl}phenyl)-N-[amino(imino)methyl]acetamide, (3R)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, (3S)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, N-({2-(acetylamino)-4-[2-(4-{[amino(imino)methyl]amino}-phenyl)ethyl]-1,3-thiazol-5-yl}methyl)-N-methyl-4-(methylsulfonyl)benzamide, or N-({2-(acetylamino)-4-[2-(4-{[amino(imino)methyl]amino}-phenyl)ethyl]-1,3-thiazol-5-yl}methyl)-N-methyl-4-nitrobenzamide,
   or a pharmaceutically acceptable salt thereof.
[17] A method for preventing or treating a VAP-1 associated disease, which method comprises administering an effective amount of the compound of [1] or a pharmaceutically acceptable salt thereof to a mammal.
[18] The method of [17], wherein said VAP-1 associated disease is selected from the group consisting of cirrhosis, essential stabilized hypertension, diabetes, arthrosis, endothelium damage (in diabetes, atherosclerosis and hypertension), a cardiovascular disorder associated with diabetes and uremia, pain associated with gout and arthritis, retinopathy (in diabetes patients), an (connective tissue) inflammatory disease or condition (rheumatoid arthritis, ankylosing spondylitis, psoriatic arthritis and osteoarthritis or degenerative joint disease, Reiter's syndrome, Sjögren's syndrome, Behçet's syndrome, relapsing polychondritis, systemic lupus erythematosus, discoid lupus erythematosus, systemic sclerosis, eosinophilic fasciitis, polymyositis, dermatomyositis, polymyalgia rheumatica, vasculitis, temporal arteritis, polyarteritis nodosa, Wegener's granulomatosis, mixed connective tissue disease, and juvenile rheumatoid arthritis), a gastrointestinal inflammatory disease or condition [Crohn's disease, ulcerative colitis, irritable bowel syndrome (spastic colon), fibrotic conditions of the liver, inflammation of the oral mucosa (stomatitis), and recurrent aphtous stomatitis], a.central nervous system inflammatory disease or condition (multiple sclerosis, Alzheimer's disease, and ischemia-reperfusion injury associated with ischemic stroke), a pulmonary inflammatory disease or condition (asthma, adult respiratory distress syndrome, and chronic obstructive pulmonary disease), a (chronic) skin inflammatory disease or condition (psoriasis, allergic lesions, lichen planus, pityriasis rosea, contact dermatitis, atopic dermatitis, and pityriasis rubra pilaris), a disease related to carbohydrate metabolism (diabetes and complications from diabetes) including microvascular and macrovascular disease (atherosclerosis, vascular retinopathies, retinopathy, nephropathy, nephrotic syndrome and neuropathy (polyneuropathy, mononeuropathies and autonomic neuropathy), foot ulcers, joint problems, and increased risk of infection), a disease related to aberrations in adipocyte differentiation or function or smooth muscle cell function (atherosclerosis and obesity), a vascular disease [atheromatous ateriosclerosis, nonatheromatous ateriosclerosis, ischemic heart disease including myocardial infarction and peripheral arterial occlusion, Raynaud's disease and phenomenon, and thromboangiitis obliterans (Buerger's disease)], chronic arthritis, inflammatory bowel diseases, skin dermatoses, diabetes mellitus, SSAO-mediated complication [diabetes (insulin dependent diabetes mellitus (IDDM) and non-insulin dependent diabetes mellitus (NIDDM)) and vascular complication (heart attack, angina, strokes, amputations, blindness and renal failure)], macular edema (diabetic and non-diabetic macular edema), hepatitis and transplantation.
[19] The method of [18], wherein said VAP-1 associated disease is macular edema.
[20] The method of [19], wherein said macular edema is diabetic macular edema.
[21] The method of [19], wherein said macular edema is non-diabetic macular edema.

### DETAILED DESCRIPTION OF THE INVENTION

The present invention is predicated on the discovery that an inhibitor for vascular adhesion protein-1 (VAP-1; also referred to as semicarbazide sensitive amine oxidase (SSAO) or copper-containing amine oxidase) is effective in treating or ameliorating VAP-1 associated diseases, especially macular edema, and the like. Accordingly, the present invention provides Compounds (I), (II), (III) and (IV) and a pharmaceutically acceptable salt thereof useful as a VAP-1 inhibitor, a pharmaceutical composition, a method for preventing or treating a VAP-1 associated disease, and the like.

In the above and subsequent descriptions of the present specification, suitable examples and illustration of the various definitions to be included within the scope of the invention are explained in detail as follows.

Suitable "halogen" includes fluorine, chlorine, bromine and iodine.

The term "lower" is used to intend a group having 1 to 6, preferably 1 to 4, carbon atom(s), unless otherwise provided.

Suitable "lower alkyl" includes straight or branched alkyl having 1 to 6 carbon atom(s) (i.e., C₁-C₆ alkyl), such as methyl, ethyl, propyl, isopropyl, butyl, isobutyl, sec-butyl, tert-butyl, pentyl, tert-pentyl and hexyl, in which more preferred one is C₁-C₄ alkyl.

Suitable "lower alkylene" includes straight or branched alkylene having 1 to 6 carbon atom(s) (i.e., C₁-C₆ alkylene), such as methylene, ethylene, trimethylene, tetramethylene, propylene, ethylidene and propylidene, in which more preferred one is C₁-C₄ alkylene, still more preferred one is C₂-C₄ alkylene.

Suitable "alkylcarbonyl" includes alkylcarbonyl wherein the alkyl moiety has 1 to 6 carbon atom(s) [i.e. the alkyl moiety is C₁-C₆ alkyl of the above "lower alkyl"], such as acetyl, propionyl, butyryl, isobutyryl, valeryl, isovaleryl, pivaloyl, hexanoyl and heptanoyl, in which more preferred one is C₁-C₄ alkyl-carbonyl.

Suitable "-NH(lower alkyl)" includes an amino group substituted with the "lower alkyl" defined above (i.e., C₁-C₆ alkyl amino), such as methylamino, ethylamino, propylamino, isopropylamino, butylamino, isobutylamino, sec-butylamino, tert-butylamino, pentylamino, tert-pentylamino, hexylamino and the like.

Suitable "mono- or di-(lower alkyl)amino" includes an amino group substituted with 1 or 2 of the "lower alkyl" defined above (i.e., mono- or di-(C₁-C₆ alkyl)amino), such as methylamino, ethylamino, propylamino, isopropylamino, butylamino, isobutylamino, sec-butylamino, tert-butylamino, pentylamino, tert-pentylamino, hexylamino, dimethylamino, diethylamino, dipropylamino, diisopropylamino, dibutylamino, diisobutylamino, di(sec-butyl)amino, di(tert-butyl)amino, dipentylamino, di(tert-pentyl)amino, dihexylamino and the like. The lower alkyls may be same or different.

Suitable "(lower alkyl)sulfonyl" includes a sulfonyl group having the "lower alkyl" defined above (i.e., (C₁-C₆ alkyl)sulfonyl), such as methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, tert-pentylsulfonyl, hexylsulfonyl and the like.

Suitable "di(lower alkyl)aminosulfonyl" includes a sulfonyl group having the "di(lower alkyl)amino" defined above (i.e., di(C₁-C₆ alkyl) aminosulfonyl), such as dimethylaminosulfonyl, diethylaminosulfonyl, dipropylaminosulfonyl, diisopropylaminosulfonyl, dibutylaminosulfonyl, diisobutylaminosulfonyl, di(sec-butyl)aminosulfonyl, di(tert-butyl)aminosulfonyl, dipentylaminosulfonyl, di(tert-pentyl)aminosulfonyl, dihexylaminosulfonyl and the like. The lower alkyls may be same or different.

Suitable "di(lower alkyl)aminocarbonyl" includes a carbonyl group having the "di(lower alkyl)amino" defined above (i.e., di(C₁-C₆ alkyl)aminocarbonyl), such as dimethylaminocarbonyl, diethylaminocarbonyl, dipropylaminocarbonyl, diisopropylaminocarbonyl, dibutylaminocarbonyl, diisobutylaminocarbonyl, di(sec-butyl)aminocarbonyl, di(tert-butyl)aminocarbonyl, dipentylaminocarbonyl, di(tert-pentyl)aminocarbonyl, dihexylaminocarbonyl and the like. The lower alkyls may be same or different.

Suitable "-CH=CH-CO-di(lower alkyl)amino" includes a carbonylvinyl group having the "di(lower alkyl)amino" defined above (i.e., -CH=CH-CO-di(C₁-C₆ alkyl)amino), such as (E) or (Z) dimethylaminocarbonylvinyl, diethylaminocarbonylvinyl, dipropylaminocarbonylvinyl, diisopropylaminocarbonylvinyl, dibutylaminocarbonylvinyl, diisobutylaminocarbonylvinyl, di(sec-butyl)aminocarbonylvinyl, di(tert-butyl)aminocarbonylvinyl, dipentylaminocarbonylvinyl, di(tert-pentyl)aminocarbonylvinyl, dihexylaminocarbonylvinyl and the like. The lower alkyls may be same or different.

In Compound (I), X₁ may be attached to 4- or 5-position of the thiazolyl group.

In Compound (I), Y₁ may be attached to *ortho, meta* or para position of the phenyl group.

In Compound (I), when X₁ is ethylene, then Y₁ should be C₂-C₆ alkylene, -CH₂-CO-, -CH₂-CH₂-CO-, -CH₂-CH₂-CO-CH₂- or -NH-CH₂-cH₂-, when X₁ is a bond, then Y₁ should be a bond, methylene, C₃-C₆ alkylene, -CH₂-CO-, -CH₂-CH₂-CO-, -CH₂-CH₂-CO-CH₂- or -NH-CH₂-CH₂-, and when R¹ is acetyl, X₁ is ethylene, Y₁ is ethylene and Z₁ is -NH₂, then Y₁ should be attached to *ortho* or *meta* position of the phenyl group.

In Compound (II), Y₂ may be attached to *ortho, meta* or *para* position of the phenyl group.

The substitution site of R^{a} on the phenyl is not particularly limited.

The substitution site of -COR^{b} on the pyrrolidinyl is not particularly limited.

The substitution site of R^{d} on the phenyl is not particularly limited.

In Compound (II), when R¹ is acetyl, X₂ is ethylene, Y₂ is a bond and Z₂ is -NH₂, then R² should not be 3-(methanesulfonyl)benzyl, 4-(methanesulfonyl)benzyl, 4-(ethanesulfonyl)benzyl and 2-(dimethylaminocarbonyl)-pyrrolidin-1-ylmethyl.

In Compound (III), X₃ may be attached to 4- or 5-position of the thiazolyl group.

In Compound (III), Y₃ may be attached to *ortho, meta* or *para* position of the phenyl group.

In Compound (IV), X₄ may be attached to 4- or 5-position of the thiazolyl group.

Any nitrogen atom in the amino (i.e. -NH₂), imino (i.e. =NH or -NH-) or the like in Compound (I), (II), (III) or (IV) may be protected according to the methods, which are known to those skilled in the art, such as the methods described in Protective Groups in Organic Synthesis, published by John Wiley and Sons (1980), and the like.

When Compound (I), (II), (III) or (IV) has an asymmetric carbon atom in the structure, those skilled in the art will recognize that Compound (I), (II), (III) or (IV) includes all stereoisomers.

When Compound (I), (II), (III) or (IV) has a double bond (i.e., >C=C<) in the structure, those skilled in the art will recognize that Compound (I), (II), (III) or (IV) includes E or Z isomer and mixture thereof.

The "vascular adhesion protein-1 (VAP-1) associated disease" comprise a disease selected from the group consisting of cirrhosis, essential stabilized hypertension, diabetes, arthrosis, endothelium damage (in diabetes, atherosclerosis and hypertension), a cardiovascular disorder associated with diabetes and uremia, pain associated with gout and arthritis, retinopathy (in diabetes patients), an (connective tissue) inflammatory disease or condition (rheumatoid arthritis, ankylosing spondylitis, psoriatic arthritis and osteoarthritis or degenerative joint disease, Reiter's syndrome, Sögren's syndrome, Behçet's syndrome, relapsing polychondritis, systemic lupus erythematosus, discoid lupus erythematosus, systemic sclerosis, eosinophilic fasciitis, polymyositis, dermatomyositis, polymyalgia rheumatica, vasculitis, temporal arteritis, polyarteritis nodosa, Wegener's granulomatosis, mixed connective tissue disease, and juvenile rheumatoid arthritis), a gastrointestinal inflammatory disease or condition [Crohn's disease, ulcerative colitis, irritable bowel syndrome (spastic colon), fibrotic conditions of the liver, inflammation of the oral mucosa (stomatitis), and recurrent aphtous stomatitis], a central nervous system inflammatory disease or condition (multiple sclerosis, Alzheimer's disease, and ischemia-reperfusion injury associated with ischemic stroke), a pulmonary inflammatory disease or condition (asthma, adult respiratory distress syndrome, and chronic obstructive pulmonary disease), a (chronic) skin inflammatory disease or condition (psoriasis, allegic lesions, lichen planus, pityriasis rosea, contact dermatitis, atopic dermatitis, and pityriasis rubra pilaris), a disease related to carbohydrate metabolism (diabetes and complications from diabetes) including microvascular and macrovascular disease (atherosclerosis, vascular retinopathies, retinopathy, nephropathy, nephrotic syndrome and neuropathy (polyneuropathy, mononeuropathies and autonomic neuropathy), foot ulcers, joint problems, and increased risk of infection), a disease related to aberrations in adipocyte differentiation or function or smooth muscle cell function (atherosclerosis and obesity), a vascular disease [atheromatous ateriosclerosis, nonatheromatous ateriosclerosis, ischemic heart disease including myocardial infarction and peripheral arterial occlusion, Raynaud's disease and phenomenon, and thromboangiitis obliterans (Buerger's disease)], chronic arthritis, inflammatory bowel diseases, skin dermatoses, diabetes mellitus, SSAO-mediated complication [diabetes (insulin dependent diabetes mellitus (IDDM) and non-insulin dependent diabetes mellitus (NIDDM)) and vascular complication (heart attack, angina, strokes, amputations, blindness and renal failure)], macular edema (e.g., diabetic and non-diabetic macular edema), hepatitis, transplantation and the like.

The "preventing or treating a vascular adhesion protein-1 (VAP-1) associated disease" and "prophylaxis or treatment of a vascular adhesion protein-1 (VAP-1) associated disease", particularly "preventing or treating macular edema" and "prophylaxis or treatment of macular edema" are in tended to include administration of a compound having VAP-1 inhibitory activity (i.e. VAP-1 inhibitor) to a subject for therapeutic purposes, which may include prophylaxis, amelioration, prevention and cure of the above described VAP-1 associated disease, particularly macular edema. As used herein, by the "subject" is meant a target of the administration of VAP-1 inhibitor in the present invention, which is specifically various animals such as mammal, e.g., human, mouse, rat, swine, dog, cat, horse, bovine and the like, especially human.

The therapeutic method comprises administration of a VAP-1 inhibitor in an amount sufficient to treat the VAP-1 associated disease, especially macular edema. Any VAP-1 inhibitor can be used in the method of the present invention as long as it is safe and effective. Herein, the "VAP-1 inhibitor" will be used to refer to such compounds/medicaments, which include Compound (I), (II), (III) or (IV), and is intended to encompass all compounds that inhibit enzyme activity of VAP-1 at any and all points in the action mechanism thereof. For example, the VAP-1 inhibitor used in the present invention may further include fluoroallylamine derivatives, semicarbazide derivatives, hydrazide derivatives, hydrazino derivatives, 1,3,4-oxadiazine derivatives, 4-alkyl-5-alkoxycarbonyl-4,5,6,7-tetrahydroimidazo[4,5-c]pyridine derivatives, 2,6-diethoxybenzylamine, 2,6-di(n-propoxy)benzylamine, 2,6-diisopropoxybenzylamine, 2,6-di(n-butoxy)benzylamine, 2,6-bis(methoxymethoxy)benzylamine, 2,6-bis(methoxymethyl)benzylamine, 2,6-diethylbenzylamine, 2,6-di-n-propylbenzylamine, 2,6-bis(2-hydroxyethoxy)benzylamine, and the like.

The above compounds can be exemplified as follows.
1) fluoroallylamine derivatives, semicarbazide derivatives and hydrazide derivatives described in WO 93/23023,
2) hydrazino derivatives described in WO 02/02090,
3) 1,3,4-oxadiazine derivatives described in WO 02/02541,
4) 4-alkyl-5-alkoxycarbonyl-4,5,6,7-tetrahydroimidazo[4,5-c]pyridine derivatives described in WO 02/38153,
5) 2,6-diethoxybenzylamine, 2,6-di(n-propoxy)benzylamine, 2,6-diisopropoxybenzylamine, 2,6-di(n-butoxy)benzylamine, 2,6-bis(methoxymethoxy)benzylamine, 2,6-bis(methoxymethyl)benzylamine, 2,6-diethylbenzylamine, 2,6-din-propylbenzylamine and 2,6-bis(2-hydroxyethoxy)benzylamine described in USP 4,888,283.

The compounds exemplified in the description of the present invention, in WO 93/23023 as an SSAO inhibitor, such as those described by Lyles et al. (Biochem. Pharmacol. 36:2847, 1987), and in USP 4,650,907, USP 4,916,151, USP 4,943,593, USP 4,965,288, USP 5,021,456, USP 5,059,714, USP 4,699,928, European patent application No. 295604, European patent application No. 224924 and European patent application No. 168013, are also encompassed in the VAP-1 inhibitor.

Of the above-mentioned compounds, preferred are Compound (I), (II), (III) and (IV), more preferably, N-{4-[2-(4-{[amino(imino)methyl]amino}phenyl)ethyl]-5-[4-(aminosulfonyl)benzyl]-1,3-thiazol-2-yl}acetamide (Production Example 3), N-{4-[4-(4-{[amino(imino)methyl]amino}butyl)phenyl]-1,3-thiazol-2-yl}acetamide (Production Example 7), 2-(4-{2-[2-(acetylamino)-1,3-thiazol-4-yl]ethyl}phenyl)-N-[amino(imino)methyl]acetamide (Production Example 9), (3R)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide (Production Example 12), (3S)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide (Production Example 14), N-({2-(acetylamino)-4-[2-(4-{[amino(imino)methyl]amino}-phenyl)ethyl]-1,3-thiazol-5-yl}methyl)-N-methyl-4-(methylsulfonyl)benzamide (Production Example 16), and N-({2-(acetylamino)-4-[2-(4-{[amino(imino)methyl]amino}-phenyl)ethyl]-1,3-thiazol-5-yl}methyl)-N-methyl-4-nitrobenzamide (Production Example 19), and derivatives thereof.

The term "derivative" is intended to include all compounds derived from the original compound.

In the present invention, the VAP-1 inhibitor can be administered as a prodrug to a subject. The term "prodrug" is intended to include all compounds that convert to the VAP-1 inhibitor in the body of the administration subject. The prodrug can be any pharmaceutically acceptable prodrug of VAP-1 inhibitor.

Moreover, the VAP-1 inhibitor can be administered to the administration subject as a pharmaceutically acceptable salt.

The pharmaceutically acceptable salt of the VAP-1 inhibitor is nontoxic and a pharmaceutically acceptable conventional salt, which is exemplified by salts with inorganic or organic base such as alkali metal salt (e.g., sodium salt, potassium salt and the like), alkaline earth metal salt (e.g., calcium salt, magnesium salt and the like), ammonium salt, and amine salt (e.g., triethylamine salt, N-benzyl-N-methylamine salt and the like).

In addition, the pharmaceutically acceptable salt of the VAP-1 inhibitor includes a pharmaceutically acceptable acid addition salt. Examples of the pharmaceutically acceptable acid addition salts include those derived from mineral acids, such as hydrochloric, hydrobromic, hydriodic, phosphoric, metaphosphoric, nitric and sulfuric acids, and organic acids, such as tartaric, acetic, citric, malic, lactic, fumaric, benzoic, glycolic, gluconic, succinic and arylsulfonic acids, for example, p-toluenesulfonic acid.

As a pharmaceutically acceptable salt of the VAP-1 inhibitor represented by the formula (I), (II), (III) or (IV), a pharmaceutically acceptable acid addition salt such as hydrochloride and hydriodide, particularly (mono-, di- or tri-)hydrochloride, is preferable.

Some VAP-1 inhibitors except Compound (I), (II), (III) and (IV) may be commercially available or can be produced based on known references.

Also, Compounds (I), (II), (III) and (IV) can be synthesized according to the following Production Method, Reference Example, Production Examples, the analogous methods thereto and the organic synthetic methods known to the art.

The VAP-1 inhibitor or a pharmaceutically acceptable salt thereof can be administered in accordance with the present inventive method via any suitable route. Suitable routes of administration include systemic, such as orally or by injection, topical, periocular (e.g., subTenon's), subconjunctival, intraocular, subretinal, suprachoroidal and retrobulbar administrations. The manner in which the VAP-1 inhibitor is administered is dependent, in part, upon whether the treatment of a VAP-1 associated disease is prophylactic or therapeutic.

The VAP-1 inhibitor is preferably administered as soon as possible after it has been determined that a subject such as mammal, specifically a human, is at risk for a VAP-1 associated disease (prophylactic treatments) or has begun to develop a VAP-1 associated disease (therapeutic treatments). Treatment will depend, in part, upon the particular VAP-1 inhibitor to be used, the amount of the VAP-1 inhibitor to be administered, the route of administration, and the cause and extent, if any, of a VAP-1 associated disease realized.

One skilled in the art will appreciate that suitable methods of administering a VAP-1 inhibitor, which is useful in the present inventive method, are available. Although more than one route can be used to administer a particular VAP-1 inhibitor, a particular route can provide a more immediate and more effective reaction than another route.

Accordingly, the described routes of administration are merely exemplary and are in no way limiting.

The dose of the VAP-1 inhibitor administered to the administration subject such as animal including human, particularly a human, in accordance with the present invention, should be sufficient to effect the desired response in the subject over a reasonable time frame. One skilled in the art will recognize that dosage will depend upon a variety of factors, including the strength of the particular VAP-1 inhibitor to be employed; the age, species, conditions or disease states, and body weight of the subject; and the degree of a VAP-1 associated disease. The size of the dose also will be determined depending on the route, timing and frequency of administration; the existence, nature and extent of any adverse side effects that might accompany the administration of a particular VAP-1 inhibitor; and the desired physiological effect. It will be appreciated by one of ordinary skill in the art that various conditions or disease states may require prolonged treatment involving multiple administrations.

Suitable doses and dosage regimens can be determined by conventional range-finding techniques known to those of ordinary skill in the art. Generally, treatment is initiated with smaller dosages, which are less than the optimum dose of the compound. Thereafter, the dosage is increased by small increments until the optimum effect under the circumstances is reached.

Generally, the VAP-1 inhibitor can be administered in the dose of from about 1 µg/kg/day to about 300 mg/kg/day, preferably from about 0.1 mg/kg/day to about 10 mg/kg/day, which is given in a single dose or 2 to 4 doses a day or in a sustained manner.

Pharmaceutical compositions for use in the present inventive method preferably comprise a "pharmaceutically acceptable carrier" and an amount of a VAP-1 inhibitor sufficient to treat a VAP-1 associated disease, especially macular edema, prophylactically or therapeutically as an active ingredient. The carrier can be any of those conventionally used and is limited only by chemico-physical considerations, such as the solubility and lack of the reactivity of the compound, and by the route of administration.

The VAP-1 inhibitor can be administered in various manners to achieve the desired VAP-1 inhibitory effect. The VAP-1 inhibitor can be administered alone or in combination with pharmaceutically acceptable carriers or diluents, the properties and nature of which are determined by the solubility and chemical properties of the inhibitor selected, the chosen administration route, and standard pharmaceutical practice. The VAP-1 inhibitor may be administered orally in solid dosage forms, e.g., capsules, tablets, powders, or in liquid forms, e.g., solutions or suspensions. The inhibitor may also be injected parenterally in the form of sterile solutions or suspensions. Solid oral forms may contain conventional excipients, for instance, lactose, sucrose, magnesium stearate, resins, and similar materials. Liquid oral forms may contain various flavoring, coloring, preserving, stabilizing, solubilizing or suspending agents. Parenteral preparations are sterile aqueous or non-aqueous solutions or suspensions which may contain certain various preserving, stabilizing, buffering, solubilizing or suspending agents. If desired, additives, such as saline or glucose, may be added to make the solutions isotonic.

The present inventive method also can involve the co-administration of other pharmaceutically active compound(s). By "co-administration" is meant administration of the other pharmaceutically active compound(s) before, concurrently with, e.g., in combination with a VAP-1 inhibitor in the same formulation or in separate formulations, or after administration of the VAP-1 inhibitor as described above.

For example, corticosteroids, prednisone, methylprednisolone, dexamethasone or.triamcinolone acetinide, or noncorticosteroid anti-inflammatory compounds, such as ibuprofen or flubiprofen, can be co-administered. Similarly, vitamins and minerals (e.g., zinc), anti-oxidants (e.g., carotenoids (such as a xanthophyll carotenoid like zeaxanthin or lutein)), and micronutrients can be co-administered.

In addition, the VAP-1 inhibitor according to the present invention is useful for preparing a medicament such as a therapeutic or prophylactic agent for the VAP-1 associated diseases.

Compounds (I), (II), (III) and (IV) can be synthesized according to the Production Method given below.

### Production Method

Compounds (I), (II), (III) and (IV) are prepared in accordance with, but is not limited to, the following procedures. Those skilled in the art will recognize that these procedures can be modified according to the conventional methods known *per* s*e.*

### Procedure A: Synthesis of Compounds (I) to (IV)

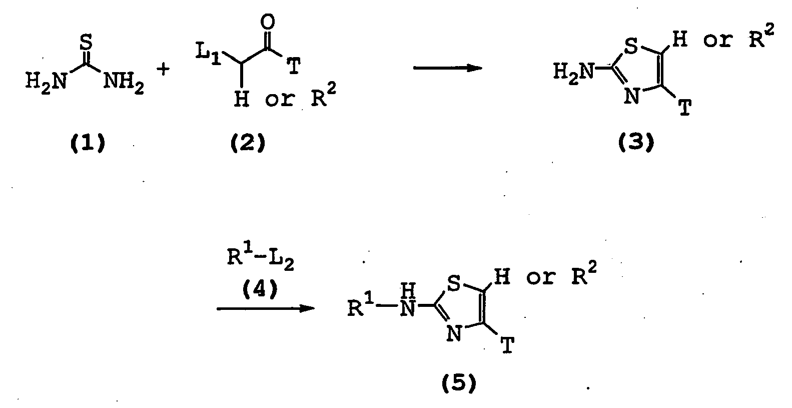

wherein
R¹ and R² are as defined above;
L₁ is a leaving group such as halogen (e.g., chlorine, bromine, iodine);
T is alkylcarbonyloxy(lower alkyl) wherein the alkylcarbonyl and the lower alkyl are defined above (e.g., acetyloxymethyl), 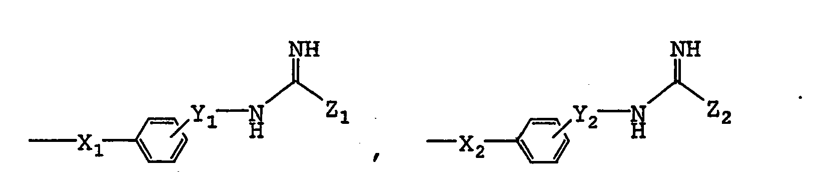 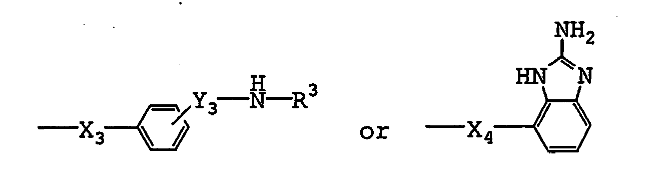 wherein R³, X₁, X₂, X₃, X₄, Y₁, Y₂, Y₃, Z₁ and Z₂ are as defined above; and
L₂ is a leaving group such as -OH, halogen (e.g., chlorine, bromine, iodine), -O-alkylcarbonyl wherein the alkylcarbonyl is as defined above (e.g., -O-acetyl and the like).

### Formation of Thiazole Moiety

Compound (1) is reacted with Compound (2) or its salt to give Compound (3).

Suitable salt of Compound (2) may be the same as those exemplified for Compound (I), (II), (III) or (IV).

Compounds (1) and (2) or its salt may be commercially available or can be prepared in accordance with the methods known *per se.*

The reaction is usually carried out in a conventional solvent such as ethanol, acetone, dichloromethane, acetic acid, and other organic solvent which does not adversely affect the reaction, or a mixture thereof.

The reaction temperature is not critical, and the reaction can be carried out under cooling to heating.

Compound (3) thus obtained can be isolated or purified by known separation or purification means, such as concentration, concentration *in vacuo,* solvent extraction, crystallization, recrystallization, phase transfer, chromatography and the like, and can be converted to a salt same as those exemplified for Compound (I), (II), (III) or (IV).

### Acylation

Compound (3) or its salt is reacted with Compound (4) to give Compound (5). Since R¹ is an alkylcarbonyl group, this reaction is an acylation.

The conventional acylation methods may be employed in the present invention.

Compound (4) may be commercially available or can be prepared in accordance with the methods known per *se.*

The reaction is usually carried out in a conventional solvent such as dichloromethane, chloroform, methanol, and other organic solvent which does not adversely affect the reaction, or a mixture thereof.

The reaction is also preferably carried out in the presence of a conventional base such as 4-dimethylaminopyridine, pyridine etc. A liquid base can be also used as the solvent.

The reaction temperature is not critical, and the reaction can be carried out under cooling to heating.

Compound (5) thus obtained can be isolated or purified by known separation or purification means, such as concentration, concentration *in vacuo,* solvent extraction, crystallization, recrystallization, phase transfer, chromatography and the like, and can be converted to a salt same as those exemplified for Compound (I), (II), (III) or (IV).

The acylation may be applied to Compound (1) in advance.

The nitrogen atom(s) in Compound (1), (2), (3) or (5) may be protected or deprotected, as necessary, in accordance with methods known per *se* such as the methods described in

Protective Groups in Organic Synthesis, published by John Wiley and Sons (1980), and the like.

### Procedure B: Synthesis of Compounds (I) to (IV) wherein X₁, X₂, X₃ and X₄ are lower alkylene such as ethylene (i.e. -CH₂-CH₂-), for example,

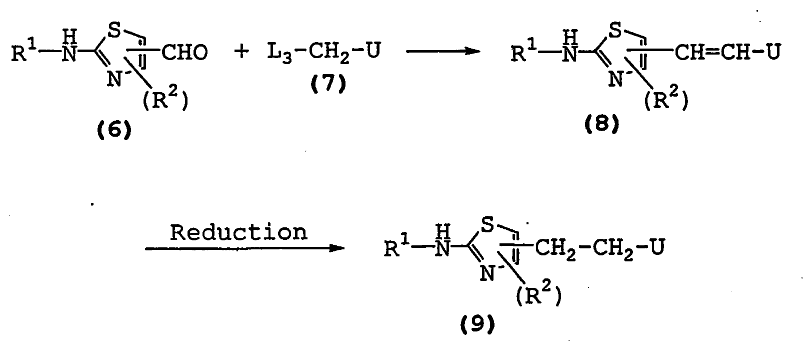

wherein
R¹ and R² are as defined above;
L₃ is a leaving group such as halogen (e.g., chlorine, bromine, iodine) and/or halogenotriphenylphosphinyl (e.g., BrPh₃P-); and
U is carboxy(lower alkyl)phenyl (e.g., carboxymethylphenyl), 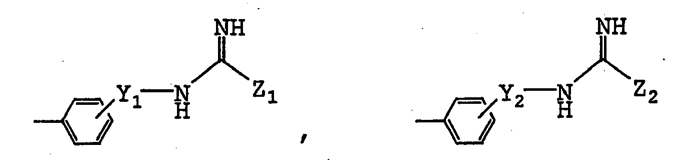 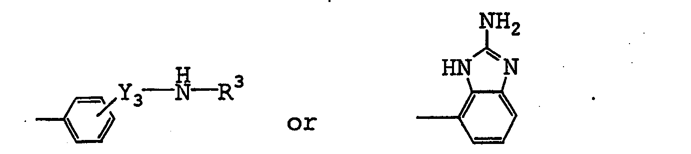 wherein R³, Y₁, Y₂, Y₃, Z₁ and Z₂ are as defined above.

### Formation of Olefin Compound

Compound (6) or its salt is reacted with Compound (7) or its salt to give an olefin compound (8).

Suitable salts of Compounds (6) and (7) may be the same as those exemplified for Compound (I), (II), (III) or (IV).

Compounds (6) and (7) or salts thereof may be commercially available or can be prepared in accordance with the methods known *per se.*

The reaction is usually carried out in a conventional solvent such as N,N-dimethylformamide, dimethylsulfoxide, tetrahydrofuran, dichloromethane, and other organic solvent which does not adversely affect the reaction, or a mixture thereof.

The reaction is also usually carried out in the presence of triphenylphosphine and/or a conventional base such as potassium tert-butoxide, sodium hydride, sodium hydroxide and the like.

The reaction temperature is not critical, and the reaction can be carried out under cooling to heating.

Compound (8) thus obtained can be isolated or purified by known separation or purification means, such as concentration, concentration in *vacuo,* solvent extraction, crystallization, recrystallization, phase transfer, chromatography and the like, and can be converted to a salt same as those exemplified for Compound (I), (II), (III) or (IV).

### Reduction

Compound (8) or its salt is reduced in accordance with a conventional method to give Compound (9).

The conventional reduction includes hydrogenation, catalytic hydrogenation, etc.

Among others, catalytic hydrogenation is preferable.

The catalytic hydrogenation is carried out in the presence of a catalyst such as palladium on carbon, preferably 10% palladium on carbon.

The catalytic hydrogenation is usually carried out in a conventional solvent such as tetrahydrofuran, methanol, ethanol, ethyl acetate, and other solvent which does not adversely affect the reaction, or a mixture thereof.

The catalytic hydrogenation is also preferably carried out in the presence of a conventional acid such as acetic acid, hydrochloric acid and the like. A liquid acid can be also used as the solvent.

The reaction temperature is not critical, and the reaction can be carried out under cooling to heating.

Compound (9) thus obtained can be isolated or purified by known separation or purification means, such as concentration, concentration *in vacuo,* solvent extraction, crystallization, recrystallization, phase transfer, chromatography and the like, and can be converted to a salt same as those exemplified for Compound (I), (II), (III) or (IV).

Therefore, as indicated in the following scheme, Compound (11) or a salt thereof can be prepared from Compound (10) or a salt thereof in a similar manner as described above. Suitable salts of Compounds (10) and (11) may be the same as those exemplified for Compound (I), (II), (III) or (IV). 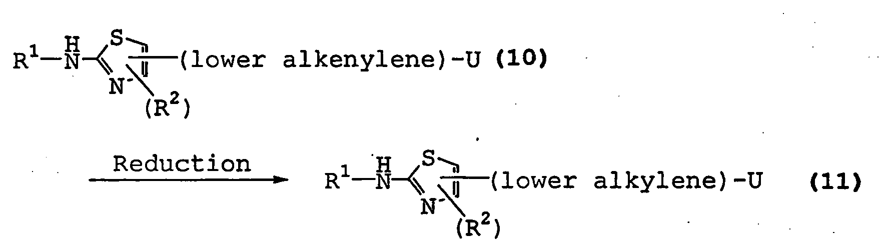 wherein R¹, R² and U are as defined above.
Suitable "lower alkenylene" includes straight or branched alkenylene having 2 to 6 carbon atom(s), wherein the position and the number of the double bond are not particularly limited, such as -CH=CH-, -CH₂-CH=CH-, -CH₂-CH=CH-CH₂-, -CH₂-CH₂-CH=CH-, -CH=CH-CH=CH-, -CH=CH-CH₂-CH₂-CH₂-, -CH=CH-CH=CH-CH₂-CH₂- and -CH=CH-CH=CH-CH=CH- etc.

The nitrogen atom(s) in Compound (6), (7), (8), (9), (10) or (11) may be protected or deprotected, as necessary, in accordance with methods known *per se* such as the methods described in Protective Groups in Organic Synthesis, published by John Wiley and Sons (1980), and the like.

The present invention is explained in more detail in the following by way of Reference Example, Production Examples and Example, which are not to be construed as limitative.

### Reference Example 1: Synthesis of N-{4-[2-(4-{[amino(imino)methyl]amino}phenyl)ethyl]-1,3-thiazol-2-yl}acetamide

### Step 1

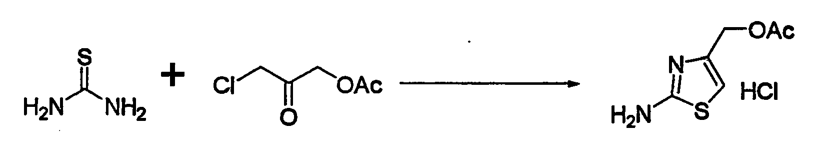

A mixture of 3-chloro-2-oxopropyl acetate (5 g) and thiourea (2.5 g) in ethanol (25 ml) was refluxed for 4 hours. The reaction mixture was cooled to ambient temperature and the resulting crystalline precipitate was collected by filtration and washed with ethanol (20 ml) to give (2-amino-1,3-thiazol-4-yl)methyl acetate hydrochloride (3.5 g) as white crystals.
¹H-NMR (DMSO-d₆), δ (ppm): 2.07(3H, s), 4.92(2H, s), 6.87(1H, s).
MS : 173(M+H)⁺

### Step 2

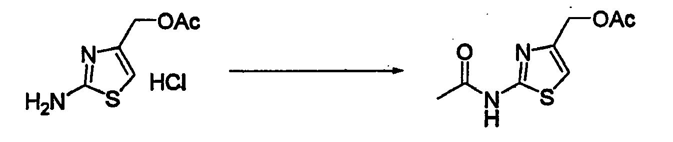

To a mixture of (2-amino-1,3-thiazol-4-yl)methyl acetate hydrochloride (56 g) and pyridine (45 g) in dichloromethane (560 ml) was added acetyl chloride (23 g) over a period of 30 minutes at 5°C, and the reaction mixture was stirred for 10 minutes at the same temperature. The reaction mixture was poured into water (500 ml) and extracted with chloroform (1 L). The organic layer was dried over sodium sulfate and concentrated in *vacuo.* The residual solid was collected by filtration with isopropyl ether to give (2-(acetylamino)-1,3-thiazol-4-yl)methyl acetate (47 g) as white crystals.
¹H-NMR (CDCl₃), δ (ppm): 2.12(3H, s), 2.29(3H, s), 5.08(2H, s), 6.93(1H, s).
MS : 215(M+H)⁺

### Step 3

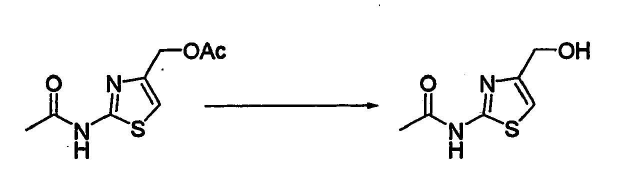

A mixture of (2-(acetylamino)-1,3-thiazol-4-yl)methyl acetate (46 g) and potassium carbonate (30 g) in methanol (640 ml) was stirred for 3 hours at ambient temperature. The reaction mixture was concentrated *in vacuo.* The residue was diluted with chloroform, and the insoluble material was filtered off. The resulting solution was purified by flash column chromatography on silica-gel with methanol / chloroform (1/99). The resulted solid was collected by filtration with isopropyl ether to give N-(4-(hydroxymethyl)-1,3-thiazol-2-yl)acetamide (35 g) as white crystals.
¹H-NMR (DMSO-d₆), δ (ppm): 2.12(3H, s), 4.44(2H, d, J=5.0Hz), 5.20(1H, t, J=5.0Hz), 6.88(1H, s), 12.02(1H, brs).
MS: 173(M+H)⁺

### Step 4

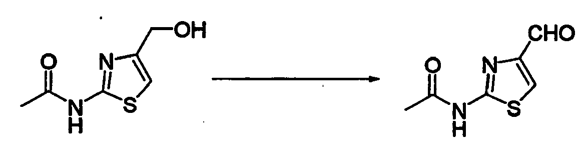

N-(4-(Hydroxymethyl)-1,3-thiazol-2-yl)acetamide (2.8 g) was dissolved in methanol (10 ml) and chloroform (200 ml). Then, manganese (IV) oxide (28.3 g) was added to the solution under nitrogen atmosphere. The reaction mixture was stirred at room temperature for 7 hours, and filtered through a celite pad. The filtrate was concentrated *in vacuo.* The resulting solid was washed with ethyl ether to give N-(4-formyl-1,3-thiazol-2-yl)acetamide (2.01 g) as an off-white solid.
mp. 195.5-199°C
¹H-NMR (DMSO-d₆), δ (ppm): 2.17(3H, s), 8.28(1H, s), 9.79(1H, s), 12.47(1H, brs).

### Step 5

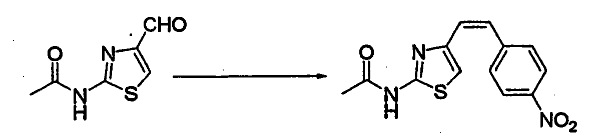

1-(Bromomethyl)-4-nitrobenzene (1.9 g), triphenylphosphine (2.31 g) and N,N-dimethylformamide (20 ml) were combined under nitrogen atmosphere. The reaction mixture was stirred at room temperature for 2.5 hours. Then, potassium tert-butoxide (1.19 g) and N-(4-formyl-1,3-thiazol-2-yl)acetamide (1.5 g) were added and the mixture was stirred at room temperature for 14 hours. The reaction mixture was poured into ice-water and extracted with ethyl acetate. The organic layer was washed with 1N-hydrochloric acid, water and saturated sodium chloride solution, dried over anhydrous magnesium sulfate, and concentrated *in vacuo.* The residue was purified by flash column chromatography over silica gel with n-hexane / ethyl acetate (1:1) → (1:2) as an eluent, and triturated with ethyl ether to give N-{4-[(Z)-2-(4-nitrophenyl)ethenyl]-1,3-thiazol-2-yl}acetamide (1.59 g) as a yellow solid.
mp. 155-157°C
¹H-NMR (DMSO-d₆), δ (ppm): 2.13(3H, s), 6.64(1H, d, J=12.5Hz), 6.71(1H, d, J=12.5Hz), 7.18(1H, s), 7.79(2H, d, J=9.0Hz), 8.17(2H, d, J=9.0Hz), 12.02(1H, brs).
MS: 290(M+H)⁺

### Step 6

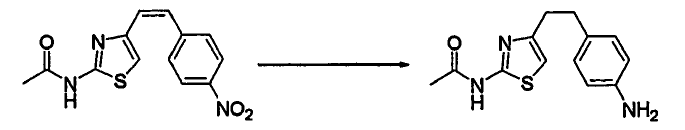

A mixture of N-{4-[(Z)-2-(4-nitrophenyl)ethenyl]-1,3-thiazol-2-yl}acetamide (2 g) and 10% palladium on carbon (400 mg) in methanol (25 ml), tetrahydrofuran (25 ml) and acetic acid (18 ml) was stirred under 4 atm hydrogen at ambient temperature for 5 hours. The reaction mixture was filtered through a celite pad, and the filtrate was concentrated *in vacuo.* The residue was dissolved in ethyl acetate. The organic solution was washed with saturated sodium hydrogen carbonate solution and saturated sodium chloride solution, dried over anhydrous magnesium sulfate, and concentrated *in vacuo.* The residue was purified by flash column chromatography over silica gel with n-hexane / ethyl acetate (1:2) → ethyl acetate as an eluent, and triturated with ethyl alcohol / ethyl ether to give N-(4-(2-(4-aminophenyl)ethyl)-1,3-thiazol-2-yl)acetamide (539.6 mg) as an off-white solid.
mp. 102.5-104°C c
¹H-NMR (DMSO-d₆), δ (ppm): 2.11(3H, s), 2.75(4H, brs), 4.82(2H, s), 6.46(2H, d, J=8.5Hz), 6.69(1H, s), 6.83(2H, d, J=8.5Hz), 12.07(1H, brs).
MS: 262(M+H)⁺

### Step 7

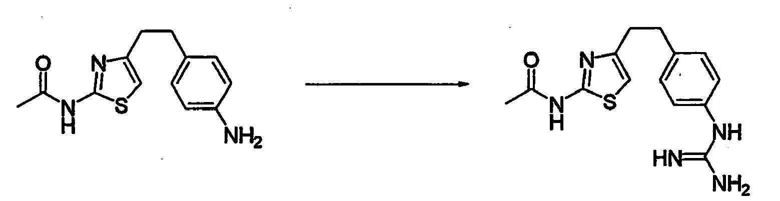

To a suspension of N-(4-(2-(4-aminophenyl)ethyl)-1,3-thiazol-2-yl)acetamide (26 g) in ethanol (500 ml) was added 4N hydrogen chloride in ethyl acetate (25 ml) and cyanamide (6.3 g). The mixture was refluxed for 26 hours. The reaction mixture was cooled to ambient temperature and poured into a mixture of ethyl acetate (500 ml) and saturated sodium hydrogen carbonate solution (500 ml). The resulted precipitate was collected by filtration and washed with water (300 ml) and ethanol (300 ml) to give N-{4-[2-(4-{[amino(imino)methyl]-amino}phenyl)ethyl]-1,3-thiazol-2-yl}acetamide (18 g) as white crystals.
¹H-NMR (DMSO-d₆), δ (ppm): 2.10(3H, s), 2.85(4H, s), 6.79(1H, s), 6.83(2H, d, J=7Hz), 7.10 (2H, d, J=7Hz).
MS: 304(M+H)⁺

### Production Example 1: Synthesis of N-{4-[2-(4-{[amino(imino)methyl]amino}phenyl)ethyl]-5-[3-(methylsulfonyl)benzyl]-1,3-thiazol-2-yl}acetamide

### Step 1

A mixture of 3-(methylthio)benzoic acid (15 g), N,O-dimethylhydroxylamine hydrochloride (8.7 g), 1-hydroxybenzotriazole (3.71 g) and 1-ethyl-3-(3-dimethylaminopropyl)carbodiimide (4.07 g) in N,N-dimethylformamide (DMF, 150 ml) was stirred at ambient temperature for 13 hours. The reaction mixture was poured into saturated NaHCO₃, and extracted with ethyl acetate (AcOEt, 2 times). The combined organic layer was washed with water and brine, dried over anhydrous MgSO₄, and concentrated *in vacuo* to give N-methoxy-N-methyl-3-(methylthio)benzamide (18.3 g) as a yellow oil.
¹H-NMR (CDCl₃) δ (ppm): 2.50 (3H, s), 3.36 (3H, s), 3.56 (3H, s), 7.28-7.45 (3H, m), 7.54 (1H, s).
MS: 212 (M+H)+

### Step 2

To a stirred solution of N-methoxy-N-methyl-3-(methylthio)benzamide (18 g) in dry tetrahydrofuran (THF, 360 ml) was added dropwise diisobutylaluminum hydride (DIBALH, 170 ml) at -78 °C over 40 min under N₂ atmosphere. The reaction mixture was stirred for 2.5 hours at r.t., and then the reaction was quenched with MeOH at 0°C. AcOEt and 1N-HCl were added to the mixture, and the mixture was extracted. The organic layer was washed with brine, dried over anhydrous MgSO₄, and concentrated *in vacuo.* The residual oil (12.9 g), methyl (triphenylphosphoranylidene)acetate (28.5 g) and THF (260 ml) were combined at r.t. under N₂ atmosphere, and the reaction mixture was refluxed for 2 hours. The solvent was removed in *vacuo,* and the residue was suspended in AcOEt. The solid was filtered off, and the filtrate was concentrated *in vacuo.* The residue was purified by flash column chromatography over silica gel with n-hexane / AcOEt (3:1) as an eluent to give methyl (2E)-3-[3-(methylthio)phenyl]acrylate (14.4g) as a colorless wax.
¹H-NMR (DMSO-d₆) δ (ppm): 2.51 (3H, s), 3.81 (3H, s), 6.44 (1H, d, J=16.0Hz), 7.24-7.32 (3H, m), 7.38 (1H, m), 7.65 (1H, d, J=16.0Hz).

### Step 3

Methyl (2E)-3-[3-(methylthio)phenyl]acrylate (14 g), methanol (MeOH, 140 ml), acetic acid (AcOH, 70 ml) and then 10% palladium on carbon (6.72 g) were combined under N₂ atmosphere. The reaction mixture was stirred at r.t. for 9 hours under H₂ atmosphere (4 atm), and filtered through a celite pad. The filtrate was concentrated *in vacuo.* The residue was purified by flash column chromatography over silica gel with n-hexane / AcOEt (3:1) as an eluent to give methyl 3-[3-(methylthio)phenyl]propanoate (12.5 g) as a colorless oil.
¹H-NMR (DMSO-d₆) δ (ppm): 2.48 (3H, s), 2.62 (2H, t, J=8.0Hz), 2.92 (2H, t, J=8.0Hz), 3.68 (3H, s), 6.94-7.00 (1H, m), 7.07-7.14 (2H, m), 7.15-7.24 (1H, m).

### Step 4

28 % Sodium methoxide solution in MeOH (10.8 ml) was added dropwise to the mixture of methyl 3-[3-(methylthio)phenyl]propanoate (11.8 g) and diethyl oxalate (15.2 ml) at 0 °C with stirring. The reaction mixture was stirred at 65 °C for 2 hours under reduced pressure. 15 % aqueous H₂SO₄ (90 ml) was added to the mixture, and refluxed for 13 hours. After cooled to r.t., the mixture was extracted with AcOEt. The organic layer was washed with water and brine, dried over anhydrous MgSO₄, and concentrated *in vacuo.* The residual oil was dissolved in EtOH (50 ml), and concentrated H₂SO₄ (0.5 ml) was added dropwise to the solution. The reaction mixture was refluxed for 6 hours. After cooled to r.t., EtOH was removed *in vacuo.* AcOEt and water were added to the residue, and the mixture was extracted. The organic layer was washed with water and brine, dried over anhydrous MgSO₄, and concentrated *in vacuo.* The residue was purified by flash column chromatography over silica gel with n-hexane / AcOEt (6:1) as an eluent to give ethyl 4-[3-(methylthio)phenyl]-2-oxobutanoate (6.9 g) as a pale yellow oil.
¹H-NMR (CDCl₃) δ (ppm) : 1.36 (3H, t, J=7.5Hz), 2.48 (3H, s), 2.92 (2H, t, J=7.0Hz), 3.17 (2H, t, J=7.0Hz), 4.32 (2H, q, J=7.5Hz), 6.94-7.01 (1H, m), 7.05-7.13 (2H, m), 7.17-7.26 (1H, m).

### Step 5

To a suspension of copper (II) bromide (18.1 g) in AcOEt (140 ml) was added a solution of ethyl 4-[3-(methylthio)phenyl]-2-oxobutanoate (6.8 g) in 70 ml of CHCl₃.

The reaction mixture was refluxed for 10.5 hours, cooled to r.t., and filtered through a short pad of silica gel eluting with AcOEt / n-hexane (1:1). The solvent was removed *in vacuo* to give ethyl 3-bromo-4-[3-(methylthio)phenyl]-2-oxobutanoate (8.6g) as a pale brown oil.
¹H-NMR (CDCl₃) δ (ppm) : 1.38 (3H, t, J=7.5Hz), 2.47 (3H, s), 3.21 (1H, dd, J=14.5, 7.5Hz), 3.50 (1H, dd, J=14.5, 7.5Hz), 4.36 (2H, q, J=7.5Hz), 5.21 (1H, t, J=7.5Hz), 6.98-7.05 (1H, m), 7.11-7.29 (3H, m).

### Step 6

Ethyl 3-bromo-4-[3-(methylthio)phenyl]-2-oxobutanoate (8.5 g) was dissolved in EtOH (85 ml), and then thiourea (3.91 g) was added to the solution. The reaction mixture was refluxed for 2.5 hours under N₂ atmosphere. The cooled reaction mixture was evaporated *in vacuo.* Saturated NaHCO₃ and water were added to the residue, and the mixture was extracted with AcOEt. The organic layer was washed with water and brine, dried over anhydrous MgSO₄, and concentrated *in vacuo.* The residue was purified by flash column chromatography over silica gel with CHCl₃ / AcOEt (1:1) as an eluent to give ethyl 2-amino-5-[3-(methylthio)benzyl]-1,3-thiazole-4-carboxylate (7.1 g) as a brown oil.
¹H-NMR (DMSO-d₆) δ (ppm): 1.25 (3H, t, J=7.0Hz), 2.45 (3H, s), 4.21 (2H, q, J=7.0Hz), 4.30 (2H, s), 6.96-7.29 (4H, m). MS: 309 (M+H)+

### Step 7

Ethyl 2-amino-5-[3-(methylthio)benzyl]-1,3-thiazole-4-carboxylate (7 g) was dissolved in CH₂Cl₂ (100 ml) under N₂ atmosphere. Then pyridine (3.85 ml) and AcCl (1.78 ml) were added dropwise to the solution at 0 °C. The reaction mixture was stirred at r.t. for 1.5 hours. The precipitate was filtered *in vacuo* to give ethyl 2-(acetylamino)-5-[3-(methylthio)benzyl]-1,3-thiazole-4-carboxylate (4.77 g) as a colorless solid.
mp. 187.5-188.5 °C
¹H-NMR (DMSO-d₆) δ (ppm): 1.28 (3H, t, J=7.0Hz), 2.09 (3H, s), 2.45 (3H, s), 4.28 (2H, q, J=7.0Hz), 4.45 (2H, s), 7.00-7.23 (3H, m), 7.26 (1H, t, J=7.5Hz), 12.43 (1H, s).
MS: 351 (M+H)+.

### Step 8

Ethyl 2-(acetylamino)-5-[3-(methylthio)benzyl]-1,3-thiazole-4-carboxylate (7.3 g) was suspended in THF (100 ml), and then lithium borohydride (907 mg) was added portionwise to the solution at 0 °C. The reaction mixture was refluxed for 10 hours. Na₂SO₄ 10H₂O was added to the mixture, and the mixture was stirred at r.t. for 2 hours. The suspension was filtered *in vacuo.* The filtrate was concentrated *in vacuo,* and purified by flash column chromatography over silica gel with CHCl₃ / MeOH (10:1) as an eluent. The solid was washed with ethyl ether to give N-{4-(hydroxymethyl)-5-[3-(methylthio)benzyl]-1,3-thiazol-2-yl}acetamide (5.1 g) as a colorless solid.
mp. 140-141.5 °C
¹H-NMR (DMSO-d₆) δ (ppm): 2.08 (3H, s), 2.44 (3H, s), 4.08 (2H, s), 4.48 (2H, d, J=5.5Hz), 5.08 (1H, t, J=5.5Hz), 5.08 (1H, t, J=5.5Hz), 6.99-7.19 (3H, m), 11.94 (1H, s).
MS: 309 (M+H)+

### Step 9

N-{4-(Hydroxymethyl)-5-[3-(methylthio)benzyl]-1,3-thiazol-2-yl}acetamide (5 g) was dissolved in MeOH (5 ml) and CHCl₃ (50 ml). Then, manganase (IV) oxide (14.1 g) was added to the solution under N₂ atmosphere. The reaction mixture was stirred at r.t. for 20 hours, and filtered through a celite pad. The filtrate was concentrated *in vacuo*. The residual oil was solidified with ethyl ether to give N-{4-formyl-5-[3-(methylthio)benzyl]-1,3-thiazol-2-yl}acetamide (4.46 g) as an off-white solid.
mp. 148-149.5 °C
¹H-NMR (DMSO-d₆) δ (ppm): 2.12 (3H, s), 2.45 (3H, s), 4.50 (2H, s), 6.99-7.25 (3H, m), 7.27 (1H, t, J=7.5Hz), 10.04 (1H, s), 12.35 (1H, brs).
MS: 307 (M+H)+

### Step 10

1-(Bromomethyl)-4-nitrobenzene (959 mg), triphenylphosphine (1.16 g) and DMF (12 ml) were combined under N₂ atmosphere. The reaction mixture was stirred at r.t. for 2.5 hours. Then, potassium tert-butoxide (586 mg) and N-{4-formyl-5-[3-(methylthio)benzyl]-1,3-thiazol-2-yl}acetamide (800 mg) were added to the mixture at 0 °C, and the mixture was stirred at r.t. for 15 hours. The reaction mixture was poured into ice-water, and extracted with AcOEt. The organic layer was washed with 1N-HCl, water and brine, dried over anhydrous MgSO₄, and concentrated *in vacuo.* The residue was purified by flash column chromatography over silica gel with CHCl₃ / AcOEt (1:1) as an eluent to give a mixture of N-{5-[3-(methylthio)benzyl]-4-[(E)-2-(4-nitrophenyl)vinyl]-1,3-thiazol-2-yl}acetamide and N-{5-[3-(methylthio)benzyl]-4-[(Z)-2-(4-nitrophenyl)vinyl]-1,3-thiazol-2-yl}acetamide (E : Z = 1 : 2) (1.08 g) as yellow amorphous.
¹H-NMR (DMSO-d₆) δ (ppm) : 2.08 (3H×2/3, s), 2.12 (3H×1/3, s), 2.44 (3H, s), 4.06 (2H×2/3, s), 4.32 (2H×1/3, s), 6.73 (1H×2/3, d, J=12.5Hz), 6.87 (1H×2/3, d, J=12.5Hz), 6.97-8.27 (26/3H, m), 11.87 (1H×2/3, s), 12,19(1H×1/3, s).
MS: 426 (M+H)+

### Step 11

Potassium peroxymonosulfate (1.55 g) was suspended in water (2.5 ml) and THF (8 ml), and then a mixture of N-{5-[3-(methylthio)benzyl]-4-[(E)-2-(4-nitrophenyl)vinyl]-1,3-thiazol-2-yl}acetamide and N-{5-[3-(methylthio)benzyl]-4-[(Z)-2-(4-nitrophenyl)vinyl]-1,3-thiazol-2-yl}acetamide (E : Z = 1 : 2) (1.07 g) in THF (2 ml) was added dropwise to the suspension at 0 °C. The reaction mixture was stirred at r.t. for 1 hour, and then water was added to the suspension. The mixture was extracted with AcOEt (twice). The combined organic layer was washed with brine, dried over anhydrous MgSO₄, and concentrated *in vacuo* to give N-{5-[3-(methylsulfonyl)benzyl]-4-[(Z)-2-(4-nitrophenyl)vinyl]-1,3-thiazol-2-yl}acetamide (940.7 mg) as brown amorphous.
¹H-NMR (DMSO-d₆) δ (ppm): 2.08 (3H, s), 3.18 (3H, s), 4.24 (2H, s), 6.73 (1H, d, J=12.5Hz), 6.87 (1H, d, J=12.5Hz), 6.84-7.44 (8H, m), 11.92 (1H, s).
MS: 458 (M+H)+

### Step 12

N-{5-[3-(Methylsulfonyl)benzyl]-4-[(Z)-2-(4-nitrophenyl)vinyl]-1,3-thiazol-2-yl}acetamide (1 g), 10% palladium on carbon (656 mg), MeOH (10 ml), THF (10 ml) and AcOH (1 ml) were combined. The reaction mixture was stirred at r.t. for 2 hours under 4 atm H₂ atmosphere, and filtered through a celite pad. The filtrate was concentrated *in vacuo,* and purified by flash column chromatography over silica gel with CHCl₃ / MeOH (20:1) as an eluent to give N-{4-[2-(4-aminophenyl)ethyl]-5-[3-(methylsulfonyl)benzyl]-1,3-thiazol-2-yl}acetamide (479.5 mg) as yellow amorphous.
¹H-NMR (DMSO-d₆) δ (ppm): 2.08 (3H, s), 2.59-2.86 (4H, m), 3.18 (3H, s), 4.02 (2H, s), 4.84 (2H, brs), 6.46 (2H, d, J=8.5Hz), 6.78 (2H, d, J=8.5Hz), 7.25-7.88 (4H, m), 12.03 (1H, s).
MS: 430 (M+H)+

### Step 13

N-{4-[2-(4-Aminophenyl)ethyl]-5-[3-(methylsulfonyl)benzyl]-1,3-thiazol-2-yl}acetamide (470 mg), N,N'-bis(tert-butoxycarbonyl)-1H-pyrazole-1-carboxamidine (340 mg) and THF (5 ml) were combined under N₂ atmosphere. The reaction mixture was stirred at r.t. for 18 hours, and concentrated *in vacuo.* The residue was purified by flash column chromatography over silica gel with CHCl₃ / AcOEt (1:1) as an eluent to give di-tert-butyl ({[4-(2-{2-(acetylamino)-5-[3-(methylsulfonyl)benzyl]-1,3-thiazol-4-yl}ethyl)phenyl]amino}methylidene)biscarbamate (502.9 mg) as yellow amorphous.
¹H-NMR (DMSO-d₆) δ (ppm): 1.39 (9H, s), 1.51 (9H, s), 2.09 (3H, s), 2.85 (4H, s), 3.18 (3H, s), 4.06 (2H, s), 7.13 (2H, d, J=8.0Hz), 7.37-7.45 (1H, m), 7.41 (2H, d, J=8.0Hz), 7.56 (1H, t, J=8.0Hz), 7.74-7.80 (2H, m), 9.94 (1H, s), 11.44 (1H, s), 12.05 (1H, s).
MS: 672 (M+H)+

### Step 14

Di-tert-butyl ({[4-(2-{2-(acetylamino)-5-[3-(methylsulfonyl)benzyl]-1,3-thiazol-4-yl}ethyl)phenyl]amino}methylidene)biscarbamate (480 mg), 4N HCl in 1,4-dioxane solution (8 ml) and MeOH (2 ml) were combined under N₂ atmosphere. The reaction mixture was stirred at r.t. for 14 hours. The solvent was removed *in vacuo.* The residue was dissolved in water and AcOEt. The mixture was made basic (pH=8) with saturated NaHCO₃. The precipitate was collected *in vacuo.* The solid was washed with CH₃CN to give N-{4-[2-(4-{[amino(imino)methyl]amino}phenyl)ethyl]-5-[3-(methylsulfonyl)benzyl]-1,3-thiazol-2-yl}acetamide (157.1 mg) as an off-white solid.
mp. 212-213.5 °C
¹H-NMR (DMSO-d₆) δ (ppm): 2.08 (3H, s), 2.67-2.91 (4H, m), 3.19 (3H, s), 4.08 (2H, s), 6.80 (2H, d, J=8.0Hz), 7.04 (2H, d, J=8.0Hz), 7.42 (1H, d, J=8.0Hz), 7.57 (1H, t, J=8.0Hz), 7.77 (1H, s), 7.79 (1H, d, J=8.0Hz).
MS: 472 (M+H)+

### Production Example 2: Synthesis of N-{4-[4-(2-{[amino(imino)methyl]amino}ethyl)phenyl]-5-[4-(methylsulfonyl)benzyl]-1,3-thiazol-2-yl}acetamide hydrochloride

### Step 1

Ethyl 4-acetylbenzoate (10 g) was dissolved in AcOH (80 ml), and then 90 % pyridinium tribromide (22.2 g) and 33 % hydrobromic acid in AcOH (30 ml) were added to the solution at 0 °C. The reaction mixture was stirred at r.t. for 1 hour, and poured into ice-water. The precipitate was collected *in vacuo* to give ethyl 4-(bromoacetyl)benzoate (15.1 g) as an off-white solid.
mp. 67-68.5 °C
¹H-NMR (DMSO-d₆) δ (ppm): 1.34 (3H, t, J=7.0Hz), 4.36 (2H, q, J=7.0Hz), 5.00 (2H, s), 8.09 (2H, d, J=9.0Hz), 8.14 (2H, d, J=9.0Hz).

### Step 2

Ethyl 4-(bromoacetyl)benzoate (15 g), triphenylphosphine (14.5 g), CH₃CN (200 ml) and pyridine (0.1 ml) were combined under N₂ atmosphere. The reaction mixture was stirred at r.t. for 5 hours. The solvent was removed *in vacuo.* The residual amorphous was solidified with THF / ethyl ether to give {2-[4-(ethoxycarbonyl)phenyl]-2-oxoethyl}(triphenyl)phosphonium bromide (22.7 g) as a colorless solid.
mp. 201-202.5 °C
¹H-NMR (DMSO-d₆) δ (ppm): 1.35 (3H, t, J=7.0Hz), 4.37 (2H, q, J=7.0Hz), 6.31 (2H, d, J=13.0Hz), 7.70-7.96 (15H, m), 8.14 (2H, d, J=8.5Hz), 8.22 (2H, d, J=8.5Hz).

### Step 3

Na₂CO₃ (5.96 g) was added to {2-[4-(ethoxycarbonyl)phenyl]-2-oxoethyl}(triphenyl)phosphonium bromide (15 g), benzene (140 ml) and water (140 ml) in a separatory funnel. The mixture was shaken until the solids dissolved (about 30 min). The aqueous layer was separated and extracted with benzene. The combined organic layer was washed with brine, dried over anhydrous MgSO₄, and concentrated *in vacuo.* Benzene (110 ml) was added, followed by 4-(methylthio)benzaldehyde (4.71 g), and the solution was heated at reflux for 22 hours. After cooled to r.t., the mixture was concentrated *in vacuo.* The residue was purified by flash column chromatography over silica gel with n-hexane / AcOEt (3:1) as an eluent, and triturated with n-hexane to give ethyl 4-{(2E)-3-[4-(methylthio)phenyl]-2-propenoyl}-benzoate (5 g) as a yellow solid.
mp. 115-115.5 °C
¹H-NMR (CDCl₃) δ (ppm): 1.43 (3H, t, J=7.5Hz), 2.53 (3H, s), 4.42 (2H, q, J=7.5Hz), 7.26 (2H, d, J=8.5Hz), 7.46 (1H, d, J=15.5Hz), 7.57 (2H, d, J=8.5Hz), 7.79 (1H, d, J=15.5Hz), 8.04 (2H, d, J=8.5Hz), 8.17 (2H, d, J=8.5Hz).
MS: 327 (M+H)+

### Step 4

Ethyl 4-{3-[4-(methylsulfonyl)phenyl]propanoyl}benzoate was prepared from the compound of Step 3 of Production Example 2 in a manner similar to Step 11 of Production Example 1.
mp. 118-119.5 °C
¹H-NMR (DMSO-d₆) δ (ppm): 1.34 (3H, t, J=7.0Hz), 3.06 (2H, t, J=7.5Hz), 3.19 (3H, s), 3.51 (2H, t, J=7.5Hz), 4.35 (2H, q, J=7.0Hz), 7.58 (2H, d, J=8.5Hz), 7.84 (2H, d, J=8.5Hz), 8.06 (2H, d, J=8.5Hz), 8.12 (2H, d, J=8.5Hz).
MS: 361 (M+H)+

### Step 5

Ethyl 4-{2-bromo-3-[4-(methylsulfonyl)phenyl]propanoyl}benzoate was prepared from the compound of Step 4 of Production Example 2 in a manner similar to Step 5 of Production Example 1.
¹H-NMR (DMSO-d₆) δ (ppm): 1.34 (3H, t, J=7.0Hz), 3.21 (3H, s), 3.38 (1H, dd, J=14.5, 9.0Hz), 3.70 (1H, dd, J=14.5, 5.0Hz), 4.36 (2H, q, J=7.0Hz), 6.06 (1H, dd, J=9.0, 5.0H), 7.58 (2H, d, J=8.5Hz), 7.90 (2H, d, J=8.5Hz), 8.10 (2H, d, J=8.5Hz), 8.22 (2H, d, J=8.5Hz).
MS: 439 (M+H)+

### Step 6

Ethyl 4-{2-amino-5-[4-(methylsulfonyl)benzyl]-1,3-thiazol-4-yl}benzoate was prepared from the compound of Step 5 of Production Example 2 in a manner similar to Step 6 of Production Example 1.
mp. 205.5-207 °C
¹H-NMR (DMSO-d₆) δ (ppm): 1.32 (3H, t, J=7.0Hz), 3.20 (3H, s), 4.26 (2H, s), 4.31 (2H, q, J=7.0Hz), 7.03 (2H, s), 7.47 (2H, d, J=8.0Hz), 7.69 (2H, d, J=8.5Hz), 7.88 (2H, d, J=8.0Hz), 7.97 (2H, d, J=8.5Hz) .MS: 417 (M+H)+

### Step 7

Ethyl 4-(2-(acetylamino)-5-[4-(methylsulfonyl)benzyl]-1,3-thiazol-4-yl)benzoate was prepared from the compound of Step 6 of Production Example 2 in a manner similar to Step 7 of Production Example 1.
¹H-NMR (DMSO-d₆) δ (ppm): 1.31 (3H, t, J=7.0Hz), 2.13 (3H, s), 3.20 (3H, s), 4.33 (2H, q, J=7.0Hz), 4.42 (2H, s), 7.49 (2H, d, J=8.0Hz), 7.77 (2H, d, J=8.0Hz), 7.88 (2H, d, J=8.0Hz), 8.03 (2H, d, J=8.0Hz), 12.28 (1H, s) .MS: 459 (M+H)+

### Step 8

N-{4-[4-(Hydroxymethyl)phenyl]-5-[4-(methylsulfonyl)benzyl]-1,3-thiazol-2-yllacetamide was prepared from the compound of Step 7 of Production Example 2 in a manner similar to Step 8 of Production Example 1.
¹H-NMR (DMSO-d₆) δ (ppm): 2.12 (3H, s), 3.12 (3H, s), 4.35 (2H, s), 4.53 (2H, d, J=5.5Hz), 5.22 (1H, t, J=5.5Hz), 7.38 (2H, d, J=8.5Hz), 7.46 (2H, d, J=8.5Hz), 7.56 (2H, d, J=8.5Hz), 7.87 (2H, d, J=8.5Hz), 12.20 (1H, s).MS: 417 (M+H)+

### Step 9

N-{4-[4-(Hydroxymethyl)phenyl]-5-[4-(methylsulfonyl)benzyl]-1,3-thiazol-2-yl}acetamide (1.25 g), CHCl₃ (12 ml), CH₃CN (12 ml) and Dess-Martin periodinane (1.91 g) were combined at 0 °C under N₂ atmosphere. The reaction mixture was stirred at r.t. for 1 hour, and diluted in CHCl₃. The organic solution was washed with saturated NaHCO₃, water and brine, dried over anhydrous MgSO₄, and concentrated *in vacuo.* The residue was purified by flash column chromatography over silica gel with CHCl₃ / MeOH (20:1) as an eluent to give N-{4-(4-formylphenyl)-5-[4-(methylsulfonyl)benzyl]-1,3-thiazol-2-yl}acetamide (1.99 g) as a brown oil.
¹H-NMR (DMSO-d₆) δ (ppm): 2.14 (3H, s), 3.20 (3H, s), 4.45 (2H, s), 7.49 (2H, d, J=8.0Hz), 7.85 (2H, d, J=8.5Hz), 7.88 (2H, d, J=8.0Hz), 7.99 (2H, d, J=8.5Hz), 10.04 (1H, s), 12.28 (1H, s).
MS: 415 (M+H)+

### Step 10

N-{4-(4-Formylphenyl)-5-[4-(methylsulfonyl)benzyl]-1,3-thiazol-2-yl}acetamide (1.99 g), methyl (triphenylphosphoranylidene)acetate (3.21 g) and THF (20 ml) were combined at r.t. under N₂ atmosphere, and the mixture was refluxed for 1 hour. The solvent was removed *in vacuo.* The residual solid was washed with AcOEt to give methyl (2E)-3-(4-{2-(acetylamino)-5-[4-(methylsulfonyl)benzyl]-1,3-thiazol-4-yl}phenyl)acrylate (779.3 mg) as an off-white solid.
mp. 217-218.5 °C
¹H-NMR (DMSO-d₆) δ (ppm): 2.13 (3H, s), 3.20 (3H, s), 3.73 (3H, s), 4.41 (2H, s), 6.67 (1H, d, J=16.0Hz), 7.48 (2H, d, J=8.0Hz), 7.65 (2H, d, J=8.5Hz), 7.69 (1H, d, J=16.0Hz), 7.80 (2H, d, J=8.5Hz), 7.88 (2H, d, J=8.0Hz), 12.23 (1H, s). MS: 471 (M+H)+

### Step 11

Methyl (2E)-3-(4-{2-(acetylamino)-5-[4-(methylsulfonyl)benzyl]-1,3-thiazol-4-yl}phenyl)acrylate (2.3 g), 10% palladium on carbon (1.96 g), MeOH (23 ml), THF (23 ml) and AcOH (2.3 ml) were combined under N₂ atmosphere. The reaction mixture was stirred at r.t. for 7 hours under H₂ atmosphere (4 atm), and the mixture was filtered through a celite pad. The filtrate was concentrated *in vacuo* to give methyl 3-(4-{2-(acetylamino)-5-[4-(methylsulfonyl)benzyl]-1,3-thiazol-4-yl}phenyl)propanoate (1.28 g) as a colorless solid.
mp. 1.29-131 °C
¹H-NMR (DMSO-d₆) δ (ppm): 2.12 (3H, s), 2.66 (2H, t, J=7.0Hz), 2.88 (2H, t, J=7.0Hz), 3.20 (3H, s), 3.58 (3H, s), 4.35 (2H, s), 7.29 (2H, d, J=8.0Hz), 7.46 (2H, d, J=8.0Hz), 7.52 (2H, d, J=8.0Hz), 7.88 (2H, d, J=8.0Hz), 12.17 (1H, s).
MS: 473 (M+H)

### Step 12

Methyl 3-(4-{2-(acetylamino)-5-[4-(methylsulfonyl)benzyl]-1,3-thiazol-4-yl}phenyl) propanoate (1.01 g), 1N-NaOH (5.34 ml) and 1,4-dioxane (10 ml) were combined at 0 °C, and the reaction mixture was stirred at r.t. for 1 hour. The organic solvent was evaporated *in vacuo.* The residual aqueous solution was washed with AcOEt. The aqueous layer was acidified with 1N-HCl. The precipitate was filtered off *in vacuo* to give 3-(4-{2-(acetylamino)-5-[4-(methylsulfonyl)benzyl]-1,3-thiazol-4-yl}phenyl)propanoic acid (800.5 mg) as a colorless solid.
mp. 208.5-210 °C
¹H-NMR (DMSO-d₆) δ (ppm): 2.12 (3H, s), 2.55 (2H, t, J = 7.5 Hz), 2.85 (2H, t, J = 7.5 Hz), 3.20 (3H, s), 4.35 (2H, s), 7.30 (2H, d, J = 8.5 Hz), 7.46 (2H, d, J = 8.5 Hz), 7.51 (2H, d, J = 8.5 Hz), 7.87 (2H, d, J = 8.5 Hz), 12.16 (1H, brs), 12.18 (1H, s).
MS: 459 (M+H)

### Step 13

3-(4-{2-(Acetylamino)-5-[4-(methylsulfonyl)benzyl]-1,3-thiazol-4-yl}phenyl)propanoic acid (400 mg), Et₃N (0.182 ml) and t-BuOH (8 ml) were combined under N₂ atmosphere. Diphenylphosphoryl azide (0.226 ml) was added dropwise to the solution at r.t. The reaction mixture was refluxed for 10 hours, and cooled to r.t. The mixture was diluted with AcOEt. The organic solution was washed with 1N-HCl, water and brine, dried over anhydrous MgSO₄, and concentrated *in vacuo.* The residue was purified by flash column chromatography over NH silica gel with CHCl₃ / MeOH (20:1) as an eluent to give tert-butyl [2-(4-{2-(acetylamino)-5-[4-(methylsulfonyl)benzyl]-1,3-thiazol-4-yl}phenyl)ethyl]carbamate (168.3 mg) as colorless amorphous.
¹H-NMR (DMSO-d₆) δ (ppm): 1.36 (9H, s), 2.12 (3H, s), 2.72 (2H, m), 3.15 (2H, m), 3.20 (3H, s), 4.35 (2H, s), 6.88 (1H, t, J = 5.5 Hz), 7.26 (2H, d, J = 8.5 Hz), 7.46 (2H, d, J = 8.5 Hz), 7.52 (2H, d, J = 8.5 Hz), 7.88 (2H, d, J = 8.5 Hz), 12.17 (1H, s).
MS: 530 (M+H)

### Step 14

tert-Butyl [2-(4-{2-(acetylamino)-5-[4-(methylsulfonyl)benzyl]-1,3-thiazol-4-yl}phenyl)ethyl]carbamate (146.5 mg), 4N HCl in 1,4-dioxane solution (3 ml) and MeOH (1 ml) were combined under N₂ atmosphere. The reaction mixture was stirred at r.t. for 2 hours, and concentrated *in vacuo*. The residue, di-tert-butyl (1H-pyrazol-1-ylmethylidene)biscarbamate (85.8 mg), N,N-diisopropylethylamine (0.0964 ml), THF (3 ml) and DMF (1 ml) were combined under N₂ atmosphere. The reaction mixture was stirred at r.t. for 2 hours, and concentrated *in vacuo.* The residue was purified by flash column chromatography over silica gel with CHCl₃ / MeOH (20:1) as an eluent to give di-tert-butyl ([2-(4-{2-(acetylamino)-5-[4-(methylsulfonyl)benzyl]-1,3-thiazol-4-yl}phenyl)ethylamino]methylidene)biscarbamate (118.8 mg) as colorless amorphous.
¹H-NMR (DMSO-d₆) δ (ppm): 1.39 (9H, s), 1.44 (9H, s), 2.12 (3H, s), 2.85 (2H, m), 3.33 (3H, s), 3.56 (2H, m), 4.35 (2H, s), 7.31 (2H, d, J = 8.0 Hz), 7.46 (2H, d, J = 8.0 Hz), 7.54 (2H, d, J = 8.0 Hz), 7.87 (2H, d, J = 8.0 Hz), 8.36 (1H, t, J = 5.5 Hz), 11.49 (1H, s), 12.17 (1H, s).
MS: 672 (M+H)

### Step 15

Di-tert-butyl ({2-(4-{2-(acetylamino)-5-[4-(methylsulfonyl)benzyl]-1,3-thiazol-4-yl}phenyl)ethylamino}methylidene)biscarbamate (144.7 mg), MeOH (1 ml) and 4N HCl in 1,4-dioxane solution (3 ml) were combined under N₂ atmosphere. The reaction mixture was stirred at r.t. for 19 hours. The solvent was removed in *vacuo.* The residue was washed with AcOEt to give N-{4-[4-(2-{[amino(imino)methyl]amino}ethyl)phenyl]-5-[4-(methylsulfonyl)benzyl]-1,3-thiazol-2-yl}acetamide hydrochloride (67.3 mg) as a pale brown amorphous solid.
¹H-NMR (DMSO-d₆) δ (ppm): 2.13 (3H, s), 2.76-2.90 (2H, m), 3.21 (3H, s), 3.32-3.49 (2H, m), 4.36 (2H, s), 7.35 (2H, d, J=8.0Hz), 7.46 (2H, d, J=8.0Hz), 7.54 (2H, d, J=8.0Hz), 7.68 (1H, t, J=5.5Hz), 7.88 (2H, d, J=8.0Hz), 12.18 (1H, s).
MS: 472 (M+H) free

### Production Example 3: Synthesis of N-{4-[2-(4-{[amino(imino)methyl]amino}phenyl)ethyl]-5-[4-(aminosulfonyl)benzyl]-1,3-thiazol-2-yl}acetamide hydrochloride

### Step 1

To a suspension of copper(II) bromide (9.75 g) in AcOEt (150 ml) was added a solution of ethyl 2-oxo-4-phenylbutanoate (3 g) in 75 ml of CHCl₃. The reaction mixture was refluxed for 23 hours, cooled to r.t., and filtered through a short pad of silica gel eluting with AcOEt / n-hexane (1:1). The solvent was removed *in vacuo* to give ethyl 3-bromo-2-oxo-4-phenylbutanoate (4.2g) as a yellow liquid.
¹H-NMR (CDCl₃) δ (ppm): 1.37 (3H, t, J=7.0Hz), 3.25 (1H, dd, J=14.5, 7.5Hz), 3.54 (1H, dd, J=14.5, 7.5Hz), 4.35 (2H, q, J=7.0Hz), 5.27 (1H, d, J=7.5Hz), 7.18-7.41 (5H, m).

### Step 2

Ethyl 3-bromo-2-oxo-4-phenylbutanoate (5.8 g) was dissolved in EtOH (110 ml), and then thiourea (3.1 g) was added to the solution. The reaction mixture was refluxed for 2 hours under N₂ atmosphere. The cooled reaction mixture was evaporated *in vacuo*. The residual solid was suspended (pH=8) in saturated NaHCO₃ and water. The solid was collected by filtration, and purified by flash column chromatography over silica gel with CHCl₃ / MeOH (10:1) as an eluent to give ethyl 2-amino-5-benzyl-1,3-thiazole-4-carboxylate (808.2 mg) as a yellow wax.
¹H-NMR (DMSO-d₆) δ (ppm): 1.25 (3H, t, J=7.0Hz), 4.21 (2H, q, J=7.0Hz), 4.33 (2H, s), 7.02 (2H, s), 7.11-7.39 (5H, m).
MS: 263 (M+H)⁺

### Step 3

Ethyl 2-amino-5-benzyl-1,3-thiazole-4-carboxylate (2.1 g) was dissolved in pyridine (21 ml), and then acetyl chloride (1.71 ml) was added dropwise to the solution at 0 °C under N₂ atmosphere. The reaction mixture was stirred at room temperature for 2.5 hr. Water was added to the solution at 0 °C, and the precipitate was filtered off *in vacuo.* The solid was washed with diethyl ether to give ethyl 2-(acetylamino)-5-benzyl-1,3-thiazole-4-carboxylate (1.92 g) as a brown solid.
mp. 178-180 °C
¹H-NMR (DMSO-d₆) δ (ppm): 1.28 (3H, t, J=7.0Hz), 2.09 (3H, s), 4.28 (2H, q, J=7.0Hz), 4.48 (2H, s), 7.19-7.39 (5H, m), 12.41 (1H, s).
MS: 305 (M+H)⁺

### Step 4

Ethyl 2-(acetylamino)-5-benzyl-1,3-thiazole-4-carboxylate (1.0 g) was dissolved in THF (20 ml), and then lithium borohydride (124 mg) was added portionwise to the solution at 0 °C. The reaction mixture was refluxed for 4.5 hr, and the reaction was quenched with MeOH. The mixture was concentrated *in vacuo,* and purified by flash column chromatography over silica gel with CHCl₃ / MeOH (20:1) as an eluent. The residual amorphous substance was dissolved in MeOH (1 ml) and CHCl₃ (8 ml). Then manganase(IV) oxide (1.26 g) was added to the solution under N₂ atmosphere. The reaction mixture was stirred at r.t. for 12 hours, and filtered through a celite pad. The filtrate was concentrated *in vacuo.* The residue was purified by flash column chromatography over silica gel with CHCl₃ / MeOH (20:1) as an eluent to give N-(5-benzyl-4-formyl-1,3-thiazol-2-yl)acetamide (251 mg) as a pale yellow solid.
mp. 191-192.5 °C
¹H-NMR (DMSO-d₆), δ (ppm): 2.12 (3H, s), 4.53(2H, s), 7.19-7.40 (5H, m), 10.04 (1H, s), 12.34 (1H, s).
MS: 261 (M+H)⁺

### Step 5

N-{5-Benzyl-4-[(Z)-2-(4-nitrophenyl)vinyl]-1,3-thiazol-2-yl}acetamide was prepared from the compound of Step 4 of Production Example 3 in a manner similar to Step 10 of Production Example 1.
¹H-NMR (DMSO-d₆), δ (ppm): 2.06, 2.13 (3H, s), 4.03, 4.18 (2H, s), 6.69 (1H, s), 7.17-7.35 (6H, m), 7.40, 7.54 (2H, dx2, J = 8.9 Hz), 7.99, 8.20 (2H, dx2, J = 8.9 Hz), 9.97, 10.19 (1H, sx2).
MS: 380 (M+H)+

### Step 6

To the solution of N-{5-benzyl-4-[(Z)-2-(4-nitrophenyl)vinyl]-1,3-thiazol-2-yl}acetamide in chloroform was added chlorosulfonic acid dropwise under ice-cooling. This was stirred at room temperature for 15 hr and then rotary evaporated to a reduced volume. To the solution was added aq. saturated NaHCO₃ solution. The mixture was extracted with THF. The organic layer was dried over MgSO₄ and concentrated to give 4-({2-(acetylamino)-4-[(E)-2-(4-nitrophenyl)vinyl)-1,3-thiazol-5-yl}methyl)benzenesulfonyl chloride as crude oil. This was used for the next reaction without further purification.
MS: 478 (M+H)+

### Step 7

To a solution of 4-({2-(acetylamino)-4-[(E)-2-(4-nitrophenyl)vinyl]-1,3-thiazol-5-yl}methyl)benzenesulfonyl chloride (170 mg) in THF (5 ml) was added ammonium hydroxide (28%, 1.5 ml) at 5 °C. The mixture was stirred at 25 °C for 12 hr. The reaction was quenched with aq. saturated ammonium chloride solution. The mixture was extracted with Ethyl acetate, dried over MgSO₄, filtered, concentrated *in vacuo* to give orange powder. m/z 459 (M+H)+

The resulting orange powder was dissolved in MeOH and AcOH (1 ml). To the solution was added 10 % Pd/C (50 % wet), and the mixture was stirred at 25 °C under hydrogen for 15 hr and filtered through a celite pad. The filtrate was added aq. 0.1N NaOH. The mixture was extracted with ethyl acetate. The organic layer was dried over MgSO₄, filtered, concentrated *in vacuo.* The residue was purified by silicagel column chromatography with chloroform and methanol (20:1) as an eluent to give N-{4-[2-(4-aminophenyl)ethyl]-5-[4-(aminosulfonyl)benzyl]-1,3-thiazol-2-yl}acetamide (33 mg) as white powder.
¹H-NMR (200MHz, DMSO-d₆) δ (ppm): 1.88 (3H, s), 3.65-2.76 (4H, m), 3.93 (2H, s), 4.84 (2H, s), 6.46 (2H, d, J=8.3 Hz), 6.78 (2H, d, J=8.3 Hz), 7.22 (2H, d, J=8.3 Hz), 7.28 (2H, s), 7.72 (2H, d, J=8.3 Hz), 12.00 (1H, s).
MS: 431 (M+H)+

### Step 8

Di-tert-butyl ((Z)-{[4-(2-{2-(acetylamino)-5-[4-(aminosulfonyl)benzyl]-1,3-thiazol-4-yl}ethyl)phenyl]amino}methylidene)biscarbamate was prepared from the compound of Step 7 of Production Example 3 in a manner similar to Step 13 of Production Example 1. ¹H-NMR (DMSO-d₆), δ (ppm): 1.39 (9H, s), 1.50 (9H, s), 2.08 (3H, s), 2.85 (4H, br), 4.00 (2H, s), 7.15 (2H, d), 7.30 (2H, d), 7.43 (2H, d), 7.72 (2H, d), 9.95 (1H, s), 11.44 (1H, s), 12.03 (1H, s).
MS: 673 (M+H)+

### Step 9

The title compound was prepared from the compound of Step 8 of Production Example 3 in a manner similar to Step 14 of Production Example 1. ¹H-NMR (DMSO-d₆), δ (ppm): 1.95 (3H, s), 2.86 (4H, s), 3.99
(2H, s), 7.14 (2H, d, J = 8.5 Hz), 7.25 (2H, d, J = 8.5 Hz), 7.27-7.35 (8H, m), 7.74 (2H, d, J = 8.5 Hz), 10.30 (1H, s), 12.03 (1H, s).
MS: 473 (M+H)+

### Production Example 4: Synthesis of N-(4-[2-(4-{[amino(imino)methyl]amino}phenyl)ethyl]-5-{4-[(dimethylamino)sulfonyl]benzyl}-1,3-thiazol-2-yl)acetamide hydrochloride

### Step 1

To a solution of 4-({2-(acetylamino)-4-[(E)-2-(4-nitrophenyl)vinyl]-1,3-thiazol-5-yl}methyl)benzenesulfonyl chloride (435 mg) in THF(10 ml) was added dimethylamine (50 % solution, 400 µl) at 25 °C. The mixture was stirred at 25 °C for 12 hr. The reaction was quenched with aq. ammonium chloride solution. The mixture was extracted with ethyl acetate, and the extract was dried over MgSO₄, filtered and concentrated *in vacuo* to give orange powder.

The resulting orange powder was dissolved in DMF, MeOH and AcOH (10 ml, 5 ml and 2 ml, respectively). To this soluiton was added 10 % Pd/C (50 % wet, 200 mg) and the mixture was stirred for 1 hr under 3 atm prssure of hydrogen, and then filtered through a celite pad. The filtrate was added aq. 0.1N NaOH solution. The mixture was extracted with ethyl acetate. The organic layer was dried over MgSO₄, filtered and concentrated *in vacuo.* The residue was purified by silicagel column chromatography with chloroform and methanol (20:1) as an eluent to give N-(4-[2-(4-aminophenyl)ethyl]-5-{4-[(dimethylamino)sulfonyl]benzyl}-1,3-thiazol-2-yl)acetamide (200 mg) as white powder. ¹H-NMR (DMSO-d₆), δ (ppm): 2.08 (3H, s), 2.56 (4H, s), 3.36 (6H, s), 3.99 (2H, s), 4.84 (2H, s), 6.45 (2H, d), 6.75 (2H, d), 7.27 (2H, d), 7.64 (2H, d), 12.03 (1H, s).
MS: 459 (M+H)+

### Step 2

Di-tert-butyl [(Z)-({4-[2-(2-(acetylamino)-5-{4-[(dimethylamino)sulfonyl]benzyl}-1,3-thiazol-4-yl)ethyl]phenyl}amino)methylidene]biscarbamate was prepared from the compound of Step 1 of Production Example 4 in a manner similar to Step 13 of Production Example 1.
¹H-NMR (DMSO-d₆), δ (ppm): 1.38 (9H, s), 1.50 (9H, s), 2.08 (3H, s), 2.54 (6H, s), 2.84 (4H, s), 4.02 (2H, s), 7.10 (2H, d, J = 8.4 Hz), 7.27 (2H, d, J = 8.4 Hz), 7.43 (2H, d, J = 8.4 Hz), 7.61 (2H, d, J = 8.4 Hz), 9.99 (1H, s), 11.45 (1H, s).

### Step 3

The title compound was prepared from the compound of Step 2 of Production Example 4 in a manner similar to Step 14 of Production Example 1.
¹H-NMR (DMSO-d₆), δ (ppm): 2.09 (3H, s), 2.57 (6H, s), 2.84 (4H, s), 4.08 (2H, s), 7.12 (2H, d, J = 8.4 Hz), 7.23 (2H, d, J = 8.4 Hz), 7.40 (2H, d, J = 8.0 Hz), 7.42 (4H, br, J = 8.0 Hz), 8.02 (2H, d, J = 8.0 Hz), 9.86 (1H, s), 12.05 (1H, s).
MS: 501 (M+H)+

### Production Example 5: Synthesis of N-{4-[4-(4-{[amino(imino)methyl]amino}-3-oxobutyl)phenyl]-1,3-thiazol-2-yl}acetamide hydrochloride

### Step 1

To the solution of ethyl 3-phenylpropanoate (8 g) in CH₂Cl₂ (25 ml) was added bromoacetyl chloride (6.0 ml). This solution was maintained under -5 °C. To the solution was added alumnium chloride (16.2 g) over 15 min. After addition of AlCl₃, this was stirred at 0 °C for 30 min. Then the reaction mixture was refluxed for 1 hr. Then the reaction mixture was poured into ice-water and was extracted with CH₂Cl₂. The organic layer was washed with brine, dried over MgSO₄, filterd, and concentrated *in vacuo* to give green liquid of ethyl 3-[4-(bromoacetyl)phenyl]propanoate. This was used for the next reaction without further purification.
¹H-NMR (DMSO-d₆), δ (ppm): 1.23 (3H, t, J = 7.2 Hz), 2.65 (2H, t, J = 7.6 Hz), 3.02 (2H, t, J = 7.6 Hz), 4.13 (2H, q, J = 7.2 Hz), 4.43 (2H, s), 7.43 (2H, d, J = 8.1 Hz), 7.92 (2H, d, J = 8.1 Hz).

### Step 2

Ethyl 3-[4-(bromoacetyl)phenyl]propanoate (13 g) was dissolved into ethanol (EtOH, 70 ml). To the solution was added thiourea (4.8 g), and the mixture was refluxed for 3 hr, and then rotary evaporated to reduced volume. The resulting concentrated solution was poured into water, and the mixture was extracted with ethyl acetate, and the extract was dried over MgSO₄, filtered, and concentrated *in vacuo* to give ethyl 3-[4-(2-amino-1,3-thiazol-4-yl)phenyl]propanoate as pale yelllow oil. This was used for the next reaction without further purification.

MS: m/z 277 (M+H)+

### Step 3

To the solution of ethyl 3-[4-(2-amino-1,3-thiazol-4-yl)phenyl]propanoate (12.4 g) in CH₂Cl₂ (100 ml) were added acetyl chloride (3.82 ml) and pyridine (5.8 ml) at 25 °C. This was stirred for 12 hr at 25 °C and then concentrated *in vacuo.* The residue was dissolved in CH₂Cl₂ and this was washed with aq. NaHCO₃ solution and ammonium chloride solution. The organic layer was dried over MgSO₄, filtered and concentrated *in vacuo* to give a brownish solid. Resulting brown solid was dissolved in MeOH (80 ml) and THF (50 ml). To this solution was added 1N NaOH (50 ml) and the mixture was stirred at 25 °C for 12 hr and was concentrated to a reduced volume. To the aquous solution was added 1N HCl solution to give colourless precipitate. This was collected by filtration, washed with water to give 3-{4-[2-(acetylamino)-1,3-thiazol-4-yl]phenyl}propanoic acid (12.1 g) as a colorless solid.
¹H-NMR (DMSO-d₆), δ (ppm): 2.15 (3H, s), 2.52 (2H, t, J = 7.5 Hz), 2.83 (2H, t, J = 7.5 Hz), 7.27 (2H, d, J = 8.1 Hz), 7.52 (1H, s), 7.78 (2H, d, J = 8.1 Hz), 12.24 (1H, s).
MS: 291 (M+H)+

### Step 4

To a solution of 3-14-[2-(acetylamino)-1,3-thiazol-4-yl]phenyl}propanoic acid (3 g) in CH₂Cl₂ (30 ml) was added oxalyl chloride (1.35 ml) dropwise at 5 °C. After another 5 minutes, 3 drops of DMF were added. This was stirred for 1 hr at 25 °C. Then, the solvent and the reagent were evaporated. The residue was dissolved into THF (30 ml). To the ice-cooled solution of the resulting acid chloride was added another solution that was prepared from ethyl isocyanoacetate (2.82 ml) and 1,8-diazabicyclo[4.5.0]undec-7-ene (DBU, 4.64 ml) in THF (30 ml). This was stirred at 25 °C for 2 days. This was quenced with aq. 0.1N HCl and was extracted with ethyl acetate, and the extract was dried over MgSO₄, filtered, and concentrated *in vacuo.* The residue was purified with silicagel column chlomatography with CH₂Cl₂ and MeOH (5/1) as an eluent to give ethyl 5-(2-{4-[2-(acetylamino)-1,3-thiazol-4-yl]phenyl}ethyl)-1,3-oxazole-4-carboxylate (2.25 g) as white powder.
¹H-NMR (DMSO-d₆), δ (ppm): 1.26 (3H, t, J = 7.1 Hz), 2.15
(3H, s), 2.96 (2H, t, J = 7.4 Hz), 3.33 (2H, t, J = 7.4 Hz), 4.22 (2H, q, J = 7.1 Hz), 7.21 (2H, d, J = 8.2 Hz), 7.54 (1H, s), 7.78 (2H, d), 8.37 (1H, s), 12.20 (1H, s).
MS: 386 (M+H)+

### Step 5

To the solution of ethyl 5-(2-{4-[2-(acetylamino)-1,3-thiazol-4-yl]phenyl}ethyl)-1,3-oxazole-4-carboxylate (2.14 g) in MeOH (5 ml) was added conc. HCl (10 ml). This was stirred at 80 °C for 8 hr. Then, this was concentrated *in vacuo* to give 1-amino-4-[4-(2-amino-1,3-thiazol-4-yl)phenyl]-2-butanone dihydrochloride as a crude solid. This was used for the next reaction without further purification.
1H-NMR (DMSO-d6), d (ppm): 2.93 (4H, m), 3.95 (2H, m), 7.02 (1H, s), 7.33 (2H, d, J = 8.4 Hz), 7.63 (2H, d, J = 8.4 Hz), 8.21 (3H, br), 8.77 (2H, br).

### Step 6

To the solution of 1-amino-4-[4-(2-amino-1,3-thiazol-4-yl)phenyl]-2-butanone dihydrochloride (1.8 g) in methylene chloride (20 ml) and DMF (20 ml) were was added N,N-diisopropylethylamine (DIPEA, 3.3 ml) and di-tert-butyl dicarbonate (1.29 g). This was stirred at room temperature for 12 hr. To the solution was added water, and the mixture was extracted with CH2Cl2. The organic layer was dried over MgSO4, filtered and concentrated. The residual oil was dissolved in pyridine (20 ml). To this solution was added acetylchloride (0.57 ml) under ice-cooling. This was stirred at 25 oC for 2 hr. The solution was poured into water and organic layer was extracted with ethyl acetate. After the The extract was dried over MgSO4, the solvent was removed in vacuo and residual yellow oil was purified by silicagel column chromatography with hexane and ethyl acetate (10/1) as an eluent to give tert-butyl (4-{4-[2-(acetylamino)-1,3-thiazol-4-yl]phenyl}-2-oxobutyl)carbamate (0.3 g) as white powder.
1H-NMR (DMSO-d6), d (ppm): 1.38 (9H, s), 2.15 (3H, s), 2.76 (4H, br), 3.76 (2H, d, J = 5.8 Hz), 7.07 (1H, t), 7.25 (2H, d, J = 8.2 Hz), 7.53 (1H, s), 7.78 (2H, d), 12.22 (1H, s).
MS: m/z 404 (M+H)+

### Step 7

tert-Butyl (4-{4-[2-(acetylamino)-1,3-thiazol-4-yl]phenyl}-2-oxobutyl)carbamate (260 mg) was treated with 4N HCl in dioxane at room temperature for 2 hr. Then, the solvent was evaporated in *vacuo.* The residue was triturated with isopropyl ether (IPE) to give N-{4-[4-(4-amino-3-oxobutyl)phenyl]-1,3-thiazol-2-yl}acetamide hydrochloride (218 mg) as colourless powder.
¹H-NMR (DMSO-d₆), δ (ppm): 2.16 (3H, s), 2.89 (4H, d x2), 3.81 (2H, m), 7.28 (2H, d, J = 8.2 Hz), 7.54 (1H, s), 7.80 (2H, d, J = 8.2 Hz), 8.10 (3H, br), 12.23 (1H, br).
MS: 304 (M+H)+

### Step 8

Di-tert-butyl {(E)-[(4-{4-[2-(acetylamino)-1,3-thiazol-4-yl]phenyl}-2-oxobutyl)amino]methylidene}biscarbamate was prepared from the compound of Step 7 of Production Example 5 in a manner similar to Step 13 of Production Example 1.
¹H-NMR (DMSO-d₆), δ (ppm): 1.38 (9H, s), 1.43 (9H, s), 2.15 (3H, s), 2.84 (4H, s), 4.24 (2H, d, J = 4.8 Hz), 7.27 (2H, d, J = 8.2 Hz), 7.52 (1H, s), 7.79 (2H, d), 8.72 (1H, br), 10.15 (1H, s), 11.43 (1H, s), 12.22 (1H, s).
MS: 546 (M+H)+

### Step 9

The title compound was prepared from the compound of Step 8 of Production Example 5 in a manner similar to Step 14 of Production Example 1.
¹H-NMR (DMSO-d₆), δ (ppm): 2.16 (3H, s), 2.73-2.94 (4H, m), 3.38 (2H, m), 6.53 (1H, br), 7.26 (2H, d, J = 8.0 Hz), 7.32 (2H, s), 7.54 (1H, s), 7.80 (2H, d, J = 8.0 Hz), 11.50 (1H, s), 12.05 (1H, s), 12.21 (1H, s).
MS: m/z 346 (M+H) free

### Production Example 6: Synthesis of N-(4-{2-[4-(3-{[amino(imino)methyl]amino}propyl)phenyl]ethyl}-1,3-thiazol-2-yl)acetamide hydrochloride

### Step 1

To a solution of N-[4-(chloromethyl)-1,3-thiazol-2-yl]acetamide (23.6 g) in toluene (200 ml) and acetonitril (80 ml) was added triphenylphosphine (35.7 g) at 25 °C. The mixture was stirred at 130 °C for 12 hr. Resulting precipitate was collected by filtration and washed with IPE to give {[2-(acetylamino)-1,3-thiazol-4-yl]methyl}(triphenyl)phosphonium chloride (35.7 g) as colourless powder.
¹H-NMR (DMSO-d₆), δ (ppm): 2.11 (3H, s), 5.25 (2H, d, J = 15.3 Hz), 6.86 (1H, d, J = 3.8 Hz), 7.68-7.92 (15H, m), 12.06 (1H, s).

### Step 2

Methyl (2E)-3-(4-formylphenyl)acrylate was prepared from benzene-1,4-dicarbaldehyde in a manner similar to Step 10 of Production Example 2.
¹H-NMR (200MHz, DMSO-d₆) δ (ppm): 3.75 (3H, s), 6.83 (1H, d, J=16.1 Hz), 7.74 (1H, d, J=16.1 Hz), 7.95 (4H, s), 10.03 (1H, s).

### Step 3

Methyl (2E)-3-(4-{(E)-2-[2-(acetylamino)-1,3-thiazol-4-yl]vinyl}phenyl)acrylate was prepared from the compound of Step 1 of Production Example 6 and the compound of Step 2 of Production Example 6 in a manner similar to Step 3 of Production Example 2.
¹H-NMR (DMSO-d₆), δ (ppm): 2.15 (3H, s), 3.73 (3H, s), 6.66 (1H, d, J = 16.0 Hz), 7.24 (1H, d, J = 14.5 Hz), 7.55-7.78 (2H, m), 7.95 (4H, s), 12.20 (1H, br).
MS: 329 (M+H)+

### Step 4

Methyl 3-(4-{2-[2-(acetylamino)-1,3-thiazol-4-yl]ethyl}phenyl)propanoate was prepared from the compound of Step 3 of Production Example 6 in a manner similar to Step 11 of Production Example 2.
¹H-NMR (200MHz, CDCl₃) δ (ppm): 2.24 (3H, s), 2.61 (2H, t, J=7.8 Hz), 2.92 (2H, t, J=7.8 Hz), 2.94 (4H, s), 3.67 (3H, s), 6.49 (1H, s), 7.09 (4H, s).
MS: 333(M+H)+

### Step 5

Methyl 3-(4-{2-[2-(acetylamino)-1,3-thiazol-4-yl]ethyl}phenyl)propanoate (1.0 g) was dissolved in THF (20 ml). To the solution was added lithium tetrahydroborate (1.07 g) portionwise at 5°C. The reaction mixture was refluxed for 4.0 hr and Na₂SO₄ was added, and the mixture was stirred for 12 hr. Precipitate was removed by filtration. The organic solvent was evaporated *in vacuo.* The residue was purified by silicagel column chromatography with hexane and ethyl acetate (3:2 - 1:1) as an eluent to give N-(4-{2-[4-(3-hydroxypropyl)phenyl]ethyl}-1,3-thiazol-2-yl)acetamide (200 mg) as white powder.
¹H-NMR (DMSO-d₆), δ (ppm): 1.87 (2H, m), 2.24 (3H, s), 2.68
(2H, t, J = 7.5 Hz), 2.94 (4H, s), 3.68 (2H, t, J = 7.5 Hz), 6.50 (1H, s), 7.10 (4H, s).
MS: 305 (M+H)+

### Step 6

To a solution of N-(4-{2-[4-(3-hydroxypropyl)phenyl]ethyl}-1,3-thiazol-2-yl)acetamide (180 mg) in THF (5 ml) were added triphenylphosphine (233 mg) and CBr₄ (294 mg) at 0 °C. This was stirred at 25 °C for 1 hr and was poured into water. The mixture was extracted with ethyl acetate. The organic layer was dried over MgSO₄, filtered, concentrated *in vacuo*. The residue was purified by silicagel column chromatography with hexane and ethyl acetate (1/2) as an eluent to give N-(4-{2-[4-(3-bromopropyl)phenyl]ethyl}-1,3-thiazol-2-yl)acetamide (217.2 mg) as colourless powder.
¹H-NMR (DMSO-d₆), δ (ppm): 2.02 (2H, m), 2.11 (3H, s), 2.66 (2H, t, J = 7.5 Hz), 2.87 (4H, br), 3.49 (2H, t, J = 7.5 Hz), 6.73 (1H, s), 7.11 (4H, s), 12.08 (1H, s).
MS: 367, 369 (M+H)+

### Step 7

To a solution of N-(4-{2-[4-(3-bromopropyl)phenyl]ethyl}-1,3-thiazol-2-yl)acetamide (175 mg) in DMF (5 ml) was added potassium phthalimide at 25 °C. The mixture was stirred at 50 °C for 2.5 hr and was poured into water. The mixture was extracted with ethyl acetate. The organic layer was washed with brine, dried over MgSO₄, filtered and concentrated *in vacuo*. The residue was purified by silicagel column chromatography with hexane and ethyl acetate (1:1) as an eluent to give N-[4-(2-{4-[3-(1,3-dioxo-1,3-dihydro-2H-isoindol-2-yl)propyl]phenyl}ethyl)-1,3-thiazol-2-yl]acetamide (174.1 mg) as white powder.
¹H-NMR (DMSO-d₆), δ (ppm): 1.95 (2H, m), 2.11 (3H, s), 2.57 (2H, t, J = 7.1 Hz), 2.82 (4H, s), 3.59 (2H, t, J = 6.9 Hz), 6.71 (1H, s), 7.08 (4H, s), 7.83 (3H, s).
MS: 434 (M+H)+

### Step 8

N-[4-(2-{4-[3-(1,3-Dioxo-1,3-dihydro-2H-isoindol-2-yl)propyl]phenyl}ethyl)-1,3-thiazol-2-yl]acetamide (87.1 mg), hydrazine monohydrate (0.174 ml) and MeCN (2 ml) were combined under N₂ atmosphere. The reaction mixture was stirred at 50 °C for 1 hour. After cooled to r.t., the mixture was diluted with CHCl₃. The precipitate was filtered off. The filtrate was concentrated *in vacuo*. The residue was purified by flash column chromatography over NH silica gel with CHCl₃ / MeOH (20:1) as an eluent to give N-(4-{2-[4-(3-aminopropyl)phenyl]ethyl}-1,3-thiazol-2-yl)acetamide (50 mg) as colorless oil.
¹H-NMR (DMSO-d₆), δ (ppm): 1.61 (1H, m), 2.11 (3H, s), 2.56 (2H, m), 2.87 (4H, s), 3.32 (2H, m), 6.72 (1H, s), 7.09 (4H, s).
MS: 304 (M+H)+

### Step 9

Di-tert-butyl ((Z)-{[3-(4-{2-[2-(acetylamino)-1,3-thiazol-4-yl]ethyl}phenyl)propyl]amino}methylidene) biscarbamate was prepared from the compound of Step 8 of Production Example 6 in a manner similar to Step 13 of Production Example 1.
¹H-NMR (DMSO-d₆), δ (ppm): 1.38 (9H, s), 1.47 (9H, s), 1.79 (2H, br), 2.11 (3H, s), 2.55 (2H, br), 2.86 (4H, s), 3.26 (2H, br), 6.72 (1H, s), 7.11 (4H, s), 8.30 (1H, br), 11.45 (1H, s), 12.10 (1H, s).
MS: 546 (M+H)+

### Step 10

The title compound was prepared from the compound of Step 9 of Production Example 6 in a manner similar to Step 14 of Production Example 1.
¹H-NMR (DMSO-d₆), δ (ppm): 1.74 (2H, m), 2.11 (3H, s), 2.58 (2H, t, J = 7.1 Hz), 2.87 (4H, s x2), 3.09 (2H, m), 6.73 (1H, s), 7.12 (4H, s), 7.21 (4H, br), 7.87 (1H, br), 12.10 (1H, s).
MS: 346 (M+H)+

### Production Example 7: Synthesis of N-{4-[4-(4-{[amino(imino)methyl]amino}butyl)phenyl]-1,3-thiazol-2-yl}acetamide hydrochloride

### Step 1

To a solution of 4-phenyl-1-butanol (15 g) in CH₂Cl₂ (150 ml) were added pivaloyl chloride (14.2 ml) and diisopropylethylamine (26.1 ml) at 0°C. The mixture was stirred at 25 °C for 2 hr and poured into water. The mixture was extracted with ethyl acetate. The organic layer was washed with brine, dried over MgSO₄, filtered, concentrated *in vacuo*. The residue was purified by silicagel column chromatography with hexane and ethyl acetate to give 4-phenylbutyl pivalate (21 g) as pale yellow oil.
¹H-NMR (DMSO-d₆), δ (ppm): 1.19 (9H, s), 1.63-1.74 (4H, m), 2.64 (2H, m), 4.06 (2H, m), 7.15-7.33 (5H, m).
MS: m/z 235 (M+H)+

### Step 2

4-[4-(2-Bromoacetyl)phenyl]butyl pivalate was prepared from the compound of Step 1 of Production Example 7 in a manner similar to Step 1 of Production Example 5.
¹H-NMR (DMSO-d₆), δ (ppm): 1.16 (9H, s), 1.68-1.79 (4H, m), 2.72 (2H, t, J = 7.1 Hz), 4.08 (2H, t, J = 5.9 Hz), 4.43 (2H, s), 7.30 (2H, d, J = 8.0 Hz), 7.92 (2H, d, J = 8.0 Hz).
MS: 355, 357 (M+H)+

### Step 3

4-[4-(2-Amino-1,3-thiazol-4-yl)phenyl]butyl pivalate was prepared from the compound of Step 2 of Production Example 7 in a manner similar to Step 2 of Production Example 5.
MS: 333 (M+H)+

### Step 4

4-{4-[2-(Acetylamino)-1,3-thiazol-4-yl]phenyl}butyl pivalate was prepared from the compound of Step 3 of Production Example 7 in a manner similar to Step 7 of Production Example 1.
¹H-NMR (DMSO-d₆), δ (ppm): 1.19 (9H, s), 1.69 (4H, m), 2.67 (2H, br), 4.08 (2H, br), 7.23 (2H, d, J = 8.0 Hz), 7.72 (2H, d, J = 8.0 Hz), 10.8 (1H, br).
MS: m/z 375 (M+H)

### Step 5

To a solution of 4-{4-[2-(acetylamino)-1,3-thiazol-4-yl]phenyl}butyl pivalate (1.5 g) in MeOH (10 ml) was added sodium methoxide in MeOH (28 %, 0.89 ml) under ice-cooling. This was stirred at 45 °C for 12 hr. The organic solvent was evaporated to reduced volume and aq. 1N HCl (10 ml) was added to the residue at 5 °C. The mixture was extracted with ethylacetate. The organic layer was dried over MgSO₄, filtered, and concentrated *in vacuo*. The residue was triturated with IPE to give N-{4-[4-(4-hydroxybutyl)phenyl]-1,3-thiazol-2-yl}acetamide as white powder.
¹H-NMR (DMSO-d₆), δ (ppm): 1.37-1.68 (4H, m), 2.15 (9H, s), 2.59 (2H, t, J = 7.4 Hz), 3.41 (2H, t, J = 6.3 Hz), 3.81 (1H, br), 7.24 (2H, d, J = 8.0 Hz), 7.79 (1H, d, J = 8.0 Hz), 12.22 (1H, s).
MS: m/z 291 (M+H)

### Step 6

N-{4-[4-(4-Bromobutyl)phenyl]-1,3-thiazol-2-yl}acetamide was prepared from the compound of Step 5 of Production Example 7 in a manner similar to Step 6 of Production Example 6.
¹H-NMR (DMSO-d₆), δ (ppm): 1.63-1.89 (4H, m), 2.15 (3H, s), 2.62 (2H, t, J = 7.2 Hz), 3.56 (2H, t, J = 6.4 Hz), 7.25 (2H, d, J = 8.0 Hz), 7.80 (2H, d, J = 8.0 Hz), 12.22 (1H, s).
MS: 353, 355 (M+H)+

### Step 7

N-(4-{4-[4-(1,3-Dioxo-1,3-dihydro-2H-isoindol-2-yl)butyl]phenyl}-1,3-thiazol-2-yl)acetamide was prepared from the compound of Step 6 of Production Example 7 in a manner similar to Step 7 of Production Example 6.
¹H-NMR (DMSO-d₆), δ (ppm): 1.62 (4H, br), 2.15 (3H, s), 2.62 (2H, br), 3.60 (2H, br), 7.27 (2H, d), 7.51 (1H, s), 7.77 (2H, d), 7.82-7.89 (4H, m), 12.22 (1H, s).
MS: m/z 420 (M+H)

### Step 8

N-{4-[4-(4-Aminobutyl)phenyl]-1,3-thiazol-2-yl}acetamide was prepared from the compound of Step 7 of Production Example 7 in a manner similar to Step 8 of Production Example 6.
MS: m/z 290 (M+H)

### Step 9

Di-tert-butyl {(E)-[(4-{4-[2-(acetylamino)-1,3-thiazol-4-yl]phenyl}butyl)amino]methylidene}biscarbamate was prepared from the compound of Step 8 of Production Example 7 in a manner similar to Step 13 of Production Example 1.
¹H-NMR (DMSO-d₆), δ (ppm): 1.37 (9H, s), 1.47 (9H, s), 1.56 (4H, m), 2.15 (3H, s), 2.62 (2H, m), 3.30 (2H, m), 7.25 (2H, d), 7.51 (1H, s), 7.29 (2H, d), 8.30 (1H, t, J = 1.2 Hz), 11.49 (1H, s), 12.21 (1H, s).
MS: m/z 532 (M+H)

### Step 10

The title compound was prepared from the compound of Step 9 of Production Example 7 in a manner similar to Step 14 of Production Example 1.
¹H-NMR (DMSO-d₆), δ (ppm): 1.13-1.20 (4H, m), 2.16 (3H, s), 2.62 (2H, t, J = 7.2 Hz), 3.14 (2H, m), 7.23 (2H, d, J = 8.0 Hz), 7.53 (1H, s), 7.68 (2H, m), 7.80 (2H, d, J = 8.0 Hz), 12.23 (1H, s).
MS: m/z 332 (M+H) free

### Production Example 8: Synthesis of N-(4-{2-[3-(2-{[amino(imino)methyl]amino}ethyl)phenyl]ethyl}-1,3-thiazol-2-yl)acetamide hydrochloride

### Step 1

To a suspension of lithium aluminum hydride in dry tetrahydrofuran (50 ml) was added (3-bromophenyl)acetic acid (10 g) in tetrahydrofuran (100 ml) under ice cooling. The mixture was refluxed for 2 hurs. After cooling, to the reaction mixture were added water and aqueous Rochelle salt. The mixture was stirred for another 30 min. Aqueous layer was extracted with ethyl acetate. The organic layer was dried over anhydrous magnesium sulfate, and concentrated *in vacuo* to give 2-(3-bromophenyl)ethanol. This compound was used for the next reaction without further purification.
¹H-NMR (200 MHz, CDCl₃), δ (ppm): 1.66 (1H, brs), 2.84 (2H, dd, J=6.5, 14Hz), 3.85 (2H, dt, J=6.5, 2.6Hz), 7.13-7.39 (4H, m).

### Step 2

To a solution of 2-(3-bromophenyl)ethanol (7 g) in N,N-dimethylformamide (100 ml) were added tert-butyldimethylsilyl chloride (5.77 g) and imidazole (2.84 g) at 25 °C. The mixture was stirred at 25 °C for 12 hr. The reaction mixture was poured into water (500 ml) and extracted with ethyl acetate (100 ml x2). The combined organic layer was dried over magnesium sulfate and concentrated *in vacuo*. The residue was purified by silica gel column chromatography with mixed solvent of n-hexane and ethyl acetate to give [2-(3-bromophenyl)ethoxy](tert-butyl)dimethylsilane as colorless oil.
¹H-NMR (200 MHz, CDCl₃), δ (ppm): 0.01 (6H, s), 0.88 (9H, s), 2.81 (2H, dt, J=6.5, 9.5Hz), 3.81 (2H, dt, J=3.0, 6.5Hz), 7.14-7.39 (5H, brs).

### Step 3

To the solution of [2-(3-bromophenyl)ethoxy](tert-butyl)dimethylsilane (2.45 g) in THF (25 ml) was added n-butyl lithium (1.57 M in hexane, 5.58 ml) at -75 °C, and the mixture was stirred at same temperature for 1 hr. Then, DMF (1.69 ml) was added at the same temperature, and the mixture was stirred for 2 hr. The reaction was quenched with aq. ammonium chloride and allowed to room temperature. The mixture was extracted with ethyl acetate. The organic layer was washed with brine, dried over MgSO₄, filtered, and concentrated *in vacuo*. The residue was purified by silicagel column chromatography with hexane and ethyl acetate (20/1 - 10/1) to give 3-(2-{[tert-butyl(dimethyl)silyl]oxy}ethyl)-benzaldehyde (0.8 g) as colourless oil.
¹H-NMR (DMSO-d₆), δ (ppm): 0.01 (6H, s), 0.85 (9H, s), 2.89 (2H, t, J = 6.6 Hz), 3.83 (2H, t, J = 6.6 Hz), 7.44-7.58 (2H, m), 7.71-7.74 (2H, m), 10.00 (1H, s).

### Step 4

N-(4-{(E)-2-[3-(2-{[tert-Butyl(dimethyl)silyl]oxy}ethyl)-phenyl]vinyl}-1,3-thiazol-2-yl)acetamide was prepared from the compound of Step 3 of Production Example 8 in a manner similar to Step 3 of Production Example 2.
¹H-NMR (DMSO-d₆), δ (ppm): 0.00 (6H, s), 0.87 (9H, s), 2.22 (3H, s), 2.83 (2H, t), 3.82 (2H, t), 6.84-7.34 (7H, m), 10.10 (1H, br).
MS: 403 (M+H)+

### Step 5

To the solution of N-(4-{(E)-2-[3-(2-{[tert-butyl(dimethyl)silyl]oxy}ethyl)phenyl]vinyl}-1,3-thiazol-2-yl)acetamide (920 mg) in THF (10 ml) was added tetrabutylammonium fluoride (1 M in THF solution, 4.6 ml) at 0 °C. This was stirred at 25 °C for 2 hr and was poured into water. The mixture was extracted with ethyl acetate. The organic layer was washed with brine, dried over MgSO₄, filtered, and concentrated in *vacuo* to give N-(4-{(E)-2-[3-(2-hydroxyethyl)phenyl]vinyl}-1,3-thiazol-2-yl)acetamide (660 mg) as crude oil. This was used for the next reaction without further purification.
¹H-NMR (DMSO-d₆), δ (ppm): 2.22 (3H, s), 2.89 (2H, t, J = 6.5 Hz), 3.25 (1H, m), 3.89 (2H, t, J = 6.5 Hz), 6.84-7.72 (7H, m), 10.00 (1H, br).
MS: 289 (M+H)+

### Step 6

N-(4-{2-[3-(2-Hydroxyethyl)phenyl]ethyl}-1,3-thiazol-2-yl)acetamide was prepared from the compound of Step 5 of Production Example 8 in a manner similar to Step 11 of Production Example 2.
¹H-NMR (DMSO-d₆), δ (ppm): 2.24 (3H, s), 2.84 (2H, t, J = 6.4 Hz), 2.95 (4H, s), 3.85 (2H, t, J = 6.4 Hz), 6.50 (1H, s), 7.01-7.07 (3H, m), 7.18-7.22 (1H, m).
MS: 291 (M+H)+

### Step 7

N-(4-{2-[3-(2-Hydroxyethyl)phenyl]ethyl}-1,3-thiazol-2-yl)acetamide (300 mg), methanesulfonyl chloride (0.12 ml), diisopropyl ethylamine (0.54 ml) and CH₂Cl₂ (7 ml) were combined at 0 °C under N₂ atmosphere. The reaction mixture was stirred at room temperature for 1 hour, and the precipitate was filtered off. The filtrate was concentrated *in vacuo*. The residue was dissolved in DMF. To the solution were added potassium phthalimide (287 mg) and DMF (5 ml) combined under N₂ atmosphere. The reaction mixture was stirred at 50 °C for 3 hours. After cooled to r.t., AcOEt and 1N-HCl were added to the reaction mixture. The organic layer was washed with water, saturated NaHCO₃ and brine, dried over anhydrous MgSO₄, and concentrated *in vacuo*. The residue was purified by flash column chromatography over silica gel with hexane / ethyl acetate (2/3) as an eluent to give N-[4-(2-{3-[2-(1,3-dioxo-1,3-dihydro-2H-isoindol-2-yl)ethyl]phenyl}ethyl)-1,3-thiazol-2-yl]acetamide (433.6 mg) as white powder.
¹H-NMR (DMSO-d₆), δ (ppm): 2.31 (3H, s), 2.85-2.99 (6H, m), 3.89 (2H, dd, J = 7.8, 6.2 Hz), 6.46 (1H, s), 6.97-7.17 (4H, m), 7.72 (2H, m), 7.82 (2H, m).
MS: 420 (M+H)+

### Step 8

N-(4-{2-[3-(2-Aminoethyl)phenyl]ethyl}-1,3-thiazol-2-yl)acetamide was prepared from the compound of Step 7 of Production Example 8 in a manner similar to Step 8 of Production Example 6.
¹H-NMR (DMSO-d₆), δ (ppm): 2.11 (3H, s), 2.61 (2H, m), 2.75 (2H, m), 2.88 (4H, s), 6.72 (1H, s), 6.99-7.21 (4H, m).
MS: 290 (M+H)+

### Step 9

Di-tert-butyl ((Z)-{[2-(3-{2-[2-(acetylamino)-1,3-thiazol-4-yl]ethyl}phenyl)ethyl]amino}methylidene) biscarbamate was prepared from the compound of Step 8 of Production Example 8 in a manner similar to Step 13 of Production Example 1.
¹H-NMR (DMSO-d₆), δ (ppm): 1.46 (9H, s), 1.50 (9H, s), 2.27 (3H, s), 2.84 (2H, t, J = 7.0 Hz), 2.95 (4H, s), 3.66 (2H, dd, J = 7.0, 6.0 Hz), 5.63 (1H, dd, J = 5.0, 1.5 Hz), 6.47 (1H, s), 6.98-7.05 (3H, m), 7.15-7.23 (1H, m), 8.39 (1H, br), 11.50 (1H, br).
MS: 532 (M+H)+

### Step 10

The title compound was prepared from the compound of Step 9 of Production Example 8 in a manner similar to Step 14 of Production Example 1.
¹H-NMR (DMSO-d₆), δ (ppm): 2.12 (3H, s), 2.75 (2H, t, J = 7.3 Hz), 2.94 (4H, br), 3.35 (2H, dt, J = 7.3, 6.2 Hz), 6.74 (1H, s), 7.04-7.25 (8H, m), 7.70 (1H, t, J = 5.4 Hz), 12.09 (1H, br).
MS: m/z 332 (M+H) free

### Production Example 9: Synthesis of 2-(4-{2-[2-(acetylamino)-1,3-thiazol-4-yl]ethyl}phenyl)-N-[amino(imino)methyl]acetamide

### Step 1

A mixture of 3-chloro-2-oxopropyl acetate (5 g) and thiourea (2.5 g) in ethanol (25 ml) was refluxed for 4 hours. The reaction mixture was cooled to ambient temperature and the resulting crystalline precipitate was collected by filtration and washed with ethanol (20 ml) to give (2-amino-1,3-thiazol-4-yl)methyl acetate hydrochloride (3.5 g) as white crystals.
¹H-NMR (DMSO-d₆), δ (ppm): 2.07 (3H, s), 4.92 (2H, s), 6.87 (1H, s).
MS: 173 (M+H)⁺

### Step 2

To a mixture of (2-amino-1,3-thiazol-4-yl)methyl acetate hydrochloride (56 g) and pyridine (45 g) in dichloromethane (560 ml) was added acetyl chloride (23 g) over a period of 30 minutes at 5°C, and the reaction mixture was stirred for 10 minutes at the same temperature. The reaction mixture was poured into water (500 ml) and extracted with chloroform (1 L). The organic layer was dried over sodium sulfate and concentrated *in vacuo*. The residual solid was collected by filtration with isopropyl ether to give (2-(acetylamino)-1,3-thiazol-4-yl)methyl acetate (47 g) as white crystals.
¹H-NMR (CDCl₃), δ (ppm): 2.12 (3H, s), 2.29 (3H, s), 5.08 (2H, s), 6.93 (1H, s).
MS: 215 (M+H)⁺

### Step 3

A mixture of (2-(acetylamino)-1,3-thiazol-4-yl)methyl acetate (46 g) and potassium carbonate (30 g) in methanol (640 ml) was stirred for 3 hours at ambient temperature. The reaction mixture was concentrated *in vacuo*. The residue was diluted with chloroform, and the insoluble material was filtered off. The resulting solution was purified by flash column chromatography on silica-gel with methanol / chloroform (1/99). The resulted solid was collected by filtration with isopropyl ether to give N-(4-(hydroxymethyl)-1,3-thiazol-2-yl)acetamide (35 g) as white crystals.
¹H-NMR (DMSO-d₆), δ (ppm): 2.12 (3H, s), 4.44 (2H, d, J=5.0Hz), 5.20 (1H, t, J=5.0Hz), 6.88 (1H, s), 12.02 (1H, brs).
MS: 173 (M+H)⁺

### Step 4

N-(4-(Hydroxymethyl)-1,3-thiazol-2-yl)acetamide (2.8 g) was dissolved in methanol (10 ml) and chloroform (200 ml). Then, manganese (IV) oxide (28.3 g) was added to the solution under nitrogen atmosphere. The reaction mixture was stirred at room temperature for 7 hours, and filtered through a celite pad. The filtrate was concentrated *in vacuo*. The resulting solid was washed with ethyl ether to give N-(4-formyl-1,3-thiazol-2-yl)acetamide (2.01 g) as an off-white solid.
mp. 195.5-199°C
¹H-NMR (DMSO-d₆), δ (ppm): 2.17 (3H, s), 8.28(1H, s), 9.79(1H, s), 12.47(1H, brs).

### Step 5

To the solution of [4-(bromomethyl)phenyl]acetic acid (5.0 g) in toluene (50 ml) was added triphenylphosphine (5.8 g) at 25 °C. This was refluxed for 5 h. After cooling to room temperature, the resulting colourless precipitate was collected by filtration and washed with IPE to give [4-(carboxymethyl)-benzyl](triphenyl)phosphonium bromide (10.7 g) as white powder.
¹H-NMR (DMSO-d₆), δ (ppm): 3.52 (2H, s), 5.13 (2H, d, J = 15.6 Hz), 6.90 (2H, dd, J = 8.1, 2.3 Hz), 7.11 (2H, d, J = 8.1 Hz), 7.58-7.91 (15H, m) .
MS: 411 (M+H)+

### Step 6

To the solution of [4-(carboxymethyl)benzyl](triphenyl) phosphonium bromide (19.1 g) in DMF (180 ml) was added potassium tert-butoxide (11.9 g) under ice-cooling. This was stirred at 5 °C for 30 min. To the solution was added N-(4-formyl-1,3-thiazol-2-yl)acetamide (6.0 g) in DMF (18 ml). This was stirred at 25 °C for 3 hr. The mixture was poured into water and was extracted with ethyl acetate. The aquaous phase was acidified (pH 4-5) with 1N HCl to give colourless precipitate. The precipitate was collected by filtration to give a mixture of (4-{(E)-2-[2-(acetylamino)-1,3-thiazol-4-yl]vinyl}phenyl)acetic acid and (4-{(Z)-2-[2-(acetylamino)-1,3-thiazol-4-yl]vinyl}phenyl)acetic acid (10 g) as white powder.
¹H-NMR (DMSO-d₆), δ (ppm): 2.12, 2.14 (3x5/6, 3x1/6H, s), 3.52, 3.54 (2x5/6, 2x1/6H, s), 6.46 (5/6H, d, J = 12.7 Hz), 6.54 (5/6H, d, J = 12.7 Hz), 6.95 (1H, s), 7.11-7.49 (4+1/6H, m), 12.09 (1H, br).
MS: 303 (M+H)+

### Step 7

(4-{2-[2-(Acetylamino)-1,3-thiazol-4-yl]ethyl}phenyl)acetic acid was prepared from the compound of Step 6 of Production Example 9 in a manner similar to Step 11 of Production Example 2.
¹H-NMR (DMSO-d₆), δ (ppm): 2.11 (3H, s), 2.88 (4H, s), 3.50 (2H, s), 6.74 (1H, s), 7.14 (4H, s), 12.08 (1H, s).
MS: m/z 305 (M+H)

### Step 8

To a solution of (4-{2-[2-(acetylamino)-1,3-thiazol-4-yl]ethyl}phenyl)acetic acid (2.0 g) in DMF (15 ml) was added N,N'-carbonyldiimidazole (1.6 g). The mixture was stirred at 50 °C for 2 hr, To the mixture was added a solution of guanidine hydrochloride (3.1 g) and sodiume methoxyde (28 % MeOH solution, 6.4 ml) in DMF (5 ml) at 25 °C. The reaction mixture was stirred at 25 °C for 12 hr. The organic solvent was evaporated to reduced volume and the residue was poured into water. To the mixture was added 1N HCl to adjust pH to 8.

The mixture was extracted with ethyl acetate. The organic layer was dried over MgSO₄, filtered, and concentrated *in vacuo*. The residue was purified by silicagel (amine coated) column chromatography with CH₂Cl₂ and MeOH (10/1) as an eluent to give 2-(4-{2-[2-(acetylamino)-1,3-thiazol-4-yl]ethyl}phenyl)-N-[amino(imino)methyl]acetamide (1.4 g) as colourless powder.
¹H-NMR (DMSO-d₆), δ (ppm): 2.10 (3H, s), 2.86 (4H, m), 3.34 (2H, s), 6.74 (1H, s), 7.08 (2H, d, J=8.1 Hz), 7.12 (2H, d, J=8.1 Hz), 12.22 (1H, br).
MS: m/z 346 (M+H)

### Production Example 10: Synthesis of N-[4-(2-{4-[(2-{[amino(imino)methyl]amino}ethyl)amino]phenyl}ethyl)-1,3-thiazol-2-yl]acetamide dihydrochloride

### Step 1

A mixture of 3-chloro-2-oxopropyl acetate (5 g) and thiourea (2.5 g) in ethanol (25 ml) was refluxed for 4 hours. The reaction mixture was cooled to ambient temperature and the resulting crystalline precipitate was collected by filtration and washed with ethanol (20 ml) to give (2-amino-1,3-thiazol-4-yl)methyl acetate hydrochloride (3.5 g) as white crystals.
¹H-NMR (DMSO-d₆), δ (ppm): 2.07 (3H, s), 4.92 (2H, s), 6.87 (1H, s).
MS: 173 (M+H)⁺

### Step 2

To a mixture of (2-amino-1,3-thiazol-4-yl)methyl acetate hydrochloride (56 g) and pyridine (45 g) in dichloromethane (560 ml) was added acetyl chloride (23 g) over a period of 30 minutes at 5°C, and the reaction mixture was stirred for 10 minutes at the same temperature. The reaction mixture was poured into water (500 ml) and extracted with chloroform (1 L). The organic layer was dried over sodium sulfate and concentrated *in vacuo*. The residual solid was collected by filtration with isopropyl ether to give (2-(acetylamino)-1,3-thiazol-4-yl)methyl acetate (47 g) as white crystals.
¹H-NMR (CDCl₃), δ (ppm): 2.12 (3H, s), 2.29 (3H, s), 5.08 (2H, s), 6.93 (1H, s).
MS: 215 (M+H)⁺

### Step 3

A mixture of (2-(acetylamino)-1,3-thiazol-4-yl)methyl acetate (46 g) and potassium carbonate (30 g) in methanol (640 ml) was stirred for 3 hours at ambient temperature. The reaction mixture was concentrated *in vacuo*. The residue was diluted with chloroform, and the insoluble material was filtered off. The resulting solution was purified by flash column chromatography on silica-gel with methanol / chloroform (1/99). The resulted solid was collected by filtration with isopropyl ether to give N-(4-(hydroxymethyl)-1,3-thiazol-2-yl)acetamide (35 g) as white crystals.
¹H-NMR (DMSO-d₆), δ (ppm): 2.12 (3H, s), 4.44 (2H, d, J=5.0Hz), 5.20 (1H, t, J=5.0Hz), 6.88 (1H, s), 12.02 (1H, brs).
MS: 173 (M+H)⁺

### Step 4

N-(4-(Hydroxymethyl)-1,3-thiazol-2-yl)acetamide (2.8 g) was dissolved in methanol (10 ml) and chloroform (200 ml). Then, manganese (IV) oxide (28.3 g) was added to the solution under nitrogen atmosphere. The reaction mixture was stirred at room temperature for 7 hours, and filtered through a celite pad. The filtrate was concentrated *in vacuo*. The resulting solid was washed with ethyl ether to give N-(4-formyl-1,3-thiazol-2-yl)acetamide (2.01 g) as an off-white solid.
mp. 195.5-199 °C
¹H-NMR (DMSO-d₆), δ (ppm): 2.17 (3H, s), 8.28 (1H, s), 9.79 (1H, s), 12.47 (1H, brs).

### Step 5

1-(Bromomethyl)-4-nitrobenzene (1.9 g), triphenylphosphine (2.31 g) and N,N-dimethylformamide (20 ml) were combined under nitrogen atmosphere. The reaction mixture was stirred at room temperature for 2.5 hours. Then, potassium tert-butoxide (1.19 g) and N-(4-formyl-1,3-thiazol-2-yl)acetamide (1.5 g) were added and the mixture was stirred at room temperature for 14 hours. The reaction mixture was poured into ice-water and extracted with ethyl acetate. The organic layer was washed with 1N-hydrochloric acid, water and saturated sodium chloride solution, dried over anhydrous magnesium sulfate, and concentrated *in vacuo*. The residue was purified by flash column chromatography over silica gel with n-hexane / ethyl acetate (1:1) → (1:2) as an eluent, and triturated with ethyl ether to give N-{4-[(Z)-2-(4-nitrophenyl)ethenyl]-1,3-thiazol-2-yl}acetamide (1.59 g) as a yellow solid.
mp. 155-157 °C
¹H-NMR (DMSO-d₆), δ (ppm): 2.13 (3H, s), 6.64 (1H, d, J=12.5Hz), 6.71 (1H, d, J=12.5Hz), 7.18 (1H, s), 7.79 (2H, d, J=9.0Hz), 8.17 (2H, d, J=9.0Hz), 12.02 (1H, brs).
MS: 290 (M+H)⁺

### Step 6

A mixture of N-{4-[(Z)-2-(4-nitrophenyl)ethenyl]-1,3-thiazol-2-yl}acetamide (2 g) and 10% palladium on carbon (400 mg) in methanol (25 ml), tetrahydrofuran (25 ml) and acetic acid (18 ml) was stirred under 4 atm hydrogen at ambient temperature for 5 hours. The reaction mixture was filtered through a celite pad, and the filtrate was concentrated *in vacuo*. The residue was dissolved in ethyl acetate. The organic solution was washed with saturated sodium hydrogen carbonate solution and saturated sodium chloride solution, dried over anhydrous magnesium sulfate, and concentrated *in vacuo*. The residue was purified by flash column chromatography over silica gel with n-hexane / ethyl acetate (1:2) → ethyl acetate as an eluent, and triturated with ethyl alcohol / ethyl ether to give N-{4-[2-(4-aminophenyl)ethyl]-1,3-thiazol-2-yl}acetamide (539.6 mg) as an off-white solid.
mp. 102.5-104 °C
¹H-NMR (DMSO-d₆), δ (ppm): 2.11 (3H, s), 2.75 (4H, brs), 4.82 (2H, s), 6.46 (2H, d, J=8.5Hz), 6.69 (1H, s), 6.83 (2H, d, J=8.5Hz), 12.07 (1H, brs).
MS: 262 (M+H)⁺

### Step 7

To a suspension of N-{4-[2-(4-aminophenyl)ethyl]-1,3-thiazol-2-yl}acetamide (100mg) in toluene were added tert-butyl (2-bromoethyl)carbamate (87.5 mg) and N,N-diisopropylethylamine (52 µl), and the mixture was stirred at 80 °C for 24 hr. The reaction mixture was allowed to cool to room temperature, water (10 ml) was added, and the organic layer was separated, washed with saturated aqueous NaCl solution, dried over MgSO₄, filtered, and concentrated in *vacuo* to give tert-butyl {2-[(4-{2-[2-(acetylamino)-1,3-thiazol-4-yl]ethyl}phenyl)amino]ethyl}carbamate (41.0 mg) as pale brown amorphous.
¹H-NMR (CDCl₃) δ (ppm): 1.45(9H, s), 2.23(3H, s), 2.86(4H, s), 3.15-3.28(2H, m), 3.15-3.47(2H, m), 4.64-5.02(1H, brs), 6.49(1H, s), 6.52(2H, d, J=8.0Hz), 6.95(2H, d, J=8.0Hz), 9.22-10.10(1H, brs).
MS: 405.2(M+H)+, 427.3(M+Na)+

### Step 8

tert-Butyl {2-[(4-{2-[2-(acetylamino)-1,3-thiazol-4-yl]ethyl}phenyl)amino]ethyl}carbamate (50.7 mg) and 4N-HCl in dioxane (2 ml) were combined under N₂ atmosphere. The reaction mixture was stirred at 20 °C for 1hr. The solvent was removed *in vacuo*. The residue was solidified with AcOEt to give N-[4-(2-{4-[(2-aminoethyl)amino]phenyl}ethyl)-1,3-thiazol-2-yl]acetamide dihydrochloride (28.9 mg) as a pale brown solid.
¹H-NMR (DMSO-d₆) δ (ppm): 2.11(3H, s), 2.81(4H, s), 2.92-3.05(2H, m), 3.29(2H, t, J=6.2Hz), 6.67(2H, d, J=7.7Hz), 7.01(2H, d, J=8.1Hz), 7.87-8.24(3H, brs), 12.08(1H, s).
MS: 305.2(M+H)+, 327.2(M+Na)+ Free

### Step 9

N-[4-(2-{4-[(2-Aminoethyl)amino]phenyl}ethyl)-1,3-thiazol-2-yl]acetamide dihydrochloride (20.3 mg), N,N'-bis(tert-butoxycarbonyl)-1H-pyrazole-1-carboxamidine (16.7 mg), N,N-diisopropylethylamine (28.1 µl), THF (0.5 ml) and DMF (0.1 ml) were combined under N₂ atmosphere, and the mixture was stirred at 15 °C for 14 hr. Volatiles were evapolated and the residue was dissolved in MeOH (0.5 ml). The reaction mixture was stirred at 20 °C for 3 hr, and then AcOEt (20 ml) was added, and the mixture was washed with water and brine, dried over MgSO₄, and evaporated to give crude yellow oil (26.9 mg). The crude oil was purified by preparative silica gel thin-layer chromatography with chloroform / methanol (20:1) as an eluent to give di-tert-butyl [(Z)-({2-[(4-{2-[2-(acetylamino)-1,3-thiazol-4-yl]ethyl}phenyl)amino]ethyl}amino)methylidene]biscarbamate as pale yellow oil (23.8 mg).
¹H-NMR (200MHz, CDCl₃) δ (ppm): 1.48 (9H, s), 1.52 (9H, s), 2.22 (3H, s), 2.75 - 3.01 (4H, m), 3.20 - 3.38 (2H, m), 3.55 - 3.76 (2H, m), 4.15 - 4.68 (1H, brs), 6.51 (1H, s), 6.55 (2H, d, J = 8.4Hz ), 6.97 (2H, d, J = 8.4Hz), 8.56 (1H, t, J=5.5Hz), 9.91 - 10.46 (1H, brs), 11.46 (1H, s).
MS: 547.28 (M+H)+

### Step 10

The title compound was prepared from the compound of Step 9 of Production Example 10 in a manner similar to Step 15 of Production Example 2.
¹H-NMR (400MHz, DMSO-d₆) δ (ppm): 2.11 (3H, s), 2.81 (4H, s), 3.15 - 3.23 (2H, m), 3.28 - 3.38 (2H, m), 6.6 - 6.75 (3H, m), 7 (2H, d, J = 8Hz), 7.17 (4H, brs), 7.62 (1H, t, J = 5.1Hz), 12.07 (1H, s).
MS: 347.2 (M+H)+ free

### Production Example 11: Synthesis of N-[4-(2-{2-(acetylamino)-5-[4-(methylsulfonyl)benzyl]-1,3-thiazol-4-yl}ethyl)phenyl]-2-{[amino(imino)methyl]amino}acetamide hydrochloride

### Step 1

3-(4-Mercaptophenyl)propanoic acid (5 g), K₂CO₃ (11.4 g) and DMF (30 ml) were combined, and iodomethane (5.12 ml) was added dropwise to the mixture at 0 °C under N₂ atmosphere. The reaction mixture was stirred at r.t. for 13 hours, and poured into ice-water. The mixture was extracted with AcOEt. The organic layer was washed with water (twice) and brine, dried over anhydrous MgSO₄, and concentrated *in vacuo* to give methyl 3-[4-(methylthio)phenyl]propanoate (4.19 g) as pale yellow oil.
¹H-NMR (CDCl₃), δ (ppm): 2.47 (3H, s), 2.61 (2H, t, J=8.0Hz), 2.91 (2H, t, J=8.0Hz), 3.67 (3H, s), 7.12 (2H, d, J=8.5Hz), 7.20 (2H, d, J=8.5Hz).

### Step 2

28% Sodium methoxide solution in MeOH (3.67 ml) were added dropwise to the mixture of methyl 3-[4-(methylthio)phenyl]propanoate (4 g) and diethyl oxalate (5.17 ml) at 0 °C with stirring. The reaction mixture was stirred at 65 °C for 30 minutes under reduced pressure. 15% Aqueous H₂SO₄ (35 ml) was added to the mixture, and the mixture was refluxed for 15 hours. After cooled to r.t., the mixture was extracted with AcOEt. The organic layer was washed with water and brine, dried over anhydrous MgSO₄ and concentrated *in vacuo*. The residual oil was dissolved in EtOH (20 ml), and conc. H₂SO₄ (0.4 ml) was added dropwise to the solution. The reaction mixture was refluxed for 2 hours. After cooled to r.t., EtOH was removed *in vacuo*. AcOEt and water were added to the residue, and the mixture was extracted. The organic layer was washed with water and brine, dried over anhydrous MgSO₄, and concentrated *in vacuo*. The residue was purified by flash column chromatography over silica gel with *n*-hexane / AcOEt (6:1) as an eluent to give ethyl 4-[4-(methylthio)phenyl]-2-oxobutanoate (2.43 g) as a yellow liquid.
¹H-NMR (CDCl₃), δ (ppm): 1.35 (3H, t, J=7.0Hz), 2.46 (3H, s), 2.92 (2H, t, J=7.0Hz), 3.16 (2H, t, J=7.0Hz), 4.31 (2H, q, J=7.0Hz), 7.13 (2H, d, J=8.5Hz), 7.20 (2H, d, J=8.5Hz).

### Step 3

To a suspension of copper(II) bromide (6.11 g) in AcOEt (110 ml) was added a solution of ethyl 4-[4-(methylthio)phenyl]-2-oxobutanoate (2.3 g) in 55 ml of CHCl₃. The reaction mixture was refluxed for 17 hours, cooled to r.t., and filtered through a short pad of silica gel eluting with AcOEt / n-hexane (1:1). The solvent was removed in *vacuo* to give ethyl 3-bromo-4-[4-(methylthio)phenyl]-2-oxobutanoate (2.56 g) as yellow oil.
¹H-NMR (CDCl₃), δ (ppm): 1.37 (3H, t, J=7.0Hz), 2.47 (3H, s), 3.20 (1H, dd, J=14.5, 7.5Hz), 3.49 (1H, dd, J=14.5, 7.5Hz), 4.35 (2H, q, J=7.0Hz), 5.22 (1H, d, J=7.5Hz), 7.17 (2H, d, J=8.5Hz), 7.20 (2H, d, J=8.5Hz).

### Step 4

Ethyl 3-bromo-4-[4-(methylthio)phenyl]-2-oxobutanoate (2.4 g) was dissolved in EtOH (40 ml), and then thiourea (1.1 g) was added to the solution. The reaction mixture was refluxed for 1 hour under N₂ atmosphere. The cooled reaction mixture was evaporated *in vacuo*. Saturated NaHCO₃ and water were added to the residue, and the mixture was extracted with AcOEt. The organic layer was washed with water and brine, dried over anhydrous MgSO₄, and concentrated *in vacuo*. The residue was purified by flash column chromatography over silica gel with CHCl₃ / MeOH (20:1) as an eluent to give ethyl 2-amino-5-[4-(methylthio)benzyl]-1,3-thiazole-4-carboxylate (2.01 g) as yellow amorphous.
¹H-NMR (DMSO-d₆), δ (ppm): 1.25 (3H, t, J=7.0Hz), 2.44 (3H, s), 4.20 (2H, q, J=7.0Hz), 4.28 (2H, s), 7.02 (2H, s), 7.19 (4H, s).
MS: 309 (M+H)⁺

### Step 5

Ethyl 2-amino-5-[4-(methylthio)benzyl]-1,3-thiazole-4-carboxylate (1.9 g) was dissolved in CH₂Cl₂ (38 ml) and pyridine (1.05 ml), and then acetyl chloride (0.482 ml) was added dropwise to the solution at 0 °C under N₂ atmosphere. The reaction mixture was stirred at r.t. for 1 hour. The organic solution was washed with 1N-HCl water and brine, dried over anhydrous MgSO₄, and concentrated *in vacuo*. The residual solid was washed with IPE to give ethyl 2-(acetylamino)-5-[4-(methylthio)benzyl]-1,3-thiazole-4-carboxylate (2.01 g) as an off-white solid.
mp. 205-206 °C
¹H-NMR (DMSO-d₆), δ (ppm): 1.28 (3H, t, J=7.0Hz), 2.09 (3H, s), 2.45 (3H, s), 4.27 (2H, q, J=7.0Hz), 4.43 (2H, s), 7.22 (4H, s), 12.41 (1H, s).
MS: 351 (M+H)⁺

### Step 6

Ethyl 2-(acetylamino)-5-[4-(methylthio)benzyl]-1,3-thiazole-4-carboxylate (1.0 g) was dissolved in THF (20 ml), and then lithium borohydride (124 mg) was added portionwise to the solution at 0 °C. The reaction mixture was refluxed for 4.5 hours and the reaction was quenched with MeOH. The mixture was concentrated *in vacuo*, and purified by flash column chromatography over silica gel with CHCl₃ / MeOH (20:1) as an eluent. The residual off-white solid (548.5 mg) was dissolved in MeOH (2 ml) and CHCl₃ (20 ml). Then, manganase(IV) oxide (2.48 g) was added to the solution under N₂ atmosphere. The reaction mixture was stirred at r.t. for 12 hours, and filtered through a celite pad. The filtrate was concentrated *in vacuo*. The residue was purified by flash column chromatography over silica gel with CHCl₃ / MeOH (20:1) as an eluent to give N-{4-formyl-5-[4-(methylthio)benzyl]-1,3-thiazol-2-yl}acetamide (500.1 mg) as a yellow wax.
¹H-NMR (DMSO-d₆), δ (ppm): 2.12 (3H, s), 2.45 (3H, s), 4.48 (2H, s), 7.23 (4H, s), 10.03 (1H, s), 12.33 (1H, s).
MS: 307 (M+H)⁺

### Step 7

1-(Bromomethyl)-4-nitrobenzene (564 mg), triphenylphosphine (685 mg) and DMF (9 ml) were combined under N₂ atmosphere. The reaction mixture was stirred at r.t. for 2 hours. Then, potassium tert-butoxide (345 mg) and N-{4-formyl-5-[4-(methylthio)benzyl]-1,3-thiazol-2-yl}acetamide (470.5 mg) were added to the mixture, and the mixture was stirred at r.t. for 2 hours. The reaction mixture was poured into ice-water, and extracted with AcOEt. The organic layer was washed with water and brine, dried over anhydrous MgSO₄, and concentrated *in vacuo*. The residue was purified by flash column chromatography over silica gel with CHCl₃ / AcOEt (1:1) as an eluent to give a mixture of N-{5-[4-(methylthio)benzyl]-4-[(E)-2-(4-nitrophenyl)vinyl]-1,3-thiazol-2-yl}acetamide and N-{5-[4-(methylthio)benzyl]-4-[(Z)-2-(4-nitrophenyl)vinyl]-1,3-thiazol-2-yl}acetamide (E : Z = 1 : 2) (671 mg) as yellow amorphous.
¹H-NMR (DMSO-d₆), δ (ppm): 2.08 (3Hx2/3, s), 2.12 (3Hx1/3, s), 2.44 (3H, s), 4.04 (2Hx2/3, s), 4.30 (2Hx1/3, s), 6.71 (1Hx2/3, d, J=12.5Hz), 6.84 (1Hx2/3, d, J=12.5Hz), 7.18 (4Hx2/3, s), 7.23 (4Hx1/3, s), 7.24 (1Hx1/3, d, J=15.5Hz), 7.40 (1Hx1/3, d, J=15.5Hz), 7.65 (2Hx2/3, d, J=9.0Hz), 7.92 (2Hx1/3, d, J=9.0Hz), 8.12 (2Hx2/3, d, J=9.0Hz), 8.22 (2Hx1/3, d, J=9.0Hz), 11.85 (1Hx2/3, brs), 12.16 (1Hx1/3, brs).
MS: 426 (M+H)⁺

### Step 8

Potassium peroxymonosulfate (1.41 g) was suspended in water (4 ml) and THF (4 ml), and then a mixture of N-{5-[4-(methylthio)benzyl]-4-[(E)-2-(4-nitrophenyl)vinyl]-1,3-thiazol-2-yl}acetamide and N-{5-[4-(methylthio)benzyl]-4-[(Z)-2-(4-nitrophenyl)vinyl]-1,3-thiazol-2-yl}acetamide (E : Z = 1 : 2) (650 mg) in THF (9 ml) was added dropwise to the suspension at 0 °C. The reaction mixture was stirred at r.t. for 1 hour, and then water was added to the suspension. The mixture was extracted with AcOEt. The organic layer was washed with water and brine, dried over anhydrous MgSO₄, and concentrated *in vacuo* to give a mixture of N-{5-[4-(methylsulfonyl)benzyl]-4-[(E)-2-(4-nitrophenyl)vinyl]-1,3-thiazol-2-yl}acetamide and N-{5-[4-(methylsulfonyl)benzyl]-4-[(Z)-2-(4-nitrophenyl)vinyl]-1,3-thiazol-2-yl}acetamide (E : Z = 1 : 2) (693.3 mg) as yellow amorphous.
Z : E = 2 : 1
¹H-NMR (DMSO-d₆), δ (ppm): 2.09 (3Hx2/3, s), 2.13 (3Hx1/3, s), 3.18 (3H, s), 4.24 (2Hx2/3, s), 4.49 (2Hx1/3, s), 6.73 (1Hx2/3, d, J=12.5Hz), 6.86 (1Hx2/3, d, J=12.5Hz), 7.33 (1Hx1/3, d, J=15.5Hz), 7.41-7.97 (5/3H, m), 7.48 (2Hx2/3, d, J=9.0Hz), 7.55 (2Hx1/3, d, J=9.0Hz), 7.65 (2Hx2/3, d, J=9.0Hz), 7.85 (2Hx2/3, d, J=9.0Hz), 8.14 (2Hx2/3, d, J=9.0Hz), 8.22 (2Hx1/3, d, J=9.0Hz), 11.90 (1Hx2/3, s), 12.22 (1Hx1/3, s).
MS: 458 (M+H)⁺

### Step 9

A mixture of N-{5-[4-(methylsulfonyl)benzyl]-4-[(E)-2-(4-nitrophenyl)vinyl]-1,3-thiazol-2-yl}acetamide and N-{5-[4-(methylsulfonyl)benzyl]-4-[(Z)-2-(4-nitrophenyl)vinyl]-1,3-thiazol-2-yl}acetamide (E : Z = 1 : 2) (380 mg), 10% palladium on carbon (380 mg), MeOH (3.5 ml), THF (3.5 ml) and AcOH (0.5 ml) were combined. The reaction mixture was stirred under 3 atm H₂ at r.t. for 3 hours, and filtered through a celite pad. The filtrate was concentrated *in vacuo*. 1N-NaOH was added to the residue, and the mixture was extracted with AcOEt. The organic layer was washed with water and brine, dried over anhydrous MgSO₄, and concentrated *in vacuo*. The residue was purified by flash column chromatography over silica gel with CHCl₃ / MeOH (30:1→10:1) as an eluent to give N-{4-[2-(4-aminophenyl)ethyl]-5-[4-(methylsulfonyl)benzyl]-1,3-thiazol-2-yl}acetamide (161.8 mg) as off-white amorphous.
¹H-NMR (DMSO-d₆), δ (ppm): 2.08 (3H, s), 2.58-2.87 (4H, m), 3.18 (3H, s), 3.98 (2H, s), 4.85 (2H, s), 6.46 (2H, d, J=8.5Hz), 6.77 (2H, d, J=8.5Hz), 7.27 (2H, d, J=8.5Hz), 7.82 (2H, d, J=8.5Hz), 12.02 (1H, s).
MS: 430 (M+H)⁺

### Step 10

A mixture of N-{4-[2-(4-aminophenyl)ethyl]-5-[4-(methylsulfonyl)benzyl]-1,3-thiazol-2-yl}acetamide (73.3 mg), [(tert-butoxycarbonyl)amino]acetic acid (29.9 mg), 1-hydroxybenzotriazole (25.4 mg) and 1-ethyl-3-(3-dimethylaminopropyl)carbodiimide hydrochloride (34.3 mg) in DMF (1 ml) was stirred at r.t. for 7 hours. The reaction mixture was poured into saturated NaHCO₃, and extracted with AcOEt. The organic layer was washed with water and brine, dried over anhydrous MgSO₄, and concentrated *in vacuo*. The residue was purified by preparative silica gel chromatography with CHCl₃ / MeOH (20:1) as an eluent to give tert-butyl (2-{[4-(2-{2-(acetylamino)-5-[4-(methylsulfonyl)benzyl]-1,3-thiazol-4-yl}ethyl)phenyl]amino}-2-oxoethyl)carbamate (75 mg) as an off-white solid.

### Step 11

tert-Butyl (2-{[4-(2-{2-(acetylamino)-5-[4-(methylsulfonyl)benzyl]-1,3-thiazol-4-yl}ethyl)phenyl]amino}-2-oxoethyl)carbamate (61.2 mg) and 4N HCl in 1,4-dioxane solution (1 ml) were combined under N₂ atmosphere. The reaction mixture was stirred at r.t. for 1 hour. The solvent was removed *in vacuo*. The residue was solidified with AcOEt to give N-[4-(2-{2-(acetylamino)-5-[4-(methylsulfonyl)benzyl]-1,3-thiazol-4-yl}ethyl)phenyl]-2-aminoacetamide hydrochloride (56.2 mg) as an off-white solid.

### Step 12

N-[4-(2-{2-(Acetylamino)-5-[4-(methylsulfonyl)benzyl]-1,3-thiazol-4-yl}ethyl)phenyl]-2-aminoacetamide hydrochloride (30 mg), N,N'-bis(tert-butoxycarbonyl)-1H-pyrazole-1-carboxamidine (17.8 mg), N,N-diisopropylethylamine (20.0 µl), THF (0.5 ml) and DMF (0.1 ml) were combined under N₂ atmosphere. The reaction mixture was stirred at 15 °C for 14 hr, and concentrated *in vacuo*. The residue was purified by preparative silica gel thin-layer chromatography with chloroform / methanol (20:1) as an eluent to give di-tert-butyl {(Z)-[(2-{[4-(2-{2-(acetylamino)-5-[4-(methylsulfonyl)benzyl]-1,3-thiazol-4-yl}ethyl)phenyl]amino}-2-oxoethyl)amino]methylidene}biscarbamate as a colorless solid (17.3 mg).
¹H-NMR (200MHz, CDCl₃) δ (ppm): 1.50 (18H, s), 2.24 (3H, s), 2.74 - 2.94 (4H, m), 3.05 (3H, s), 3.85 (2H, s), 4.21 (2H, d, J = 5.7Hz), 6.9 (2H, d, J = 8.3Hz), 7.08 (2H, d, J = 8.3Hz), 7.35 (2H, d, J = 8.4Hz), 7.75 (2H, d, J = 8.2Hz), 8.99 (1H, t, J = 5.5Hz), 9.32 - 9.48 (1H, brs), 9.53 (1H, s), 11.35 (1H, s).
MS: 729.29 (M+H)+

### Step 13

The title compound was prepared from the compound of Step 12 of Production Example 11 in a manner similar to Step 15 of Production Example 2.
¹H-NMR (400MHz, DMSO-d₆) δ (ppm): 2.09 (3H, s), 2.84 (4Hx3/4, s), 2.88 (4Hx1/4, s), 3.17 (3Hx3/4, s), 3.19 (3Hx1/4, s), 3.98 - 4.07 (4H, m), 7.02 - 7.64 (11H, m), 7.78 (2Hx3/4, d, J = 8.4Hz), 7.84 (2Hx1/4, d, J = 8.4Hz), 10.18 (1H, s), 12.05 (1H, s).
MS: 529.2 (M+H)+ free

### Production Example 12: Synthesis of (3R)-1-({2-(acetylamino)-4-[2-(4-{[amino(imino)methyl]amino}phenyl)ethyl]-1,3-thiazol-5-yl}methyl)-N,N-dimethyl-3-pyrrolidinecarboxamide dihydrochloride

### Step 1

To a solution of N-{4-[(Z)-2-(4-nitrophenyl)vinyl]-1,3-thiazol-2-yl}acetamide (293 mg) in acetic acid (1.8 ml) were added methyl (3R)-3-pyrrolidinecarboxylate hydrochloride (201 mg) and paraformaldehyde (36.5 mg), and the mixture was stirred at 100 °C (bath temp.) for 2 hr. The solvent was removed *in vacuo*, the residue was adjusted to pH=9 with saturated aq. NaHCO₃, extracted with AcOEt. The organic layer was washed with brine, dried over MgSO₄ and evaporated to give methyl (3R)-1-({2-(acetylamino)-4-[(Z)-2-(4-nitrophenyl)vinyl]-1,3-thiazol-5-yl}methyl)-3-pyrrolidinecarboxylate as orange foam (402.2 mg), that was used as crude in the next reaction.
MS: 431.18 (M+H)+

### Step 2

Methyl (3R)-1-({2-(acetylamino)-4-[(Z)-2-(4-nitrophenyl)vinyl]-1,3-thiazol-5-yl}methyl)-3-pyrrolidinecarboxylate (400 mg), MeOH (7 ml), THF (7 ml) and then 10 % Pd/C (50 % wet) (810 mg) were combined under N₂ atmosphere. The mixture was stirred at 15 °C for 10 min under H₂ atmosphere (3 atm). The reaction mixture was filtered through a celite pad, and the filtrate was concentrated *in vacuo* to give yellow foam (367.1 mg, 101.3 %, MS: 403.20 (M+H)+).

To a solution of the yellow foam (367.1 mg) in THF (4.5 ml) was added N,N'-bis(tert-butoxycarbonyl)-1H-pyrazole-1-carboxamidine (419 mg), and the mixture was stirred for 62 hr at 15 °C. Volatiles were evaporated, and the residue (895.9 mg) was purified by flash column chromatography over silica gel with CHCl₃ : AcOEt (100:0-100:2) as an eluent to give methyl (3R)-1-[(2-(acetylamino)-4-{2-[4-({(Z)-[(tert-butoxycarbonyl)amino][(tert-butoxycarbonyl)imino]methyl}-amino)phenyl]ethyl}-1,3-thiazol-5-yl)methyl]-3-pyrrolidinecarboxylate (237.8 mg) as pale yellow foam.
¹H-NMR (200MHz, CDCl₃) δ (ppm) : 1.5 (9H, s), 1.53 (9H, s), 1.98 - 2.14 (2H, m), 2.22 (3H, s), 2.39 - 2.76 (3H, m), 2.78 - 3.11 (6H, m), 3.55 (2H, s), 3.68 (3H, s), 7.07 (2H, d, J = 8.5Hz), 7.45 (2H, d, J = 8.5Hz), 9.06 (1H, brs), 10.24 (1H, s), 11.64. (1H, s).
MS: 645.3 (M+H)+, 667.3 (M+Na)+

### Step 3

Methyl (3R)-1-[(2-(acetylamino)-4-{2-[4-({(Z)-[(tert-butoxycarbonyl)amino][(tert-butoxycarbonyl)imino]methyl} amino)phenyl]ethyl}-1,3-thiazol-5-yl)methyl]-3-pyrrolidinecarboxylate (232.2 mg), 1N-NaOH (0.9 ml) and dioxane (3 ml) were combined at 0 °C, and the mixture was stirred at 20 °C for 2 hr. To the mixture was added 1N-HCl (0.9 ml), and the solvent was evaporated *in vacuo.* To the residue was added CHCl₃. The insoluble salt was removed by filtration, and the filtrate was concentrated *in vacuo* to give (3R)-1-[(2-(acetylamino)-4-{2-[4-({(Z)-[(tert-butoxycarbonyl)amino]-[(tert-butoxycarbonyl)imino]methyl}amino)phenyl]ethyl}-1,3-thiazol-5-yl)methyl]-3-pyrrolidinecarboxylic acid (175.5 mg) as a white solid, that was used as crude in the next reaction.
MS: 631.29 (M+H)+

### Step 4

To a solution of (3R)-1-[(2-(acetylamino)-4-{2-[4-({(Z)-[(tert-butoxycarbonyl)amino][(tert-butoxycarbonyl) imino]methyl}amino)phenyl]ethyl}-1,3-thiazol-5-yl)methyl]-3-pyrrolidinecarboxylic acid (60 mg) in 0.5 ml of dichloromethane were added methylamine hydrochloride (10.1 mg), 1-hydroxybenzotriazole hydrate (HOBt, 19.3 mg) and 1-(3-dimethylaminopropyl)-3-ethylcarbodiimide hydrochloride (EDCI, 52.118 µl), and then the mixture was stirred for 14 hr at 20 °C. The reaction mixture was diluted with 4ml of dichloromethane and washed with water. The organic layer was dried over diatomaceous earth and evaporated under vaccum to give crude pale yellow oil. The crude oil was purified by preparative silica gel thin-layer chromatography with chloroform / methanol (15:1) as an eluent to give di-tert-butyl {(Z)-[(4-{2-[2-(acetylamino)-5-({(3R)-3-[(dimethylamino)carbonyl]-1-pyrrolidinyl}methyl)-1,3-thiazol-4-yl]ethyl}phenyl)amino]methylidene}biscarbamate (45.4 mg) as a white solid.
¹H-NMR (400MHz, CDCl₃) δ (ppm) : 1.5 (9H, s), 1.54 (9H, s), 1.96 - 2.11 (2H, m), 2.22 (3H, s), 2.31 - 2.42 (1H, m), 2.44 - 2.53 (1H, m), 2.82 - 2.92 (5H, m), 2.94 (3H, s), 3.01 (3H, s), 3.01 - 3.08 (1H, m), 3.15 - 3.27 (1H, m), 3.57 (2H, s), 7.08 (2H, d, J = 8.4Hz), 7.46 (2H, d, J = 8.4Hz), 8.88 (1H, brs), 10.24 (1H, s), 11.63 (1H, s).
MS: 659.3 (M+H)+, 680.3 (M+Na)+

### Step 5

The title comound was prepared from the compound of Step 4 of Production Example 12 in a manner similar to Step 15 of Production Example 2.
¹H-NMR (400MHz, DMSO-d₆) δ (ppm) : 1.72 - 3.74 (20H, m), 4.42 - 4.57 (2H, m), 7.12 - 7.18 (2H, m), 7.27 - 7.35 (2H, m), 7.43 (4H, brs), 9.91 (1Hx3/5, m), 9.97 (1Hx2/5, m), 10.26 (1Hx2/5, m), 11.01 (1Hx3/5, m), 12.32 (1Hx3/5, m), 12.34 (1Hx2/5, m).
MS: 458.4 (M+H)+, 480.2 (M+Na)+ free

### Production Example 13: Synthesis of (3R)-1-({2-(acetylamino)-4-[2-(4-{[amino(imino)methyl]amino}phenyl)ethyl]-1,3-thiazol-5-yl}methyl)-N-methyl-3-pyrrolidinecarboxamide dihydrochloride

### Step 1

Di-tert-butyl {(Z)-[(4-{2-[2-(acetylamino)-5-({(3R)-3-[(methylamino)carbonyl]-1-pyrrolidinyl}methyl)-1,3-thiazol-4-yl]ethyl}phenyl)amino]methylidene}biscarbamate was prepared from the compound of Step 3 of Production Example 12 in a manner similar to Step 4 of Production Example 12.
¹H-NMR (400MHz, CDCl₃) δ (ppm) : 1.5 (9H, s), 1.54 (9H, s), 1.88 - 1.99 (1H, m), 2.09 - 2.21 (1H, m), 2.24 (3H, s), 2.25 - 2.37 (2H, m), 2.78 (3H, d, J = 4.7Hz), 2.79 - 2.37 (7H, m), 3.52 (1H, d, J = 13.9Hz), 3.58 (1H, d, J = 14.2Hz), 6.75 (1H, d, J = 4.4Hz), 7.06 (2H, d, J = 8.4Hz), 7.45 (2H, d, J = 8.4Hz), 8.91 (1H, brs), 10.24 (1H, s), 11.63 (1H, s).
MS: 644.2 (M+H)+, 666.3 (M+Na)+

### Step 2

The title compound was prepared from the compound of Step 1 of Production Example 13 in a manner similar to Step 15 of Production Example 2.
¹H-NMR (400MHz, DMSO-d₆): 1.79 - 3.74 (17H, m), 4.41 - 4.57 (2H, m), 7.11 - 7.18 (2H, m), 7.27 - 7.35 (2H, m), 7.42 (4H, brs), 8.13 - 8.25 (1H, m), 9.88 (1Hx2/3, s), 9.95 (1Hx1/3, s), 10.34 (1Hx1/3, brs), 10.99 (1Hx2/3, brs), 12.32 (1Hx2/3, s), 12.33 (1Hx1/3, s).
MS: 444.2 (M+H)+, 466.1 (M+Na)+ free

### Production Example 14: Synthesis of (3S)-1-({2-(acetylamino)-4-[2-(4-{[amino(imino)methyl]amino}phenyl)ethyl]-1,3-thiazol-5-yl}methyl)-N,N-dimethyl-3-pyrrolidinecarboxamide dihydrochloride

### Step 1

Methyl (3S)-1-({2-(acetylamino)-4-[(Z)-2-(4-nitrophenyl)vinyl]-1,3-thiazol-5-yl}methyl)-3-pyrrolidinecarboxylate was prepared from N-{4-[(Z)-2-(4-nitrophenyl)vinyl]-1,3-thiazol-2-yl}acetamide in a manner similar to Step 1 of Production Example 12.
MS: 431.16 (M+H)+

### Step 2

Methyl (3S)-1-[(2-(acetylamino)-4-{2-[4-({(Z)-[(tert-butoxycarbonyl)amino][(tert-butoxycarbonyl)imino]methyl}-amino)phenyl]ethyl}-1,3-thiazol-5-yl)methyl]-3-pyrrolidinecarboxylate was prepared from the compound of Step 1 of Production Example 14 in a manner similar to Step 2 of Production Example 12.
¹H-NMR (200MHz, CDCl₃) δ (ppm): 1.5 (9H, s), 1.53 (9H, s), 1.99 - 2.14 (2H, m), 2.22 (3H, s), 2.38 - 2.75 (3H, m), 2.79 - 3.07 (6H, m), 3.53 (2H, s), 3.68 (3H, s), 7.07 (2H, d, J = 8.5Hz), 7.46 (2H, d, J = 8.5Hz), 9.51 (1H, brs), 10.24 (1H, s), 11.64 (1H, s).
MS: 645.3 (M+H)+, 667.3 (M+Na)+

### Step 3

(3S)-1-[(2-(Acetylamino)-4-{2-[4-({(Z)-[(tert-butoxycarbonyl)amino][(tert-butoxycarbonyl)imino]methyl} amino)phenyl]ethyl}-1,3-thiazol-5-yl)methyl]-3-pyrrolidinecarboxylic acid was prepared from the compound of Step 2 of Production Example 14 in a manner similar to Step 3 of Production Example 12.
MS: 631.29 (M+H)+

### Step 4

Di-tert-butyl {(Z)-[(4-{2-[2-(acetylamino)-5-({(3S)-3-[(dimethylamino)carbonyl]-1-pyrrolidinyl}methyl)-1,3-thiazol-4-yl]ethyl}phenyl)amino]methylidene}biscarbamate was prepared from the compound of Step 3 of Production Example 14 in a manner similar to Step 4 of Production Example 12.
¹H-NMR (400MHz, CDCl₃) δ (ppm) : 1.5 (9H, s), 1.54 (9H, s), 1.96 - 2.12 (2H, m), 2.22 (3H, s), 2.31 - 2.41 (1H, m), 2.44 - 2.52 (1H, m), 2.82 - 2.92 (5H, m), 2.94 (3H, s), 3.01 (3H, s), 3.01 - 3.08 (1H, m), 3.15 - 3.27 (1H, m), 3.57 (2H, s), 7.08 (2H, d, J = 8.4Hz), 7.46 (2H, d, J = 8.8Hz), 8.87 (1H, brs), 10.24 (1H, s), 11.63 (1H, s).
MS: 658.3 (M+H)+, 680.3 (M+Na)+

### Step 5

The title compound was prepared from the compound of Step 4 of Production Example 14 in a manner similar to Step 15 of Production Example 2.
¹H-NMR (400MHz, DMSO-d₆) δ (ppm) : 1.74 - 3.73 (20H, m), 4.42 - 4.58 (2H, m), 7.12 - 7.19 (2H, m), 7.27 - 7.35 (2H, m), 7.42 (4H, brs), 9.87 (1Hx4/7, s), 9.93 (1Hx3/7, s), 10.22 (1Hx3/7, brs), 10.94 (1Hx4/7, brs), 12.32 (1Hx4/7, s), 12.34 (1Hx3/7, s).
MS: 458.4 (M+H)+, 480.2 (M+Na)+ free

### Production Example 15: Synthesis of (3S)-1-({2-(acetylamino)-4-[2-(4-{[amino(imino)methyl]amino}phenyl)ethyl]-1,3-thiazol-5-yl}methyl)-N-methyl-3-pyrrolidinecarboxamide dihydrochloride

### Step 1

Di-tert-butyl {(Z)-[(4-{2-[2-(acetylamino)-5-({(3S)-3-[(methylamino)carbonyl]-1-pyrrolidinyl}methyl)-1,3-thiazol-4-yl]ethyl}phenyl)amino]methylidene}biscarbamate was prepared from the compound of Step 3 of Production Example 14 in a manner similar to Step 4 of Production Example 12.
¹H-NMR (400MHz, CDCl₃) δ (ppm): 1.5 (9H, s), 1.54 (9H, s), 1.88 - 1.99 (1H, m), 2.09 - 2.21 (1H, m), 2.24 (3H, s), 2.26 - 2.37 (2H, m), 2.76 - 2.94 (10H, m), 3.52 (1H, d, J = 13.9Hz), 3.58 (1H, d, J = 13.9Hz), 6.76 (1H, d, J = 4.4Hz), 7.06 (2H, d, J = 8.4Hz), 7.45 (2H, d, J = 8.4Hz), 8.91 (1H, brs), 10.25 (1H, s), 11.64 (1H, s).
MS: 644.3 (M+H)+, 666.3 (M+Na)+

### Step 2

The title compound was prepared from the compound of Step 1 of Production Example 15 in a manner similar to Step 15 of Production Example 2.
¹H-NMR (400MHz, DMSO-d₆) δ (ppm): 1.79 - 3.76 (17H, m), 4.41 - 4.57 (2H, m), 7.11 - 7.18 (2H, m), 7.27 - 7.35 (2H, m), 7.43 (4H, brs), 8.1 - 8.3 (1H, m), 9.9 (1Hx2/3, s), 9.98 (1Hx1/3, s), 10.37 (1Hx1/3, brs), 11.04 (1Hx2/3, brs), 12.32 (1Hx2/3, s), 12.33 (1Hx1/3, s).
MS: 444.2 (M+H)+, 467.2 (M+Na)+ free

### Production Example 16: Synthesis of N-({2-(acetylamino)-4-[2-(4-{[amino(imino)methyl]amino}phenyl)ethyl]-1,3-thiazol-5-yl}methyl)-N-methyl-4-(methylsulfonyl)benzamide hydrochloride

### Step 1

To a solution of N-{4-[(Z)-2-(4-nitrophenyl)vinyl]-1,3-thiazol-2-yl}acetamide (1.0 g) in acetic acid (10 ml) were added N-methylamine hydrochloride (2.33 g) and paraformaldehyde (124 mg), and the mixture was stirred at 100 °C (bath temp.) for 1 hr. The solvent was removed *in vacuo*, the residue was adjusted to pH=9 with aq. sat. NaHCO₃, the mixture was extracted with ethyl acetate. The organic layer was washed with brine, dried over MgSO₄ and evaporated to give N-{5-[(methylamino)methyl]-4-[(Z)-2-(4-nitrophenyl)vinyl]-1,3-thiazol-2-yl}acetamide as orange foam (0.92 g), that was used as crude in the next reaction.
MS: 333.29 (M+H)+

### Step 2

To a solution of N-{5-[(methylamino)methyl]-4-[(Z)-2-(4-nitrophenyl)vinyl]-1,3-thiazol-2-yl}acetamide (100 mg) in 1 ml of dichloromethane were added 4-(methylsulfonyl)benzoic acid (60.2 mg), HOBt (61 mg) and EDCI HCl (86.5 mg), and then the mixture was stirred for 3 hr at 20 °C. The reaction mixture was diluted with 4 ml of dichloromethane and washed with water. The organic layer was dried over diatomaceous earth and evaporated under vaccum to give crude pale yellow oil (144.3 mg, 93.2 %). The crude oil was purified by preparative silica gel thin-layer chromatography with chloroform / methanol (15:1) as an eluent to give N-({2-(acetylamino)-4-[(Z)-2-(4-nitrophenyl)vinyl]-1,3-thiazol-5-yl}methyl)-N-methyl-4-(methylsulfonyl)benzamide (144.3 mg).
¹H-NMR (200MHz, CDCl₃) δ (ppm) : 2.11 (3H, s), 3.00 (3H, s), 3.08 (3H, s), 4.79 (2H, s), 6.53 - 6.99 (2H, m), 7.3 - 8.28 (8H, m), 10.06 (1H, brs).
MS: 537.1 (M+Na)+

### Step 3

N-({2-(Acetylamino)-4-[(Z)-2-(4-nitrophenyl)vinyl]-1,3-thiazol-5-yl}methyl)-N-methyl-4-(methylsulfonyl)benzamide (131.9 mg), MeOH (3.5 ml), THF (3.5 ml), AcOH (0.5 ml) and then 10 % Pd/C (50 % wet) (256 mg) were combined under N₂ atmosphere. The mixture was stirred at 20 °C for 30 min under H₂ atmosphere (3 atm). The reaction mixture was filtered through a celite pad, and the filtrate was concentrated *in vacuo.* The residue was adjusted to pH=9 with saturated aq. NaHCO₃, and the mixture was extracted with chroloform. The organic layer was washed with brine, dried over MgSO₄, and concentrated *in vacuo* to give N-({2-(acetylamino)-4-[2-(4-aminophenyl)ethyl]-1,3-thiazol-5-yl}methyl)-N-methyl-4-(methylsulfonyl)benzamide as colorless oil, that was used as crude in the next reaction.
MS: 487.15 (M+H)+

### Step 4

To a solution of N-({2-(acetylamino)-4-[2-(4-aminophenyl)ethyl]-1,3-thiazol-5-yl}methyl)-N-methyl-4-(methylsulfonyl)benzamide (103.6 mg) in THF (0.2 ml) was added N,N'-bis(tert-butoxycarbonyl)-1H-pyrazole-1-carboxamidine (99.1 mg), and the mixture was stirred for 14 hr at 20 °C. Volatiles were evaporated *in vacuo* and the residue was purified by preparative silica gel thin-layer chromatography with chloroform / methanol (15:1) as an eluent to give di-tert-butyl {(E)-[(4-{2-[2-(acetylamino)-5-({methyl[4-(methylsulfonyl)benzoyl]amino}methyl)-1,3-thiazol-4-yl]ethyl}phenyl)amino]methylidene}biscarbamate (52.8 mg).
¹H-NMR (400MHz, CDCl₃) δ (ppm): 1.48 (9H, s), 1.53 (9H, s), 2.25 (3H, s), 2.55 - 3.02 (7H, m), 3.06 (3H, s), 4.2 (2Hx2/7, brs), 4.61 (2Hx5/7, brs), 6.85 - 6.99 (2Hx2/7, m), 7.09 (2Hx5/7, d, J = 7.3Hz), 7.37 - 7.51 (2H, m), 7.53 - 7.7 (2H, m), 7.99 (2H, d, J = 7.7Hz), 8.96 (1H, s), 10.24 (1H, s), 11.62 (1H, s).
MS: 729.24 (M+H)+

### Step 5

The title compound was prepared from the compound of Step 4 of Production Example 16 in a manner similar to Step 15 of Production Example 2.
¹H-NMR (400MHz, DMSO-d₆) δ (ppm): 2.13 (3H, s), 2.7 - 2.85 (3H, m), 2.94 (4H, s), 3.26 (3H, s), 4.4 (2Hx1/4, s), 4.63 (2Hx3/4, s), 7.14 (2H+2Hx1/4, d, J = 8.1Hz), 7.27 (2Hx3/4, d, J = 8Hz), 7.34 (4H, brs), 7.62 (2H, d, J = 8Hz), 7.99 (2H, d, J = 8Hz), 9.68 (1H, s), 12.13 (1H, s).
MS: 529.2 (M+H)+, 551.2 (M+Na)+ Free

### Production Example 17: Synthesis of N-({2-(acetylamino)-4-[2-(4-{[amino(imino)methyl]amino}phenyl)ethyl]-1,3-thiazol-5-yl}methyl)-N,N',N'-trimethylterephthalamide hydrochloride

### Step 1

N-({2-(Acetylamino)-4-[(Z)-2-(4-nitrophenyl)vinyl]-1,3-thiazol-5-yl}methyl)-N,N',N'-trimethylterephthalamide was prepared from the compound of Step 1 of Production Example 16 in a manner similar to Step 2 of Production Example 16.
¹H-NMR (200MHz, CDCl₃) δ (ppm): 2.14 (3H, s), 2.97 (3H, s), 3.00 (3H, s), 3.12 (3H, s), 4.75 (2H, brs), 6.55 - 6.97 (2H, m), 7.3 - 8.29 (8H, m), 10.17 (1H, bs).
MS: 508.0 (M+H)+, 530.2 (M+Na)+

### Step 2

N-({2-(Acetylamino)-4-[2-(4-aminophenyl)ethyl]-1,3-thiazol-5-yl}methyl)-N,N',N'-trimethylterephthalamide was prepared from the compound of Step 1 of Production Example 17 in a manner similar to Step 3 of Production Example 16.
MS: 480.22 (M+H)+

### Step 3

Di-tert-butyl [(Z)-({4-[2-(2-(acetylamino)-5-{[{4-[(dimethylamino)carbonyl]benzoyl}(methyl)amino]methyl}-1,3-thiazol-4-yl)ethyl]phenyl}amino)methylidene]biscarbamate was prepared from the compound of Step 2 of Production Example 17 in a manner similar to Step 4 of Production Example 16.
¹H-NMR (400MHz, CDCl₃) δ (ppm): 1.49 (9H, s), 1.53 (9H, s), 2.27 (3H, s), 2.6 - 2.88 (4H, m), 2.95 (6H, s), 3.12 (3H, s), 4.16 - 4.68 (2H, m), 6.89 - 7.18 (2H, m), 7.44 (6H, s), 10.27 (1H, s), 11.62 (1H, s).
MS: 722.3 (M+H)+, 744.2 (M+Na)+

### Step 4

The title compound was prepared from the compound of Step 3 of Production Example 17 in a manner similar to Step 15 of Production Example 2.
¹H-NMR (400MHz, DMSO-d₆) δ (ppm): 2.13 (3H, s), 2.76 (3H, s), 2.84 - 3.06 (10H, m), 4.34 - 4.7 (2H, m), 7.03 - 7.56 (12H, m), 9.76 (1H, s), 12.12 (1H, s).
MS: 522.24 (M+H)+ Free

### Production Example 18: Synthesis of 4-acetyl-N-({2-(acetylamino)-4-[2-(4-{[amino(imino)methyl]amino}phenyl)-ethyl]-1,3-thiazol-5-yl}methyl)-N-methylbenzamide hydrochloride

### Step 1

4-Acetyl-N-({2-(acetylamino)-4-[(Z)-2-(4-nitrophenyl)vinyl]-1,3-thiazol-5-yl}methyl)-N-methylbenzamide was prepared from the compound of Step 1 of Production Example 16 in a manner similar to Step 2 of Production Example 16.
¹H-NMR (200MHz, CDCl₃) δ (ppm) : 2.16 (3H, s), 2.63 (3H, s), 2.89 (3H, s), 4.78 (2H, brs), 6.58 - 6.98 (2H, m), 7.32 - 8.32 (8H, m), 10.03 (1H, brs).
MS: 479.2 (M+H)+, 501.1 (M+Na)+

### Step 2

4-Acetyl-N-({2-(acetylamino)-4-[2-(4-aminophenyl)ethyl]-1,3-thiazol-5-yl}methyl)-N-methylbenzamide was prepared from the compound of Step 1 of Production Example 18 in a manner similar to Step 3 of Production Example 16.
MS: 451.17 (M+H)+

### Step 3

Di-tert-butyl [(E)-({4-[2-(2-(acetylamino)-5-{[(4-acetylbenzoyl)(methyl)amino]methyl}-1,3-thiazol-4-yl)ethyl]phenyl}amino)methylidene]biscarbamate was prepared from the compound of Step 2 of Production Example 18 in a manner similar to Step 4 of Production Example 16.
¹H-NMR (200MHz, CDCl₃) δ (ppm) : 1.49 (9H, s), 1.53 (9H, s), 2.22 (3H, s), 2.62 (3H, s), 2.64 - 3.12 (7H, m), 4.05 - 4.77 (2H, m), 6.77 - 7.18 (2H, m), 7.31 - 7.65 (4H, m), 7.98 (2H, d, J = 8.0Hz), 10.23 (1H, s), 11.62 (1H, s).
MS: 693.1 (M+H)+, 715.3 (M+Na)+

### Step 4

The title comound was prepared from the compound of Step 3 of Production Example 18 in a manner similar to Step 15 of Production Example 2.
¹H-NMR (400MHz, CD₃OD) δ (ppm): 2.29 (3H, s), 2.62 (3H, s), 2.86 (3H, s), 2.96 - 3.18 (4H, m), 4.44 - 4.65 (2H, m), 7.02 - 8.19 (9H, m).
MS: 493.17 (M+H)+ Free

### Production Example 19: Synthesis of N-({2-(acetylamino)-4-[2-(4-{[amino(imino)methyl]amino}phenyl)ethyl]-1,3-thiazol-5-yl}methyl)-N-methyl-4-nitrobenzamide hydrochloride

### Step 1

To a solution of N-{5-[(methylamino)methyl]-4-[(Z)-2-(4-nitrophenyl)vinyl]-1,3-thiazol-2-yl}acetamide (100 mg) in dichloromethane (1.5 ml) were added N,N-diisopropylethylamine (0.177 ml) and trifluoroacetic anhydride (0.127 ml) at 0 °C, and the mixture was stirred for 2 hours at same temperature. To the reaction mixture was added aq. saturated NaHCO₃ (30 ml), and the mixture was extracted with dichloromethane (30 mlx3), the extract was washed with brine, drided over MgSO₄ and evaporated *in vacuo* to give N-({2-(acetylamino)-4-[(Z)-2-(4-nitrophenyl)vinyl]-1,3-thiazol-5-yl}methyl)-2,2,2-trifluoro-N-methylacetamide as pale yellow foam (160.4 mg), that was used as crude in the next reaction.
MS: 429.06 (M+H)+

### Step 2

Di-tert-butyl [(E)-({4-[2-(2-(acetylamino)-5-{[methyl(trifluoroacetyl)amino]methyl}-1,3-thiazol-4-yl)ethyl]phenyl}amino)methylidene]biscarbamate was prepared from the compound of Step 1 of Production Example 19 in a manner similar to Step 2 of Production Example 12.
¹H-NMR (400MHz, CDCl₃) δ (ppm): 1.5 (9H, s), 1.53 (9H, s), 2.26 - 2.32 (3H, m), 2.82 - 3.11 (7H, m), 4.33 - 4.42 (2H, m), 6.97 - 7.13 (2H, m), 7.41 - 7.51 (2H, m), 10.3 (1H, brs), 11.63 (1H, brs).
MS: 643.2 (M+H)+

### Step 3

To a solution of di-tert-butyl [(E)-({4-[2-(2-(acetylamino)-5-{[methyl(trifluoroacetyl)amino]methyl}-1,3-thiazol-4-yl)ethyl]phenyl}amino)methylidene]biscarbamate (26.4 mg) in methanol (0.5 ml) was added aq. 10 % K₂CO₃ (0.25 ml) at 0 °C, and then the mixture was stirred for 1.5 hr at 20 °C. The reaction mixture was evaporated *in vacuo,* brine (50 ml) was added, and the mixture was extracted with CHCl₃ (10 mlx3), the extract was dried over MgSO₄ and evaporated to give di-tert-butyl ((E)-{[4-(2-{2-(acetylamino)-5-[(methylamino)methyl]-1,3-thiazol-4-yl}ethyl)phenyl]amino}methylidene)biscarbamate (20.5 mg) as pale yellow foam, that was used as crude in the next reaction.
MS: 547.3 (M+H)+

### Step 4

To a solution of di-tert-butyl ((E)-{[4-(2-{2-(acetylamino)-5-[(methylamino)methyl]-1,3-thiazol-4-yl}ethyl)phenyl]amino}methylidene)biscarbamate (20 mg) in dichloromethane (0.5 ml) were added 4-nitrobenzoic acid (6.11 mg), HOBt (7.42 mg) and EDCI HCl (10.5 mg), and then the mixture was stirred for 3 hr at 20 °C. The reaction mixture was diluted with dichloromethane (4 ml) and the solution was washed with water. The organic layer was dried over MgSO₄ and evaporated under vaccum to give crude pale yellow oil. The crude oil was purified by preparative silica gel thin-layer chromatography with chloroform / methanol (15:1) as an eluent and purified by PTLC (0.5 mmx2, CHCl₃:MeOH=15:1 then CHCl₃:AcOEt=1:1) to give di-tert-butyl [(E)-({4-[2-(2-(acetylamino)-5-{[methyl(4-nitrobenzoyl)amino]methyl}-1,3-thiazol-4-yl)ethyl]phenyl}amino)methylidene]biscarbamate (14.3 mg).
¹H-NMR (400MHz, CDCl₃) δ (ppm): 1.4 - 1.62 (18H, m), 2.18 - 2.3 (3H, m), 2.54 - 3.1 (7H, m), 3.97 - 4.75 (2H, m), 6.68 - 8.37 (8H, m), 10.23 (1H, brs), 11.62 (1H, brs).
MS: 696.27 (M+H)+

### Step 5

The title compound was prepared from the compound of Step 4 of Production Example 19 in a manner similar to Step 15 of Production Example 2.
¹H-NMR (400MHz, DMSO-d₆) δ (ppm) : 2.13 (3H, s), 2.69 - 3.04 (7H, m), 4.63 (2H, s), 7.14 (2H, d, J = 8Hz), 7.27 (2H, d, J = 8.4Hz), 7.31 (4H, s), 7.63 (2H, d, J = 8.4Hz), 8.29 (2H, d, J = 8.4Hz), 9.63 (1H, s), 12.12 (1H, s).
MS: 496.1 (M+H) Free

### Production Example 20: Synthesis of (2E)-3-{2-(acetylamino)-4-[2-(4-{[amino(imino)methyl]amino}phenyl)ethyl]-1,3-thiazol-5-yl}-N,N-dimethylacrylamide hydrochloride

### Step 1

Ethyl 4-chloro-3-oxobutanoate (35 g) was dissolved in dichloromethane (70 ml), and then sulfuryl chloride (17.1 ml) in dichloromethane (20 ml) was added dropwise to the solution at 0 °C over 15 minutes under nitrogen atmosphere. The reaction mixture was stirred at room temperature for 3 hours, and concentrated *in vacuo.* The residual oil, N'-((E)-ethanoyl)carbamimidothioic acid (25.1 g) and acetone (600 ml) were combined. The reaction mixture was refluxed for 2.5 hours. After cooled to room temperature, the mixture was concentrated *in vacuo.* The residual solid was washed with water and isopropyl ether to give ethyl 2-(acetylamino)-4-(chloromethyl)-1,3-thiazole-5-carboxylate (21.2 g) as a pale yellow solid.
mp. 164-165 °C
¹H-NMR (DMSO-d₆), δ (ppm) : 1.30 (3H, t, J=7.0Hz), 2.19 (3H, s), 4.29 (2H, q, J=7.0Hz), 5.00 (2H, s), 12.72 (1H, s).
MS: 263 (M+H)⁺

### Step 2: Ethyl 2-(acetylamino)-4-[(E)-2-(4-nitrophenyl)ethenyl]-1,3-thiazole-5-carboxylate

To a stirring solution of ethyl 2-(acetylamino)-4-(chloromethyl)-1,3-thiazole-5-carboxylate (1.0 g, 3.81 mmol) in N,N-dimethylformamide (20 mL) was added triphenylphosphine (1.2 g, 4.57 mmol) at room temperature. The resultant mixture was stirred at 65 °C for 5 hours. To the mixture was added potassium tert-butoxide (555 mg, 4.95 mmol) at 5 °C, and the resultant mixture was stirred at 5 °C for 30 minutes. p-Nitrobenzaldehyde (805 mg, 5.33 mmol) was added at 5 °C. After stirring for 1 hour at room temperature, the reaction was quenched with water, and the mixture was filtered to give the title compound (1.0 g, 72.7%) as a yellow solid.
¹H-NMR (CDCl₃), δ (ppm): 1.40 (3H, t, J=7.2Hz), 2.33 (3H, s), 4.38 (2H, q, J=7.2Hz), 7.59 (1H, d, J=16.0Hz), 7.70 (2H, d, J=8.8Hz), 8.18 (1H, d, J=16.0Hz), 8.22 (2H, d, J=8.8Hz), 8.90 (1H, m).

### Step 3

Ethyl 2-(acetylamino)-4-[2-(4-aminophenyl)ethyl]-1,3-thiazole-5-carboxylate was prepared from the compound of Step 2 of Production Example 20 in a manner similar to Step 6 of Production Example 10.
¹H-NMR (CDCl₃), δ (ppm): 1.35 (3H, t, J=7.0Hz), 2.27 (3H, s), 2.84 (2H, m), 3.28 (2H, m), 3.56 (2H, m), 4.31 (2H, q, J=7.0Hz), 6.61 (2H, d, J=8.3Hz), 7.01 (2H, d, J=8.3Hz), 9.12 (1H, m).

### Step 4

Ethyl 2-(acetylamino)-4-[2-(4-aminophenyl)ethyl]-1,3-thiazole-5-carboxylate (310 mg) was dissolved in tetrahydrofuran (6 ml) under nitrogen atmosphere. Then, di(tert-butyl) dicarbonate (223 mg) in tetrahydrofuran (1 ml) was added to the solution at room temperature. The reaction mixture was refluxed for 2 hours. After cooled to room temperature, the mixture was concentrated *in vacuo.* The residual solid was washed with ethyl ether to give ethyl 2-(acetylamino)-4-(2-{4-[(tert-butoxycarbonyl)amino]phenyl}-ethyl)-1,3-thiazole-5-carboxylate (370.7 mg) as an off-white solid.
mp. 213-214 °C
¹H-NMR (DMSO-d₆), δ (ppm): 1.26 (3H, t, J=7.0Hz), 1.46 (9H, s), 2.17 (3H, s), 2.85 (2H, t, J=7.5Hz), 3.23 (2H, t, J=7.5Hz), 4.22 (2H, q, J=7.0Hz), 7.04 (2H, d, J=8.5Hz), 7.33 (2H, d, J=8.5Hz), 9.23 (1H, brs), 12.55 (1H, brs).
MS: 434 (M+H)⁺

### Step 5

Ethyl 2-(acetylamino)-4-(2-{4-[(tert-butoxycarbonyl)-amino]phenyl}ethyl)-1,3-thiazole-5-carboxylate (3 g), 1N-aqueous sodium hydroxide solution (17.3 ml) and ethanol (30 ml) were combined, and the mixture was refluxed for 5 hours. After cooled to room temperature, the organic solvent was removed *in vacuo.* The aqueous solution was acidified (pH=4) with 1N-hydrochloric acid, and extracted with ethyl acetate (twice). The combined organic layer was dried over anhydrous magnesium sulfate, and concentrated *in vacuo.* The residual solid was dissolved in pyridine (45 ml), and then acetyl chloride (1.48 ml) was added dropwise to the solution at 0 °C under nitrogen atmosphere. The reaction mixture was stirred at room temperature for 13 hours, and pyridine was removed *in vacuo.* Water was added to the residue, and the mixture was acidified with 1N-hydrochloric acid. The precipitate was collected *in vacuo.* The solid was washed with water and ethyl ether to give 2-(acetylamino)-4-(2-{4-[(tert-butoxycarbonyl)-amino]phenyl}ethyl)-1,3-thiazole-5-carboxylic acid (2.23 g) as an off-white solid.
mp. 237-238 °C
¹H-NMR (DMSO-d₆), δ (ppm): 1.46 (9H, s), 2.16 (3H, s), 2.85 (2H, m), 3.23 (2H, m), 7.04 (2H, d, J=8.5Hz), 7.33 (2H, d, J=8.5Hz), 9.24 (1H, s), 12.46 (1H, s).
MS: 404 (M-H)⁺

### Step 6

To a solution of 2-(acetylamino)-4-(2-{4-[(tert-butoxycarbonyl)amino]phenyl}ethyl)-1,3-thiazole-5-carboxylic acid in CH₂Cl₂ (3 ml) and DMF (3 ml) were added N,O-dimethylhydroxyamine hydrochloride (118 mg), EDCI (0.509 ml) and HOBt (188 mg), and then the mixture was stirred for 3 days at ambient temperature. The reaction mixture was diluted with AcOEt (50 ml) and washed with water (50 mlx3). The organic layer was dried over MgSO₄ and evaporated under vaccum. The residue was triturated with IPE and collected by filtration to give tert-butyl {4-[2-(2-(acetylamino)-5-{[methoxy(methyl)amino]-carbonyl}-1,3-thiazol-4-yl)ethyl]phenyl}carbamate (366 mg) as a pale yellow solid.
¹H-NMR (CDCl₃), δ (ppm): 1.46 (9H, s), 2.15 (3H, s), 2.74-2.93 (2H, m), 3.12-3.29 (2H, m), 3.22 (3H, s), 3.59 (3H, s), 7.05 (2H, d, J=8.5Hz), 7.33 (2H, d, J=8.5Hz), 9.21 (1H, s), 12.34 (1H, s).
MS: 471.1 (M+Na)⁺

### Step 7

To a solution of the compound obtained in Step 6 of Production Example 20 (3.93 g) in THF (80 mL) was added lithium aluminium hydirde (499 mg) slowly (over 15 min) at 5-10 °C (under ice-cooling). The mixture was stirred at 5 °C for 1 hr. 30 mL of aquaous solution of sodium potassium tartrate (1 M) was added slowly under ice-cooling, and then the mixture was stirred for another 0.5 hr at r.t. The mixture was extracted with ethyl acetate, and the organic layer was dried over MgSO₄, and concecntrated *in vacuo* to give pale yellow oil. This oil was triturated with IPE and EtOAc to give tert-butyl (4-{2-[2-(acetylamino)-5-formyl-1,3-thiazol-4-yl]ethyl}phenyl)carbamate as pale yellow powder (2.67g).
¹H-NMR (200MHz, DMSO-d₆), δ (ppm): 1.46 (9H, s), 2.19 (3H, s), 2.90 (2H, t, J=7.3 Hz), 3.22 (2H, t, J=7.3 Hz), 7.01 (2H, d, J=8.5 Hz), 7.32 (2H, d, J=8.5 Hz), 9.22 (1H, s), 9.77 (1H, s), 12.68 (1H, s).
MS: 390 (M+H)⁺

### Step 8

To a suspension of tert-butyl (4-{2-[2-(acetylamino)-5-formyl-1,3-thiazol-4-yl]ethyl}phenyl)carbamate (500 mg) in CHCl₃ (10 ml) was added (carbethoxymethylene)triphenylphosphorane (894 mg) at 20 °C, and the mixture was stirred for 1hr. To the reaction mixture was added brine, the mixture was extracted with CHCl₃, dried over MgSO₄ and evaporated. The residue was purified by SiO₂-column chromatography (toluene:AcOEt = 1 : 1) to give a mixture of ethyl (2E)-3-[2-(acetylamino)-4-(2-{4-[(tert-butoxycarbonyl)amino]phenyl}ethyl)-1,3-thiazol-5-yl]acrylate and ethyl (2Z)-3-[2-(acetylamino)-4-(2-{4-[(tert-butoxycarbonyl)amino]phenyl}ethyl)-1,3-thiazol-5-yl]acrylate as a pale yellow solid (495.9 mg).

### Step 9

A mixture of ethyl (2E)-3-[2-(acetylamino)-4-(2-{4-[(tert-butoxycarbonyl)amino]phenyl}ethyl)-1,3-thiazol-5-yl]acrylate and ethyl (2Z)-3-[2-(acetylamino)-4-(2-{4-[(tert-butoxycarbonyl)amino]phenyl}ethyl)-1,3-thiazol-5-yl]acrylate (290 mg) was purified by SiO₂-column chromatography (CHCl₃:MeOH = 100:0-100:2) to give ethyl (2E)-3-[2-(acetylamino)-4-(2-{4-[(tert-butoxycarbonyl)amino]phenyl}ethyl)-1,3-thiazol-5-yl]acrylate (118.6 mg) as a white solid.
¹H-NMR (400MHz, DMSO-d₆) δ (ppm): 1.22 (3H, t, J = 7.1Hz), 1.46 (9H, s), 2.16 (3H, s), 2.84 (2H, t, J = 7.3Hz), 2.97 (2H, t, J = 7.3Hz), 4.13 (2H, q, J = 7.1Hz), 5.88 (1H, d, J = 15.4Hz), 7.01 (2H, d, J = 8.4Hz), 7.32 (2H, d, J = 8.4Hz), 7.55 (1H, d, J = 15.4Hz), 9.22 (1H, s), 12.45 (1H, s).
MS: 457.67 (M+H)+

### Step 10

Ethyl (2E)-3-[2-(acetylamino)-4-(2-{4-[(tert-butoxycarbonyl)amino]phenyl}ethyl)-1,3-thiazol-5-yl]acrylate (53.8 mg) and trifluoroacetic acid (TFA, 2 ml) were combined at 0 °C. The reaction mixture was stirred at 25 °C for 30 min and concentrated *in vacuo.* To the residue were added AcOEt (20 ml), THF (1 ml) and aq. saturated NaHCO₃ solution (20 ml). The oraganic layer was separated, dried over magnesium sulfate and evaporated to give crude ethyl (2E)-3-[2-(acetylamino)-4-(2-{4-aminophenyl}ethyl)-1,3-thiazol-5-yl]acrylate as a yellow oil (46.2 mg, MS: 360.14 (M+H)+)

The crude ethyl (2E)-3-[2-(acetylamino)-4-(2-{4-aminophenyl}ethyl)-1,3-thiazol-5-yl]acrylate (46.2 mg), N,N'-bis(tert-butoxycarbonyl)-1H-pyrazole-1-carboxamidine (54.5 mg) and THF (0.5 ml) were combined under N₂ atmosphere. The reaction mixture was stirred at 20 °C for 17 hr, and then concentrated *in vacuo.* The residue was purified by preparative silica gel thin-layer chromatography with chloroform / methanol (20:1) as an eluent to give ethyl (2E)-3-(2-(acetylamino)-4-{2-[4-({(Z)-[(tert-butoxycarbonyl)amino]-[(tert-butoxycarbonyl)imino]methyl}amino)phenyl]ethyl}-1,3-thiazol-5-yl)acrylate (65.6 mg).
¹H-NMR (200MHz, CDCl₃) δ (ppm) : 1.32 (3H, t, J = 7.3Hz), 1.49 (9H, s), 1.53 (9H, s), 2.23 (3H, s), 2.8 - 3.13 (4H, m), 4.24 (2H, q, J = 7.2Hz), 6.03 (1H, d, J = 15.6Hz), 7.09 (2H, d, J = 8.5Hz), 7.43 (2H, d, J = 8.5Hz), 7.65 (1H, d, J = 15.6Hz), 9.99 - 10.56 (1H, brs), 11.64 (1H, s).

MS: 602.2 (M+H)+, 624.2 (M+Na)+

### Step 11

Ethyl (2E)-3-(2-(acetylamino)-4-{2-[4-({(Z)-[(tert-butoxycarbonyl)amino][(tert-butoxycarbonyl)imino]methyl}amino) phenyl]ethyl}-1,3-thiazol-5-yl)acrylate (65.4 mg), 1N-NaOH (0.543 ml) and dioxane (1 ml) were combined at 0 °C, and the mixture was stirred at 60 °C for 2 hr. The mixture was adjusted to pH=2 with 1N-HCl, and the organic solvent was evaporated *in vacuo.* The residual aqueous solution was extracted with AcOEt:THF (10:1). The organic layer was washed with water and brine, dried over MgSO₄, and concentrated *in vacuo* to give crude (2E)-3-(2-(acetylamino)-4-{2-[4-({(Z)-[(tert-butoxycarbonyl)amino]-[(tert-butoxycarbonyl)imino]methyl}amino)phenyl]ethyl}-1,3-thiazol-5-yl)acrylic acid as a yellow solid, that was used as crude in the next reaction.
MS: 574.19 (M+H)+

### Step 12

Di-tert-butyl ((Z)-{[4-(2-{2-(acetylamino)-5-[(lE)-3-(dimethylamino)-3-oxo-1-propen-1-yl]-1,3-thiazol-4-yl}ethyl)phenyl]amino}methylidene)biscarbamate was prepared from the compound of Step 11 of Production Example 20 in a manner similar to Step 4 of Production Example 12.
¹H-NMR (400MHz, CDCl₃) δ (ppm) : 1.5 (9H, s), 1.53 (9H, s), 2.26 (3H, s), 2.85 - 3.21 (10H, m), 6.49 (1H, d, J = 14.6Hz), 7.11 (2H, d, J = 8.4Hz), 7.45 (2H, d, J = 8.4Hz), 7.71 (1H, d, J = 15Hz), 9.15 (1H, brs), 10.25 (1H, s), 11.63 (1H, s).
MS: 601.0 (M+H)+, 623.2 (M+Na)+

### Step 13

The title compound was prepared from the compound of Step 12 of Production Example 20 in a manner similar to Step 15 of Production Example 2.
¹H-NMR (400MHz, DMSO-d₆) δ (ppm) : 2.17 (3H, s), 2.85 - 3.03 (7H, m), 3.08 (3H, s), 6.56 (1H, d, J = 15H), 7.14 (2H, d, J = 8.4Hz), 7.25 (2H, d, J = 8.4Hz), 7.35 (4H, s), 7.39 (1H, d, J = 15Hz), 9.71 (1H, s), 12.36 (1H, s).
MS: 401.2 (M+H)+, 423.3 (M+Na)+ Free

### Production Example 21: Synthesis of N-{4-[2-(2-amino-1H-benzimidazol-7-yl)ethyl]-1,3-thiazol-2-yl}acetamide

### Step 1

To NaBH₄ (678 mg) in THF (10 ml) at 0 °C under N₂ was added 2,3-dinitrobenzoic acid (2 g) in THF (4 ml) dropwise over 15 min, then added boron trifluoride diethyl ether complex (3.23 ml) dropwise over 30 min. The reaction mixture was stireed for 2 hr at 20 °C, and then the reaction was quenched with 1N-HCl (40 ml), and the mixture was extracted with ethyl acetate (20 mlx3). The combined organic layers were washed with water and brine, dried over MgSO₄, filtered and evaporated to give 2,3-dinitrobenzylalcohol (2.52 g) as a yellow solid, and that was used as crude in the next reaction.
¹H-NMR (200MHz, CDCl₃) δ (ppm) : 4.81 (2H, s), 7.73 (1H, t, J = 8Hz), 8.02 (1H, d, J = 8Hz), 8.09 (1H, d, J = 8Hz).

### Step 2

Crude 2,3-dinitrobenzylalcohol (2.52 g) was added slowly to 48 % hydrobromic acid (66 ml), and the mixture was stirred for 6 h at 90 °C. The reaction mixture was cooled to 20 °C, diluted with water (60 ml) and extracted with tert-butyl methyl ether (40 mlx3). The combined organic layers were washed with water and aq. NaHCO₃ solution, dried over Na₂SO₄, filtered and evaporated to give 1-bromomethyl-2,3-dinitrobenzene (2.34 g) as a yellow solid.
¹H-NMR (200MHz, CDCl₃) δ (ppm) : 4.48 (2H, s), 7.71 (1H, t, J = 8Hz), 7.9 (1H, dd, J = 1.3, 8Hz), 8.14 (1H, dd, J = 1.5, 8Hz).

### Step 3

To a solution of 1-bromomethyl-2,3-dinitrobenzene (2.29 g) in acetone (60 g) was added triphenylphosphine (2.31 g), and the mixture was refluxed for 3 hr (bath temp.=70 °C). The reaction mixture was cooled to 20 °C, the resulting precipitate was collected by filtration and washed with diethyl ether to give (2,3-dinitrobenzyl)triphenylphosphonium bromide as a yellow solid.
¹H-NMR (200MHz, DMSO-d₆): 5.29 (2H, d, J = 15Hz), 7.47 - 8.01 (17H, m), 8.3 (1H, d, J = 8Hz).

MS: 443.2 (M-Br-)+

### Step 4

(2,3-Dinitrobenzyl)triphenylphosphonium bromide (615 mg) and DMF (2 ml) were combined under N₂ atmosphere, and then potassium tert-butoxide (145 mg) was added to the suspension at 0 °C. The reaction mixture was stirred at 0 °C for 10 min, and N-(4-formyl-1,3-thiazol-2-yl)acetamide (200 mg) was added to the mixture at 0 °C. The reaction mixture was stirred at 20 °C for 2 hr. To the reaction mixture was added ethyl acetate (50 ml), and the mixture was washed with water (20 mlx3) and brine. The organic layer was dried over magnesium sulfate and evaporated to give crude brown oil (750 mg). The crude oil was purified by flash column chromatography over silica gel with CHCl₃ / AcOEt (1:1) as an eluent to give N-{4-[(Z)-2-(2,3-dinitrophenyl)vinyl]-1,3-thiazol-2-yl}acetamide (135.6 mg) as orange foam.
¹H-NMR (400MHz, DMSO-d₆) δ (ppm): 2.09 (3H, s), 6.45 (1H, d, J = 12.2Hz), 6.83 (1H, d, J = 12.3Hz), 7.12 (1H, s), 7.79 (1H, dd, J = 8, 8Hz), 7.89 (1H, d, J = 7.5Hz), 8.27 (1H, dd, J = 1, 8.1Hz), 11.8 (1H, s).MS: 335.0 (M+H)+, 357.1 (M+Na)+

### Step 5

N-{4-[2-(2,3-Diaminophenyl)ethyl]-1,3-thiazol-2-yl}acetamide was prepared from the compound of Step 4 of Production Example 21 in a manner similar to Step 3 of Production Example 16.
¹H-NMR (400MHz, DMSO-d₆) δ (ppm): 2.11 (3H, s), 2.7 - 2.86 (4H, m), 4.18 (2H, s), 4.41 (2H, s), 6.28 - 6.36 (2H, m), 6.41 (1H, dd, J = 2.2, 7Hz), 6.77 (1H, s), 12.07 (1H, s).
MS: 277.09 (M+H)+

### Step 6

To a suspension of N-{4-[2-(2,3-diaminophenyl)ethyl]-1,3-thiazol-2-yl}acetamide (21.6 mg) in MeOH (0.2 ml) was added cyanogen bromide (12.4 mg), and then the mixture was stirred at 20 °C for 14 hr. To the reaction mixture was added aq. 1N-NaOH (0.117 ml), and the mixture was concentrated *in vacuo.* To the residue was added CHCl₃:MeOH=10:1 (10 ml), and the insoluble material was removed by filtration. The filtrate was purified by preparative NH-silica gel thin-layer chromatography with chloroform / methanol (10:1) as an eluent to give a solid (16.4 mg). The solid was washed with CH₂Cl₂ to give N-{4-[2-(2-amino-1H-benzimidazol-7-yl)ethyl]-1,3-thiazol-2-yl}acetamide (15.4 mg) as an off-white solid.
¹H-NMR (400MHz, DMSO-d₆) δ (ppm) : 2.11 (3H, s), 2.85 - 3 (2H, m), 3 - 3.15 (2H, m), 5.81 - 6.21 (2H, m), 6.59 - 6.85 (3H, m), 6.9 - 6.98 (1H, m), 10.56 - 10.96 (1H, m), 12.07 (1H, s).
MS: 302.2 (M+H)+, 324.1 (M+Na)+

### Production Example 22: Synthesis of N-{4-[3-(4-{[amino(imino)methyl]amino}phenyl)propyl}-1,3-thiazol-2-yl}acetamide hydrochloride

### Step 1

N-{4-[3-(4-Nitrophenyl)propyl]-1,3-thiazol-2-yl}acetamide (100 mg) and 10 % palladium on carbon (50 % wet) (98.2 mg) in methanol (2 ml), tetrahydrofuran (2 ml) and acetic acid (0.3 ml) were stirred under 3 atm hydrogen atmosphere at 20 °C for 5 hours. The reaction mixture was filtered through a celite pad, and the filtrate was concentrated *in vacuo.* The residue was dissolved in ethyl acetate. The organic solution was washed with saturated sodium hydrogen carbonate solution and saturated sodium chloride solution, dried over anhydrous magnesium sulfate, and concentrated *in vacuo* to give N-{4-[3-(4-aminophenyl)propyl]-1,3-thiazol-2-yl}acetamide (94.3 mg) as pale yellow oil, that was used as crude in the next reaction.
MS: 276.21 (M+H)+

### Step 2

Di-tert-butyl {(Z)-[(4-{3-[2-(acetylamino)-1,3-thiazol-4-yl]propyl}phenyl)amino]methylidene}biscarbamate was prepared from the compound of Step 1 of Production Example 22 in a manner similar to Step 4 of Production Example 16.
¹H-NMR (200MHz, CDCl₃) δ (ppm) : 1.5 (9H, s), 1.53 (9H, s), 1.86 - 2.07 (2H, m), 2.22 (3H, s), 2.62 (2H, t, J = 8Hz), 2.66 (2H, t, J = 8Hz), 6.53 (1H, s), 7.12 (2H, d, J = 8.4Hz), 7.48 (2H, d, J = 8.4Hz), 10.26 (1H, s), 11.64 (1H, s).
MS: 518.2 (M+H)+, 540.3 (M+Na)+

### Step 3

The title compound was prepared from the compound of Step 2 of Production Example 22 in a manner similar to Step 15 of Production Example 2.
¹H-NMR (400MHz, DMSO-d₆) δ (ppm) : 1.86 - 1.98 (2H, m), 2.11 (3H, s), 2.57 - 2.65 (4H, m), 6.75 (1H, s), 7.15 (2H, d, J = 8.3Hz), 7.27 (2H, d, J = 8.3Hz), 7.41 (4H, s), 9.82 (1H, s), 12.03 (1H, s).
MS: 318.3 (M+H)+ free

### Production Example 23: Synthesis of N-(4-{3-[4-({[amino(imino)methyl]amino}methyl)phenyl]propyl}-1,3-thiazol-2-yl)acetamide hydrochloride

### Step 1

To a solution of methyl 4-(4-oxopentyl)benzoate (6.395 g) in MeOH (64 ml) was added Br₂ (1.35 ml), and the mixture was stirred for 2 hr at 20 °C. To the reaction mixture were added thiourea (2.21 g) and K₂CO₃ (10 g), the mixture was stirrd for 2 hr. at 50 °C, and then cooled to 20 °C, and CHCl₃ (256 ml) was added. The resulting precipitate was removed by filtration. The filtrate was evaporated, and to the residue was added CHCl₃ (200 ml), and the insoluble material was removed by filtration, and the filtrate was evaporated *in vacuo* to give crude brown oil. The crude oil was purified by flash column chromatography over silica gel with CH₂Cl₂ / MeOH (100: 0-100:2) as an eluent to give methyl 4-[3-(2-amino-1,3-thiazol-4-yl)propyl]benzoate (1.68 g) as brown oil.
¹H-NMR (200MHz, CDCl₃) δ (ppm): 1.89 - 2.09 (2H, m), 2.57 (2H, t, J = 7.6Hz), 2.71 (2H, t, J = 7.6Hz), 3.9 (3H, s), 5.19 (2H, brs), 6.08 (1H, s), 7.26 (2H, d, J = 8.2Hz), 7.95 (2H, d, J = 8.2Hz).
MS: 277.14 (M+H)+

### Step 2

Methyl 4-[3-(2-amino-1,3-thiazol-4-yl)propyl]benzoate (1.68 g) was dissolved in CH₂Cl₂ (16.8 ml) under N₂ atmosphere. Then, pyridine (1.57 ml) and AcCl (0.692 ml) were added dropwise to the solution at 0 °C. The reaction mixture was stirred at 20 °C for 30 min. The organic solution was washed with 1N-HCl, water and brine, then dried over MgSO₄, and concentrated *in vacuo* to give crude brown oil (2.52 g, 130 %). The crude oil was purified by flash column chromatography over silica gel with CHCl₃ / MeOH (100:0-100:2) as an eluent to give methyl 4-{3-[2-(acetylamino)-1,3-thiazol-4-yl]propyl}benzoate (1.974 g) as a pale yellow solid.
¹H-NMR (200MHz, CDCl₃) δ (ppm) : 1. 92 - 2.11 (2H, m), 2.27 (3H, s), 2.68 (2H, t, J = 7.3Hz), 2.71 (2H, t, J = 7.3Hz), 3.91 (3H, s), 6.52 (1H, s), 7.25 (2H, d, J = 8Hz), 7.96 (2H, d, J = 8Hz).
MS: 319.11 (M+H)+

### Step 3

To a stirred solution of methyl 4-{3-[2-(acetylamino)-1,3-thiazol-4-yl]propyl}benzoate (1.968 g) in dry THF (40 ml) was added dropwise diisobutylaluminum hydride, 1.0 M solution in toluene (18.5 ml) over 10 min at -78 °C under N₂ atmosphere. The reaction mixture was stirred at -78 °C for 10 min, at 20 °C for 2 hr, and then the reaction was quenched with MeOH. 1N-HCl was added to the mixture and the mixture was extracted with AcOEt. The organic layer was washed with brine, dried over MgSO₄, and concentrated *in vacuo* to give crude yellow oil (1.36 g). The crude oil was purified by flash column chromatography over silica gel with CHCl₃ / AcOEt (100:0-1:1) as an eluent to give N-(4-{3-[4-(hydroxymethyl)phenyl]propyl}-1,3-thiazol-2-yl)acetamide (523 mg) as a yellow solid.
¹H-NMR (200MHz, CDCl₃) δ (ppm): 1.99 (2H, quintet, J = 7.7Hz), 2.13 (3H, s), 2.65 (4H, t, J = 7.5Hz), 4.66 (2H, s), 6.54 (1H, s), 7.15 (2H, d, J = 8Hz), 7.27 (2H, d, J = 8Hz).
MS: 291.3 (M+H)+, 313.1 (M+Na)+

### Step 4

N-(4-{3-[4-(Hydroxymethyl)phenyl]propyl}-1,3-thiazol-2-yl)acetamide (100 mg), CH₂Cl₂ (1 ml) and DMF (1 ml) were combined under N₂ atmosphere, and then Et₃N (60.0 µl) and methanesulfonyl chloride (30.7 µl) were added to the suspension at 0 °C. The reaction mixture was stirred at 20 °C for 16 hr. CHCl₃ and water were added to the mixture. The organic layer was washed with brine, dried over MgSO₄, and concentrated *in vacuo* to give crude N-(4-{3-[4-(chloromethyl)phenyl]propyl}-1,3-thiazol-2-yl)acetamide as oil (MS: 309.03 (M+H)+). To the crude N-(4-{3-[4-(chloromethyl)phenyl]propyl}-1,3-thiazol-2-yl)acetamide in DMF (1 ml) was added phthalimide potassium salt (63.7 mg) and the mixture was stirred at 50 °C for 7 h, then water was added to the reaction mixture, the mixture was extracted with ethyl acetate, and the extract was washed with brine, dried over magnesium sulfate and evaporated to give crude N-[4-(3-{4-[(1,3-dioxo-1,3-dihydro-2H-isoindol-2-yl)methyl]phenyl}propyl)-1,3-thiazol-2-yl]acetamide (147.7 mg), that was used as crude in the next reaction.
¹H-NMR (200MHz, CDCl₃) δ (ppm): 1 .84 - 2.06 (2H, m), 2.25 (3H, s), 2.62 (2H, t, J = 7.5Hz), 2.66 (2H, t, J = 7.5Hz), 4.81 (2H, s), 6.51 (1H, s), 7.12 (2H, d, J = 8Hz), 7.35 (2H, d, J = 8Hz), 7.63 - 7.93 (4H, m) .
MS: 420.2 (M+H)+, 442.1 (M+Na)+

### Step 5

To a solution of N-[4-(3-{4-[(1,3-dioxo-1,3-dihydro-2H-isoindol-2-yl)methyl]phenyl}propyl)-1,3-thiazol-2-yl]acetamide (140 mg) in acetonitrile (1.4 ml) was added hydrazine monohydrate (162 µl), and the mixture was stirred at 50 °C for 30 min. Volatiles were evaporated. To the mixture was added chloroform (1 ml) and the insoluble material was removed by filtration to give N-(4-{3-[4-(aminomethyl)phenyl]propyl}-1,3-thiazol-2-yl)acetamide as crude pale yellow foam (103.4 mg), that was used as crude in the next reaction.
MS: 290.10 (M+H)+

### Step 6

Di-tert-butyl {(Z)-[(4-{3-[2-(acetylamino)-1,3-thiazol-4-yl]propyl}benzyl)amino]methylidene}biscarbamate was prepared from the compound of Step 5 of Production Example 23 in a manner similar to Step 4 of Production Example 16.
¹H-NMR (400MHz, CDCl₃) δ (ppm): 1.48 (9H, s), 1.52 (9H, s), 1.97 - 2.07 (2H, m), 2.3 (3H, s), 2.59 - 2.74 (4H, m), 4.6 (2H, d, J = 5.1Hz), 6.55 (1H, s), 7.16 (2H, d, J = 8.4Hz), 7.23 (2H, d, J = 8Hz), 8.56 (1H, s), 10.23 (1H, brs), 11.54 (1H, s).
MS: 532.3 (M+H)+

### Step 7

The title compound was prepared from the compound of Step 6 of Production Example 23 in a manner similar to Step 15 of Production Example 2.
¹H-NMR (400MHz, DMSO-d₆) δ (ppm): 1.85 - 1.95 (2H, m), 2.11 (3H, s), 2.54 - 2.63 (4H, m), 4.34 (2H, d, J = 6.2Hz), 6.75 (1H, s), 7.22 (4H, s), 7.32 (4H, brs), 8.08 (1H, t, J = 5.9Hz), 12.04 (1H, s).
MS: 332.2 (M+H) + Free

### Production Example 24: Synthesis of N-(4-{3-[4-(2-{[amino(imino)methyl]amino}ethyl)phenyl]propyl}-1,3-thiazol-2-yl)acetamide

### Step 1

To a solution of N-(4-{3-[4-(hydroxymethyl)phenyl] propyl}-1,3-thiazol-2-yl)acetamide (423 mg) in chloroform (10 ml) and MeOH (0.6 ml) was added MnO₂ (3.80 g) at 20 °C under N₂ atmosphere, and the mixture was stirred for 2 days. The reaction mixture was filtered through a celite pad. The filtrate was evaporated to give N-{4-[3-(4-formylphenyl)propyl]-1,3-thiazol-2-yl}acetamide (409.4 mg) as a pale yellow solid, that was used as crude in the next reaction.
MS: 289.04 (M+H)+

### Step 2

To a suspension of N-{4-[3-(4-formylphenyl)propyl]-1,3-thiazol-2-yl}acetamide (409.4 mg) in chloroform (8 ml) was added (carbethoxymethylene)triphenylphosphorane (989 mg) at 20 °C, and the mixture was stirred for 1 hr. The reaction mixture was evaporated. The residue was purified by column chromatography over silica gel with hexane / ethyl acetate (1:1-1:2) as an eluent to give ethyl (2E)-3-(4-{3-[2-(acetylamino)-1,3-thiazol-4-yl]propyl}phenyl)acrylate (463.9 mg) as a pale yellow solid.
¹H-NMR (400MHz, DMSO-d₆) δ (ppm): 1.26 (3H, t, J = 7.1Hz), 1.87 - 1.98 (2H, m), 2.11 (3H, s), 2.54 - 2.68 (4H, m), 4.18 (2H, q, J = 7.1Hz), 6.58 (1H, d, J = 15.7Hz), 6.76 (1H, s), 7.25 (2H, d, J = 8Hz), 7.58 - 7.68 (3H, m), 12.04 (1H, s).
MS: 359.2 (M+H)+

### Step 3

Ethyl (2E)-3-(4-{3-[2-(acetylamino)-1,3-thiazol-4-yl]propyl}phenyl)acrylate (100 mg), MeOH (2 ml), THF (2 ml) and then 10 % Pd/C (50 % wet) (97.6 mg) were combined under N₂ atmosphere. The mixture was stirred at 20°C for 1 hr under H₂ atmosphere (3 atm). The reaction mixture was filtered through a celite pad, and the filtrate was concentrated *in vacuo* to give ethyl 3-(4-{3-[2-(acetylamino)-1,3-thiazol-4-yl]propyl}phenyl)propanoate (109.8 mg) as colorless oil, that was used as crude in the next reaction.
¹H-NMR (400MHz, DMSO-d₆) δ (ppm): 1.14 (3H, t, J = 7.1Hz), 1.83 - 1.95 (2H, m), 2.1 (3H, s), 2.52 - 2.62 (6H, m), 2.76 - 2.84 (2H, m), 4.03 (2H, q, J = 7.1Hz), 6.74 (1H, s), 7.09 (2H, d, J = 8.4Hz), 7.13 (2H, d, J = 8Hz), 12.03 (1H, s).
MS: 361.3 (M+H)+

### Step 4

To a solution of ethyl 3-(4-{3-[2-(acetylamino)-1,3-thiazol-4-yl]propyl}phenyl)propanoate (95.8 mg) in dioxane (958 µl) was added 1N-NaOH (664.394 µl) at 0 °C, then the mixture was stirred for 30 min at 20°C. Volatiles were evaporated *in vacuo.* The residue was dissolved in water (20 ml) and washed with AcOEt (20 ml). The aqueous layer was adjusted to pH=2, and the resulting precipitate was collected by filtration to give 3-(4-{3-[2-(acetylamino)-1,3-thiazol-4-yl]propyl}phenyl)propanoic acid (79.3 mg) as a white solid.
¹H-NMR (200MHz, DMSO-d₆) δ (ppm): 1 .77 - 2 (2H, m), 2.1 (3H, s), 2.43 - 2.65 (6H, m), 2.78 (2H, t, J = 7.3Hz), 6.75 (1H, s), 7.09 (2H, d, J = 8.5Hz), 7.14 (2H, d, J = 8.5Hz), 12.03 (2H, s).
MS: 333.3 (M+H)+, 355.1 (M+Na)+

### Step 5

tert-Butyl [2-(4-{3-[2-(acetylamino)-1,3-thiazol-4-yl]propyl}phenyl)ethyl]carbamate was prepared from the compound of Step 4 of Production Example 24 in a manner similar to Step 13 of Production Example 2.
¹H-NMR (400MHz, DMSO-d₆) δ (ppm): 1.36 (9H, s), 1.84 - 1.95 (2H, m), 2.10 (3H, s), 2.53 - 2.69 (6H, m), 3. 06 - 3.15 (2H, m), 6.74 (1H, s), 6.86 (1H, t, J = 5.7Hz), 7.1 (4H, s), 12.02 (1H, s).
MS: 404.2 (M+H)+, 426.2 (M+Na)+

### Step 6

tert-Butyl [2-(4-{3-[2-(acetylamino)-1,3-thiazol-4-yl]propyl}phenyl)ethyl]carbamate (36.9 mg) and 4N HCl in dioxane (1 ml) were combined under N₂ atmosphere. The reaction mixture was stirred at 25 °C for 4 hr. Volatiles were evaporated *in vacuo* to give a white solid. To the solid in DMF (0.9 ml) were added N,N-diisopropylethylamine (60.524 µl) and N,N'-bis(tert-butoxycarbonyl)-1H-pyrazole-1-carboxamidine (85.136 mg), and the mixture was stirred for 48 hr at 25 °C. Volatiles were evaporated, and the residue was purified by preparative silica gel thin-layer chromatography with chloroform / ethyl acetate (2:1) as an eluent to give di-tert-butyl ((Z)-{[2-(4-{3-[2-(acetylamino)-1,3-thiazol-4-yl]propyl}phenyl)ethyl]amino}methylidene)biscarbamate (44.6 mg) as colorless oil.
¹H-NMR (400MHz, CDCl₃) δ (ppm): 1.47 (9H, s), 1.5 (9H, s), 1.93 - 2.04 (2H, m), 2.25 (3H, s), 2.56 - 2.71 (4H, m), 2.84 (2H, t, J = 7.3Hz), 3.6 - 3.7 (2H, m), 6.53 (1H, s), 7.1 (2H, d, J = 8.4Hz), 7.13 (2H, d, J = 8.4Hz), 8.33 - 8.42 (1H, m), 11.47 (1H, brs).
MS: 546.46 (M+H)+

### Step 7

Di-tert-butyl ((Z)-{[2-(4-{3-[2-(acetylamino)-1,3-thiazol-4-yl]propyl}phenyl)ethyl]amino}methylidene) biscarbamate (39.1 mg) and 4N HCl in dioxane (2 ml) were combined under N₂ atmosphere. The reaction mixture was stirred at 20 °C for 14 hr. The solvent was removed *in vacuo.* The residue was dissolved in water, the solution was adjusted to pH=9 with aq. saturated NaHCO₃, extracted with AcOEt:THF=1:1, the extract was dried over MgSO₄ and evaporated to give a colorless oil (25 mg). The oil was purified by preparative NH-silica gel thin-layer chromatography with chloroform / methanol (4:1) as an eluent to give N-(4-{3-[4-(2-{[amino(imino)methyl]amino}ethyl)phenyl]propyl}-1,3-thiazol-2-yl)acetamide (7.7 mg) as a colorless oil.
¹H-NMR (400MHz, CDCl₃:CD₃OD=1:1) δ (ppm): 1.98 (2H, quintet, J = 7.6Hz), 2.23 (3H, s), 2.57 - 2.73 (4H, m), 2.86 (2H, t, J = 7.1Hz), 3.41 (2H, t, J = 7.1Hz), 6.55 (1H, s), 7.15 (4H, s).
MS: 346.38 (M+H)+

### Production Example 25: Synthesis of N-(4-{2-[4-(2-{[amino(imino)methyl]amino}ethyl)phenyl]ethyl}-1,3-thiazol-2-yl)acetamide

Di-tert-butyl ((Z)-{[2-(4-{2-[2-(acetylamino)-1,3-thiazol-4-yl]ethyl}phenyl)ethyl]amino}methylidene) biscarbamate (443.1 mg) and 4N HCl in 1,4-dioxane solution (10 ml) were combined under N₂ atmosphere. The reaction mixture was stirred at 20 °C for 14 hours. The solvent was removed *in vacuo.* The residue was dissolved in water. The solution was made basic (pH=9) by saturated sodium hydrogen carbonate aqeous solution. The precipitate was filtered *in vacuo* to give N-(4-{2-[4-(2-{[amino(imino)methyl]amino}ethyl)phenyl]ethyl}-1,3-thiazol-2-yl)acetamide (667.7 mg) as a white solid.
¹H-NMR (400MHz, DMSO-d₆) δ (ppm): 2.02 (3H, brs), 2.64 - 2.96 (6H, m), 3.13 - 3.5 (2H, m), 6.55 (1H, brs), 7.14 (4H, s), 8.32 (4H, brs).
MS: 332.2(M+H)+

### Production Example 26: Synthesis of 3-(4-{2-[2-(acetylamino)-1,3-thiazol-4-yl]ethyl}phenyl)-N-[amino(imino)methyl] propanamide

### Step 1

To a solution of methyl 3-(4-{2-[2-(acetylamino)-1,3-thiazol-4-yl]ethyl}phenyl)propanoate (2.73 g) in dioxane (27 ml) was added 1N-NaOH (22.5 ml) at 0 °C; and then the mixture was stirred for 30 min at 20 °C. Volatiles were evaporated *in vacuo.* The residue was dissolved in water (20 ml) and washed with AcOEt (20 ml). The aqueous layer was adjusted to pH=2, and the resulting precipitate was collected by filtration to give 3-(4-{2-[2-(acetylamino)-1,3-thiazol-4-yl]ethyl}phenyl)propanoic acid (2.672 g) as a white solid. ¹H-NMR (200MHz, DMSO-d₆) δ (ppm): 2.11 (3H, s), 2.43 - 2.56 (2H, m), 2.77 (2H, t, J = 7.8Hz), 2.87 (4H, s), 6.73 (1H, s), 7.11 (4H, s), 12.09 (2H, s).
MS: 319.09 (M+H)+

### Step 2

To a solution of 3-(4-{2-[2-(acetylamino)-1,3-thiazol-4-yl]ethyl}phenyl)propanoic acid (100 mg) in DMF (2 ml) was added 1,1'-carbonyldiimidazole (56 mg). The mixture was stirred at 50 °C for 2 hr. To the mixture was added a mixture of guanidine hydrochloride (150 mg), DMF (1 ml) and 28 % sodium methoxide in MeOH (0.307 ml) at 20 °C. The reaction mixture was stirred at 20 °C for 15 hr, and concentrated *in vacuo.* The residue was dissolved in water, and the solution was adjusted to pH=8 with 1N-HCl. The precipitate was collected. The solid was washed with water, CH₃CN and AcOEt to give 3-(4-{2-[2-(acetylamino)-1,3-thiazol-4-yl]ethyl}phenyl)-N-[amino(imino)methyl]propanamide (101.3 mg) as a white powder.
¹H-NMR (200MHz, DMSO-d₆) δ (ppm): 2.11 (3H, s), 2.36 (2H, t, J = 7.8Hz), 2.76 (2H, t, J = 8Hz), 2.86 (4H, s), 6.73 (1H, s), 6.95 (4H, brs), 7.08 (4H, s), 11.99 (1H, brs).
MS: 360.3 (M+H)+

### Production Example 27: Synthesis of N-[4-(2-{4-[2-(4,5-dihydro-1H-imidazol-2-ylamino)ethyl]phenyl}ethyl)-1,3-thiazol-2-yl]acetamide

### Step 1

To a solution of phthalimide potassium salt (46.2 g) in N,N-dimethylformamide (300 ml) was added dropwise 4-(2-bromoethyl)benzaldehyde (40.92 g) in N,N-dimethylformamide (50 ml) at 60 °C and the mixture was stirred for 2 hr. The reaction mixture was cooled to 20 °C, and then poured into water (1.5 L). The resulting presipitate was collected by filtration to give a yellow solid. The solid was dissolved in chloroform (250 ml) and the insoluble material was removed by filtration. The filtrate was concentrated in *vacuo.* The residue was washed with diethyl ether and collected by filtration to give 4-[2-(1,3-dioxo-1,3-dihydro-2H-isoindol-2-yl)ethyl]benzaldehyde (19.65 g) as an off-white solid.
¹H-NMR (200MHz, DMSO-d₆) δ (ppm): 3.04 (2H, t, J = 7Hz), 3.88 (2H, t, J = 7Hz), 7.44 (2H, d, J = 8.5Hz), 7.75 - 7.89 (6H, m), 9.94 (1H, s).
MS: 280.1(M+H)+

### Step 2

{[2-(Acetylamino)-1,3-thiazol-4-yl]methyl}(triphenyl)phosphonium chloride (46.9 mg) and DMF (190 ml) were combined under N₂ atmosphere, and then potassium tert-butoxide (12.8 g) was added to the suspension at 0 °C. The reaction mixture was stirred at 0 °C for 15 min, and 4-[2-(1,3-dioxo-1,3-dihydro-2H-isoindol-2-yl)ethyl]benzaldehyde (19.28 g) was added to the mixture at 0 °C. The reaction mixture was stirred at 20°C for 2 hr. The reaction mixture was poured into water, and the resulting precipitate was collected by filtration to give a crude brown solid. The brown solid was washed with CH₃CN:IPE=1:1, then CH₃CN to give N-[4-((E)-2-{4-[2-(1,3-dioxo-1,3-dihydro-2H-isoindol-2-yl)ethyl]phenyl}vinyl)-1,3-thiazol-2-yl]acetamide (24.88 g) as a beige solid. ¹H-NMR (400MHz, DMSO-d₆) δ (ppm): 2.15 (3H, s), 2.94 (2H, t, J = 7.1Hz), 3.83 (2H, t, J = 7.1Hz), 7.12 (1H, d, J = 15.8Hz), 7.14 (1H, d, J = 15.8Hz), 7.16 (1H, s), 7.19 (2H, d, J = 8Hz), 7.44 (2H, d, J = 8.4Hz), 7.8 - 7.88 (4H, m), 12.22 (1H, s).MS: 418.1 (M+H)+

### Step 3

N-[4-((E)-2-{4-[2-(1,3-Dioxo-1,3-dihydro-2H-isoindol-2-yl)ethyl]phenyl}vinyl)-1,3-thiazol-2-yl]acetamide (24.88 g), DMF (800 ml), MeOH (80 ml), AcOH (8 ml) and then 10 % Pd/C (50 % wet) (24.4 g) were combined under N₂ atmosphere. The mixture was stirred at 20°C for 16 hr under H₂ atmosphere (4 atm). The catalyst was renewed every 4 hr in a period of reaction time. The reaction mixture was filtered through a celite pad, and the filtrate was concentrated *in vacuo.* The residue was washed with IPE (200 ml) and purified by flash column chromatography over silica gel with CHCl₃ / AcOEt (1:1) as an eluent. The fractions containing the object compound were combined, and evaporated under reduced pressure. The residue was washed with IPE (200 ml) and collected by filtration to give N-[4-(2-{4-[2-(1,3-dioxo-1,3-dihydro-2H-isoindol-2-yl)ethyl]phenyl}ethyl)-1,3-thiazol-2-yl]acetamide (17.86 g) as an off-white solid.
¹H-NMR (400MHz, DMSO-d₆) δ (ppm) : 2.11 (3H, s), 2.78 - 2.92 (6H, m), 3.79 (2H, t, J = 7.3Hz), 6.66 (1H, s), 7.08 (2H, d, J = 8.9Hz), 7.1 (2H, d, J = 8.8Hz), 7.79 - 7.89 (4H, m), 12.08 (1H, s).
MS: 420.2 (M+H)+, 442.1 (M+Na)+

### Step 4

To a solution of N-[4-(2-{4-[2-(1,3-dioxo-1,3-dihydro-2H-isoindol-2-yl)ethyl]phenyl}ethyl)-1,3-thiazol-2-yl]acetamide (2.06 g) in acetonitrile (20 ml) was added hydrazine monohydrate (2.38 ml), and the mixture was stirred at 50 °C for 2 hr. Volatiles were evaporated. To the mixture was added chloroform (10 ml), and the insoluble material was removed by filtration to give crude pale yellow foam (1.49 g, 104.8 %). The crude oil was purified by flash column chromatography over NH₂-silica gel with CHCl₃ / MeOH (10:0-10:2) as an eluent to give N-(4-{2-[4-(2-aminoethyl)phenyl]ethyl}-1,3-thiazol-2-yl)acetamide (1.1304 g) as a pale yellow solid.
¹H-NMR (400MHz, DMSO-d₆) δ (ppm): 2.11 (3H, s), 2.58 (2H, t, J = 7.3Hz), 2.72 (2H, t, J = 7.1Hz), 2.81 - 2.94 (4H, m), 6.73 (1H, s), 7.08 (2H, d, J = 8.4Hz), 7.11 (2H, d, J = 8.4Hz).
MS: 290.2 (M+H)+

### Step 5

N-(4-{2-[4-(2-Aminoethyl)phenyl]ethyl}-1,3-thiazol-2-yl)acetamide (150 mg), ethyl 2-(methylthio)-4,5-dihydro-1H-imidazole-1-carboxylate (117.1 mg), AcOH (0.6 ml) and EtOH (3 ml) were combined under N₂ atmosphere, and the mixture was refluxed for 24 hr. After cooled to 20 °C, the reaction mixture was diluted with AcOEt. The solution was made basic by aq. sat. NaHCO₃ solution. The resulting precepitate was collected by filtration to give N-[4-(2-{4-[2-(4,5-dihydro-1H-imidazol-2-ylamino)ethyl]phenyl}ethyl)-1,3-thiazol-2-yl]acetamide (111 mg) as a white solid.
¹H-NMR (400MHz, DMSO-d₆) δ (ppm): 2.11 (3H, s), 2.76 (2H, t, J = 7.5Hz), 2.81 - 2.94 (4H, m), 3.37 (2H, t, J = 7.1Hz), 3.12-3.82 (4H, m), 6.71 (1H, s), 7.12 (2H, d, J = 8.4Hz), 7.15 (2H, d, J = 8.4Hz), 9.84 (1H, brs).
MS: 358.3 (M+H)+

### Production Example 28: Synthesis of N-[4-(2-{4-[2-(ethanimidoylamino)ethyl]phenyl}ethyl)-1,3-thiazol-2-yl]acetamide

N-(4-{2-[4-(2-Aminoethyl)phenyl]ethyl}-1,3-thiazol-2-yl)acetamide(100 mg), methyl ethanimidothioate hydroiodide (150.0 mg) and MeOH (2 ml) were combined, and the mixture was stirred for 3 hr at 20 °C. Volatiles were evaporated in *vacuo.* The residue was purified by flash column chromatography over NH-silica gel with CHCl₃ / MeOH (5:0-5:1) as an eluent to give a white foam. The foam was triturated with IPE to give N-[4-(2-{4-[2-(ethanimidoylamino)ethyl]-phenyl}ethyl)-1,3-thiazol-2-yl]acetamide (97.4 mg) as a white solid.
¹H-NMR (400MHz, DMSO-d₆) δ (ppm): 1.83 (3H, s), 2.09 (3H, s), 2.72 (2H, t, J = 7.5Hz), 2.8 - 2.93 (4H, m), 3.18 (2H, t, J = 7.5Hz), 6.69 (1H, s), 7.11 (2H, d, J = 8.4Hz), 7.13 (2H, d, J = 8.4Hz).
MS: 331.3 (M+H)+

### Production Example 29: Synthesis of N-[4-(2-{4-[2-(4,5-dihydro-1,3-thiazol-2-ylamino)ethyl]phenyl}ethyl)-1,3-thiazol-2-yl]acetamide

N-(4-{2-[4-(2-Aminoethyl)phenyl]ethyl}-1,3-thiazol-2-yl)acetamide (100 mg), 2-(methylthio)-4,5-dihydro-1,3-thiazole (92.1 mg), conc. HCl (0.04 ml) and 2-methoxyethanol (1.5 ml) were combined under N₂ atmosphere, and the mixture was stirred at 120 °C for 24 hr. After cooled to 20 °C, the reaction mixture was dissolved in water (0.5 ml), and the solution was adjusted to pH=10 by aq. K₂CO₃, and resulting precipitate was collected by filtration to give N-[4-(2-{4-[2-(4,5-dihydro-1,3-thiazol-2-ylamino)ethyl]phenyl}ethyl)-1,3-thiazol-2-yl]acetamide (111.47 mg) as a beige solid.
¹H-NMR (400MHz, DMSO-d₆) δ (ppm): 2.11 (3H, s), 2.73 (2H, t, J = 7.5Hz), 2.82 - 2.93 (4H, m), 3.21 (2H, t, J = 7.1Hz), 3.31 (2H, t, J = 7.5Hz), 3.82 (2H, t, J = 7.3Hz), 6.73 (1H, s), 7.09 (2H, d, J = 8.8Hz), 7.12 (2H, d, J = 8.8Hz).
MS: 375.2 (M+H)+

### Production Example 30: Synthesis of N-(4-{2-[4-(2-{[imino(methylamino)methyl]amino}ethyl)phenyl]ethyl}-1,3-thiazol-2-yl)acetamide

### Step 1

N-(4-{2-[4-(2-Aminoethyl)phenyl]ethyl}-1,3-thiazol-2-yl)acetamide (200 mg) was dissolved in acetone (2.8 ml) under N₂ atmosphere, and then isothiocyanic acid benzoyl ester (93.2 µl) was added dropwise to the solution at 0 °C. The reaction mixture was stirred at 20 °C for 1 hr. Water was added to the mixture, and the precipitate was filtered *in vacuo* to give a crude yellow solid (237.9 mg, 76 %). The crude solid was purified by flash column chromatography over silica gel with CHCl₃ / MeOH (100:0-100:2) as an eluent to give N-({[2-(4-{2-[2-(acetylamino)-1,3-thiazol-4-yl]ethyl}phenyl)ethyl]amino}carbonothioyl)benzamide (152.8 mg) as a pale yellow solid.
¹H-NMR (400MHz, DMSO-d₆) δ (ppm): 2.11 (3H, s), 2.81 - 2.96 (6H, m), 3.82 (2H, q, J = 6.7Hz), 6.72 (1H, s), 7.15 (2H, d, J = 8Hz), 7.19 (2H, d, J = 8Hz), 7.51 (2H, t, J = 7.7Hz), 7.63 (1H, t, J = 7.5Hz), 7.91 (2H, d, J = 7.7Hz), 10.93 (1H, t, J = 5.3Hz), 11.34 (1H, s), 12.09 (1H, s).
MS: 453.3 (M+H)+, 475.1 (M+Na)+

### Step 2

To a suspension of N-({[2-(4-{2-[2-(acetylamino)-1,3-thiazol-4-yl]ethyl}phenyl)ethyl]amino}carbonothioyl)benzamide (140 mg) in EtOH (1.5 ml) was added dropwise aq. 6N NaOH (154.7 µl) at 0 °C. The reaction mixture was stirred for 2 hr at 20 °C, and neutralized with 1N-HCl at 0 °C. The precipitate was collected by filtration to give N-{4-[2-(4-{2-[(aminocarbonothioyl)amino]ethyl}phenyl)ethyl]-1,3-thiazol-2-yl}acetamide (98.6 mg) as a pale yellow solid.
¹H-NMR (400MHz, DMSO-d₆) δ (ppm): 2.11 (3H, s), 2.68 - 2.79 (2H, m), 2.82 - 2.95 (4H, m), 3.12 - 3.65 (2H, m), 6.74 (1H, s), 6.96 (2H, brs), 7.14 (4H, s), 7.46 - 7.71 (1H, m), 12.08 (1H, s).
MS: 349.1 (M+H)+, 371.2 (M+Na)+

### Step 3

To a solution of N-{4-[2-(4-{2-[(aminocarbonothioyl)amino]ethyl}phenyl)ethyl]-1,3-thiazol-2-yl}acetamide (50 mg) in MeOH (0.5 ml) was added iodomethane (10.72 µl), and the mixture was refluxed for 5 hr. Volatiles were evaporated and the residue was solidified with AcOEt. The resulting precipitate was collected by filtration to give methyl N-[2-(4-{2-[2-(acetylamino)-1,3-thiazol-4-yl]ethyl}phenyl)ethyl]imidothiocarbamate hydroiodide (71.2 mg) as a colorless oil.
MS: 363.27 (M+H)+ Free

### Step 4

Methyl N-[2-(4-{2-[2-(acetylamino)-1,3-thiazol-4-yl]ethyl}phenyl)ethyl]imidothiocarbamate hydroiodide (71.2 mg) and 2M methylamine solution in THF (726 µl) were combined at 0 °C under N₂ atmosphere. The reaction mixture was stirred for 24 hr at 20 °C. Volatiles were evaporated. The residue was dissolved in water and MeOH, and the solution was adjusted to pH=8 with aq. saturated NaHCO₃. MeOH was evaporated, and then water and AcOEt were added. The resulting precipitate was collected by filtration to give N-(4-{2-[4-(2-{[imino(methylamino)methyl]amino}ethyl)-phenyl]ethyl}-1,3-thiazol-2-yl)acetamide (42.8 mg) as a white solid.
¹H-NMR (400MHz, DMSO-d₆) δ (ppm): 2.02 (3H, s), 2.64 - 2.79 (5H, m), 2.79 - 2.92 (4H, m), 3.32 (2H, t, J = 7.7Hz), 6.53 (1H, s), 7.13 (4H, s), 8.86 (3H, bs).
MS: 346.3 (M+H)+

### Production Example 31: Synthesis of 2-[4-(2-{2-(acetylamino)-5-[4-(methylsulfonyl)benzyl]-1,3-thiazol-4-yl}ethyl)phenyl]-N-[amino(imino)methyl]acetamide

### Step 1

To the solution of [4-(carboxymethyl)benzyl](triphenyl) phosphonium bromide (1.76 g) in DMF (30 ml) was added potassium tert-butoxide (1.10 g) at 0 °C, and the mixture was stirred for 30 min at same temperature. To the solution was added N-{4-formyl-5-[4-(methylthio)benzyl]-1,3-thiazol-2-yl}acetamide (1.00 g) (prepared in Step 6 of Production Example 11), and the mixture was stirred for 3hr at 20°C. The reaction mixture was poured in ice-0.1N-HCl, and the resulting precipitate was collected by filtration. The obtained powder was dissolved with 1N-NaOH (40 ml) and washed with AcOEt. The aqueous layer was adjusted to pH=3 with conc. hydrochloric acid, and the resulting precipitate was collected by filtration to give a mixture of [4-((E)-2-{2-(acetylamino)-5-[4-(methylthio)benzyl]-1,3-thiazol-4-yl}vinyl)phenyl]acetic acid and [4-((Z)-2-{2-(acetylamino)-5-[4-(methylthio)benzyl]-1,3-thiazol-4-yl}vinyl)phenyl]acetic acid (E:Z=1:2) (1.18 g) as yellow powder.
¹H-NMR (400MHz, DMSO-d₆) δ (ppm): 2.07 (3Hx2/3, s), 2.11 (3Hx1/3, s), 2.43 (3Hx2/3, s), 2.43 (3Hx1/3, s), 3.54 (2Hx2/3, s), 3.58 (2Hx1/3, s), 3.89 (2Hx2/3, s), 4.23 (2Hx1/3, s), 6.53 (1Hx2/3, d, J = 12.4Hz), 6.63 (1Hx2/3, d, J = 12.4Hz), 7.08 (2Hx2/3, d, J = 8.4Hz), 7.16 (2Hx2/3, d, J = 8Hz), 7.17 (2Hx2/3, d, J = 8.4Hz), 7.22 (4Hx1/3, s), 7.23 (1Hx1/3, d, J = 15.4Hz), 7.26 (2Hx1/3, d, J = 8.1Hz), 7.26 (2Hx2/3, d, J = 8.1Hz), 7.38 (1Hx1/3, d, J = 15.7Hz), 7.56 (2Hx1/3, d, J = 8Hz), 11.98 (1Hx2/3, s), 12.11 (1Hx1/3, s), 12.41 (1H, brs).
MS: 439.0 (M+H)+, 461.0 (M+Na)+

### Step 2

[4-((E)-2-{2-(Acetylamino)-5-[4-(methylsulfonyl)benzyl]-1,3-thiazol-4-yl}vinyl)phenyl]acetic acid and [4-((Z)-2-{2-(acetylamino)-5-[4-(methylsulfonyl)benzyl]-1,3-thiazol-4-yl}vinyl)phenyl]acetic acid were prepared from the mixture of Step 1 of Production Example 31 in a manner similar to Step 11 of Production Example 1.
¹H-NMR (400MHz, DMSO-d₆) δ (ppm): 2.08 (3Hx2/5, s), 2.12 (3Hx3/5, s), 3.18 (3H, s), 3.54 (2Hx2/5, s), 3.58 (2Hx3/5, s), 4.09 (2Hx2/5, s), 4.42 (2Hx3/5, s), 6.54 (1Hx2/5, d, J = 12.4Hz), 6.64 (1Hx2/5, d, J = 12.4Hz), 7.15 (2Hx2/5, d, J = 8Hz), 7.25 (1Hx3/5, d, J = 14.3Hz), 7.26 (2Hx2/5+2Hx3/5, d, J = 8Hz), 7.41 (2Hx2/5, d, J = 8.4Hz), 7.42 (1Hx3/5, d, J = 15.7Hz), 7.54 (2Hx3/5, d, J = 8.4Hz), 7.58 (2Hx3/5, d, J = 8Hz), 7.83 (2Hx2/5, d, J = 8.4Hz), 7.87 (2Hx3/5, d, J = 8Hz), 12.04 (1Hx2/5, s), 12.17 (1Hx3/5, s), 12.4 (1H, s).
MS: 471:1 (M+H)+, 493.0 (M+Na)+

### Step 3

A mixture of [4-((E)-2-{2-(acetylamino)-5-[4-(methylsulfonyl)benzyl]-1,3-thiazol-4-yl}vinyl)phenyl]acetic acid and [4-((Z)-2-{2-(acetylamino)-5-[4-(methylsulfonyl)benzyl]-1,3-thiazol-4-yl}vinyl)phenyl]acetic acid (1.010 g), MeOH (15 ml), THF (60 ml) and then 10 % Pd/C (50 % wet) (1.01 g) were combined under N₂ atmosphere. The mixture was stirred at 20 °C for 12 hr under H₂ atmosphere (4 atm). The reaction mixture was filtered through a celite pad, and to the filtrate was added 10 % Pd/C (1.01 g) under N₂ atmosphere. The mixture was stirred at 20 °C for 12 hr under H₂ atmosphere (4 atm). The reaction mixture was filtered through a celite pad, and the filtrate was concentrated *in vacuo.* The residue was washed with Et₂O and collected by filtration to give [4-(2-{2-(acetylamino)-5-[4-(methylsulfonyl)benzyl]-1,3-thiazol-4-yl}ethyl)phenyl]acetic acid (532.8 mg) as an off-white solid.
¹H-NMR (400MHz, DMSO-d₆) δ (ppm): 2.08 (3H, s), 2.85 (4H, s), 3.18 (3H, s), 3.52 (2H, s), 4.01 (2H, s), 7.08 (2H, d, J = 8Hz), 7.15 (2H, d, J = 8Hz), 7.32 (2H, d, J = 8.4Hz), 7.81 (2H, d, J = 8.4Hz), 12.06 (2H, brs).
MS: 473.2 (M+H)+, 495.1 (M+Na)+

### Step 4

To a solution of [4-(2-{2-(acetylamino)-5-[4-(methylsulfonyl)benzyl]-1,3-thiazol-4-yl}ethyl)phenyl]acetic acid (100 mg) in DMF (1 ml) was added 1,1'-carbonyldiimidazole (37.7 mg). The mixture was stirred at 50 °C for 2 hr. To the mixture was added a mixture of guanidine hydrochloride (101.1 mg) and 28% sodium methoxide solution in MeOH (0.216 ml) at 25 °C. The reaction mixture was stirred at 25 °C for 15 hr, and concentrated *in vacuo.* The residue was dissolved in water, and the solution was adjusted to pH=8 with 1N-HC1, and extracted with AcOEt/MeOH. The organic layer was dried over MgSO₄ and evaporated. The residue was purified by flash column chromatography over silica gel with CHCl₃ / MeOH (20:0-20:1) as an eluent, and triturated with diethyl ether to give 2-[4-(2-{2-(acetylamino)-5-[4-(methylsulfonyl)benzyl]-1,3-thiazol-4-yl}ethyl)phenyl]-N-[amino(imino)methyl]acetamide (49.2 mg) as a pale yellow solid.
¹H-NMR (400MHz, DMSO-d₆) δ (ppm): 2.07 (3H, s), 2.83 (4H, s), 3.18 (3H, s), 3.36 (2H, s), 4 (2H, s), 6.57 (2H, brs), 7.03 (2H, d, J = 8Hz), 7.12 (2H, d, J = 8Hz), 7.3 (2H, d, J = 8Hz), 7.81 (2H, d, J = 8Hz), 7.82 (2H, brs), 12.03 (1H, brs).
MS: 514.2 (M+H)+

The compounds according to the present invention useful as VAP-1 inhibitors are listed in the following tables.

| **No.** | **Structure** | **No.** | **Structure** |
|---|---|---|---|
| 1 | 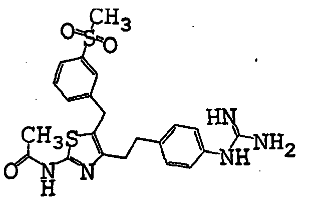 | 6 | 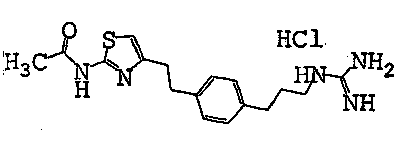 |
| 2 | 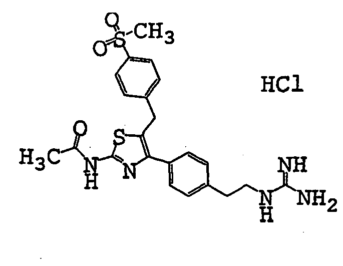 | 7 | 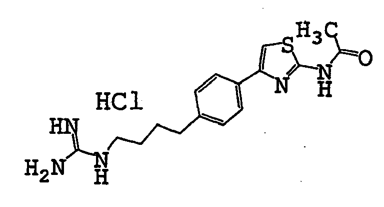 |
| 3 | 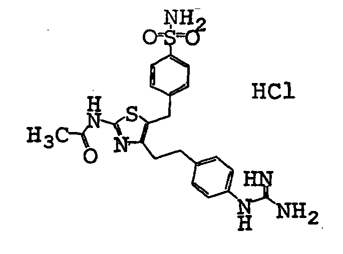 | 8 | 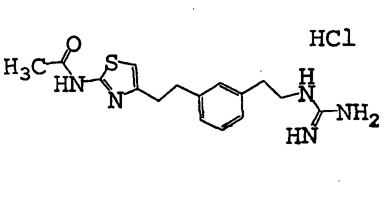 |
| 4 | 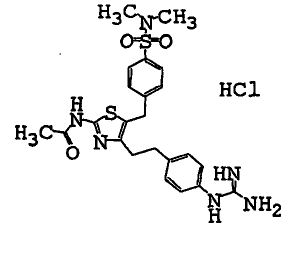 | 9 | 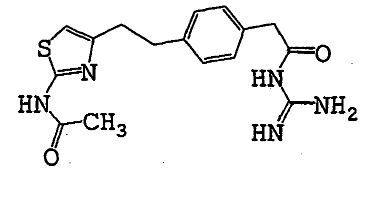 |
| 5 | 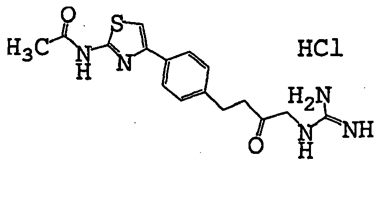 | 10 | 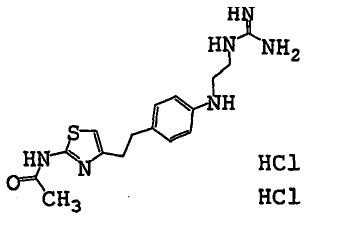 |
| 11 | 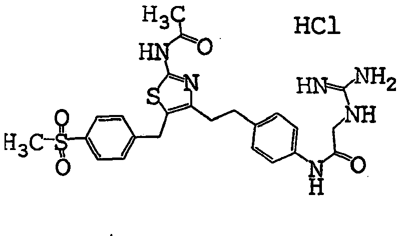 | 16 | 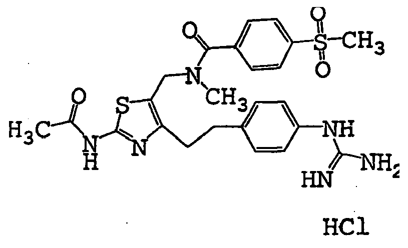 |
| 12 | 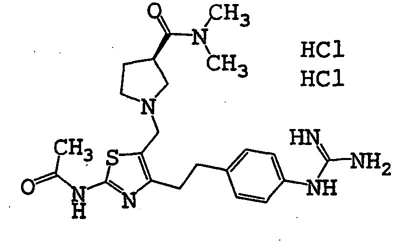 | 17 | 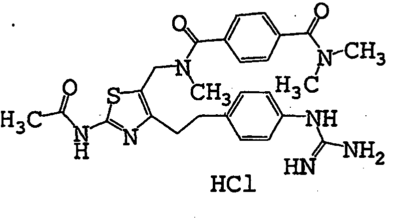 |
| 13 | 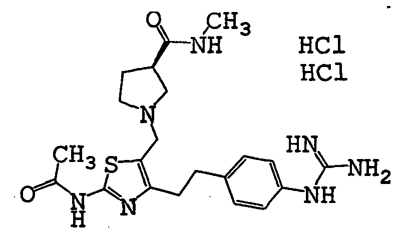 | 18 | 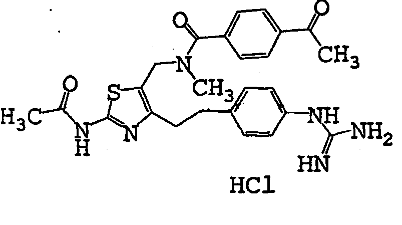 |
| 14 | 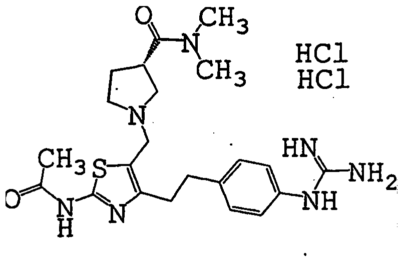 | 19 | 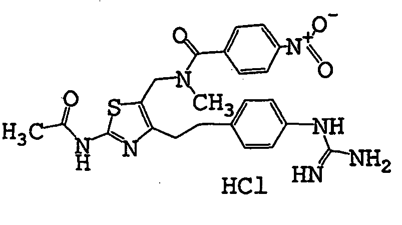 |
| 15 | 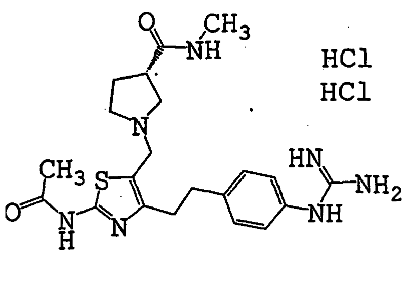 | 20 | 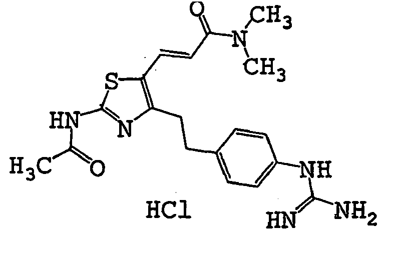 |
| 21 | 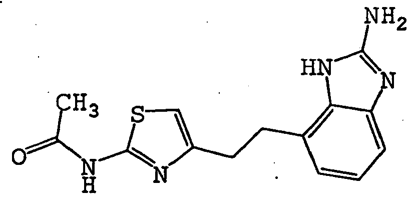 | 26 | 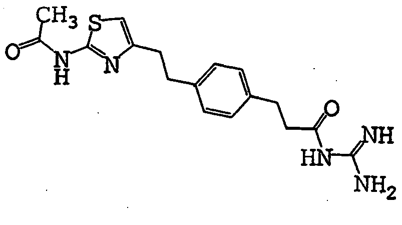 |
| 22 | 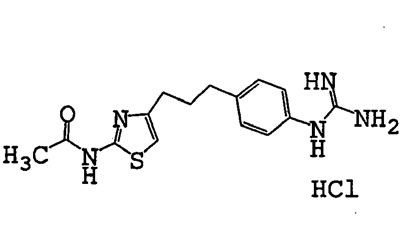 | 27 | 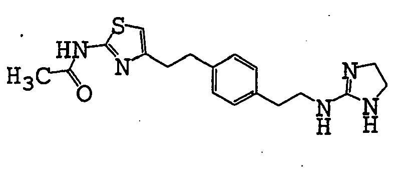 |
| 23 | 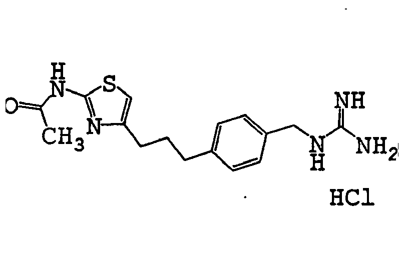 | 28 | 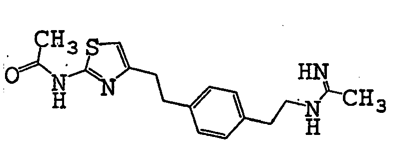 |
| 24 | 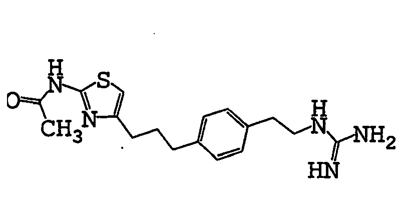 | 29 | 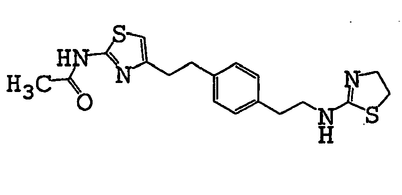 |
| 25 | 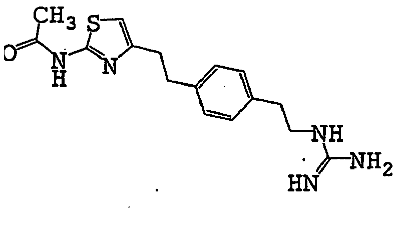 | 30 | 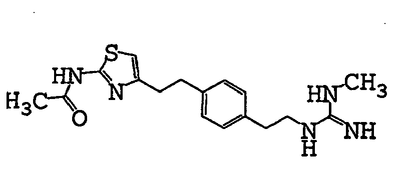 |
| 31 | 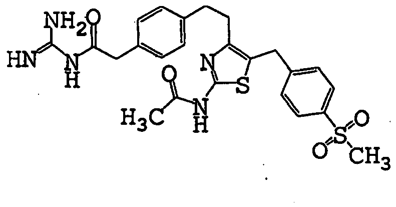 | | |

### Example 1

### Inhibitory effect of the present compound on VAP-1 enzyme (SSAO) activity in human.

VAP-1 enzyme (SSAO) activity in human plasma was determined by a radiochemical-enzyme assay using ¹⁴C-benzylamine as an artificial substrate. The enzyme suspension prepared from blood plasma was pre-incubated with a control compound (Reference Example 1) in 96-well microplate at room temperature for 30 min. The enzyme suspension was then incubated with ¹⁴C-benzylamine (2x10⁻⁵ mol/l final concentration) in a final volume of 50 µl at 37°C for 1 hour. The enzyme reaction was terminated by adding 2 mol/l (50 µl) citric acid. The oxidized products were directly extracted into a 200 µl toluene scintillator, and its radioactivity was measured by a scintillation spectrometer. Inhibition activity was expressed as IC₅₀ (µmol/l) value.

Test compounds (i.e., the present compounds) inhibited the enzyme activity of human plasma SSAO in comparison with the control compound as shown in Table 1.

**Table 1. Inhibitory effect (IC₅₀ values, µM) of test compounds**

| Compounds | Human plasma SSAO |
|---|---|
| Reference Example 1 (control) | 0.033 |
| Production Example 1 | 0.016 |
| Production Example 3 | 0.0045 |
| Production Example 7 | 0.015 |
| Production Example 9 | 0.0026 |
| Production Example 12 | 0.019 |
| Production Example 14 | 0.014 |
| Production Example 16 | 0.012 |
| Production Example 19 | 0.032 |
| Production Example 25 | 0.0057 |

### INDUSTRIAL APPLICABILITY

The present invention provides a compound of the formula (I), (II), (III) or (IV), or a pharmaceutically acceptable salt thereof useful as a VAP-1 inhibitor, a pharmaceutical composition, a method for preventing or treating a VAP-1 associated disease, especially macular edema such as diabetic macular edema and non-diabetic macular edema, which method comprises administering to a patient in need thereof a VAP-1 inhibitor in an amount sufficient to treat the patient suffering from the VAP-1 associated disease, and the like.

This application is based on a provisional patent application No. 2004904196 filed in Australia.

## Claims

1. A compound of the formula (I), (II), (III) or (IV): 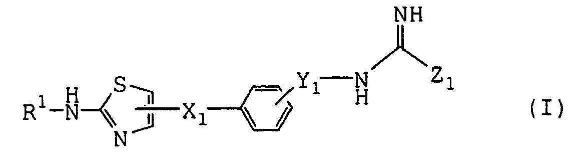 wherein
R¹ is alkylcarbonyl;
X₁ is a bond or (C₁-C₆) alkylene;
Y₁ is a bond, (C₁-C₆) alkylene, -CH₂-CO-, -CH₂-CH₂-CO-, -CH₂-CH₂-CO-CH₂- or -NH-CH₂-CH₂-; and
Z₁ is -NH₂, -NH((C₁-C₆) alkyl) or (C₁-C₆) alkyl;
provided that
when X₁ is ethylene, then Y₁ should be (C₂-C₆) alkylene, -CH₂-CO-, -CH₂-CH₂-CO-, -CH₂-CH₂-CO-CH₂- or -NH-CH₂-CH₂-,
when X₁ is a bond, then Y₁ should be a bond, methylene, C₃-C₆ alkylene, -CH₂-CO-, -CH₂-CH₂-CO-, -CH₂-CH₂-CO-CH₂- or -NH-CH₂-CH₂-, and
when R¹ is acetyl, X₁ is ethylene, Y₁ is ethylene and Z₁ is -NH₂, then Y₁ should be attached to *ortho* or *meta* position of the phenyl group; 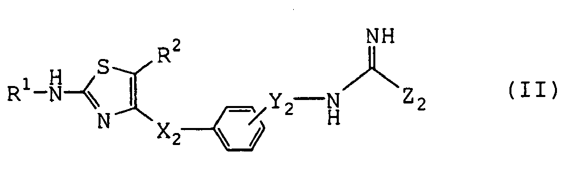 wherein
R¹ is alkylcarbonyl;
R² is 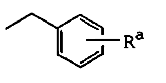 wherein R^{a} is ((C₁-C₆) alkyl)sulfonyl, aminosulfonyl or di((C₁-C₆) alkyl)aminosulfonyl, 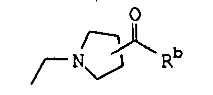 wherein R^{b} is mono- or di-((C₁-C₆) alkyl)amino, 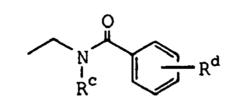 wherein R^{c} is (C₁-C₆) alkyl and R^{d} is ((C₁-C₆) alkyl)sulfonyl, di((C₁-C₆)alkyl)aminocarbonyl, alkylcarbonyl or nitro, or
-CH=CH-CO-di((C₁-C₆) alkyl)amino;
X₂ is a bond or (C₁-C₆) alkylene;
Y₂ is a bond, (C₁-C₆) alkylene, -CH₂-CO- or -NH-CO-CH₂-; and
Z₂ is -NH₂;
provided that
when R¹ is acetyl, X₂ is ethylene, Y₂ is a bond and Z₂ is -NH₂, then R² should not be 3-(methanesulfonyl)benzyl, 4-(methanesulfonyl)benzyl, 4-(ethanesulfonyl)benzyl and 2-(dimethylaminocarbonyl)pyrrolidin-1-ylmethyl; 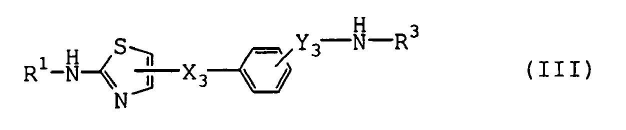 wherein
R¹ is alkylcarbonyl;
R³ is 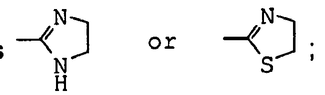 X₃ is (C₁-C₆) alkylene; and
Y₃ is (C₁-C₆) alkylene; 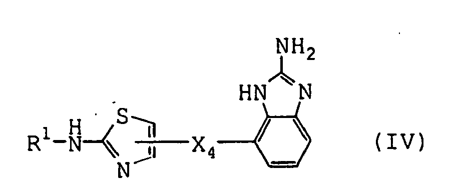 wherein
R¹ is alkylcarbonyl; and
X₄ is (C₁-C₆) alkylene;
or a pharmaceutically acceptable salt thereof.

2. The compound of claim 1, wherein R¹ is acetyl, or a pharmaceutically acceptable salt thereof.

3. The compound of claim 1, wherein Z₁ is -NH₂, or a pharmaceutically acceptable salt thereof.

4. The compound of claim 1, wherein the compound is N-{4-[2-(4-{[amino(imino)methyl]amino}phenyl)ethyl]-5-[4-(aminosulfonyl)benzyl]-1,3-thiazol-2-yl}acetamide, N-{4-[4-(4-{[amino(imino)methyl]amino}butyl)phenyl]-1,3-thiazol-2-yl}acetamide, 2-(4-{2-[2-(acetylamino)-1,3-thiazol-4-yl]ethyl}phenyl)-N-[amino(imino)methyl]acetamide, (3R)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, (3S)-1-({2-(acetylamino)-4-[2-(4-([amino(imino)methyl]amino)-phenyl)ethyl]-1,3-thiazol-5-yl}methyl)-N,N-dimethyl-3-pyrrolidinecarboxamide, N-({2-(acetylamino)-4-[2-(4-{[amino(imino)methyl]amino}-phenyl)ethyl]-1,3-thiazol-5-yl}methyl)-N-methyl-4-(methylsulfonyl)benzamide, or N-({2-(acetylamino)-4-[2-(4-{[amino(imino)methyl]amino}-phenyl)ethyl]-1,3-thiazol-5-yl}methyl)-N-methyl-4-nitrobenzamide, or a pharmaceutically acceptable salt thereof.

5. The compound of claim 1 to 4 or a pharmaceutically acceptable salt thereof for use as a medicament.

6. A pharmaceutical composition, which comprises, as an active ingredient, the compound of claim 1 to 4 or a pharmaceutically acceptable salt thereof.

7. A use of the compound of claim 1 to 4 or a pharmaceutically acceptable salt thereof for preparing a medicament as a VAP-1 inhibitor.

8. The use of claim 7, wherein the compound is N-{4-[2-(4-{[amino(imino)methyl]amino}phenyl)ethyl]-5-[4-(aminosulfonyl)benzyl]-1,3-thiazol-2-yl}acetamide, N-{4-[4-(4-{[amino(imino)methyl]amino}butyl)phenyl]-1,3-thiazol-2-yl}acetamide, 2-(4-{2-[2-(acetylamino)-1,3-thiazol-4-yl]ethyl}phenyl)-N-[amino(imino)methyl]acetamide, (3R)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, (3S)-1-({2-(acetylamino)-4-[2-(4-{[amino(imino)methyl]amino}-phenyl)ethyl]-1,3-thiazol-5-yl}methyl)-N,N-dimethyl-3-pyrrolidinecarboxamide, N-({2-(acetylamino)-4-[2-(4-{[amino(imino)methyl]amino}-phenyl)ethyl]-1,3-thiazol-5-yl}methyl)-N-methyl-4-(methylsulfonyl)benzamide, or N-({2-(acetylamino)-4-[2-(4-{[amino(imino)methyl]amino}-phenyl)ethyl]-1,3-thiazol-5-yl}methyl)-N-methyl-4-nitrobenzamide, or a pharmaceutically acceptable salt thereof.

9. A use of the compound of claim 1 to 4 or a pharmaceutically acceptable salt thereof for preparing a medicament for the prophylaxis or treatment of a VAP-1 associated disease.

10. The use of claim 9, wherein said VAP-1 associated disease is selected from the group consisting of cirrhosis, essential stabilized hypertension, diabetes, arthrosis, endothelium damage (in diabetes, atherosclerosis and hypertension), a cardiovascular disorder associated with diabetes and uremia, pain associated with gout and arthritis, retinopathy (in diabetes patients), an (connective tissue) inflammatory disease or condition (rheumatoid arthritis, ankylosing spondylitis, psoriatic arthritis and osteoarthritis or degenerative joint disease, Reiter's syndrome, Sjögren's syndrome, Behçet's syndrome, relapsing polychondritis, systemic lupus erythematosus, discoid lupus erythematosus, systemic sclerosis, eosinophilic fasciitis, polymyositis, dermatomyositis, polymyalgia rheumatica, vasculitis, temporal arteritis, polyarteritis nodosa, Wegener's granulomatosis, mixed connective tissue disease, and juvenile rheumatoid arthritis), a gastrointestinal inflammatory disease or condition [Crohn's disease, ulcerative colitis, irritable bowel syndrome (spastic colon), fibrotic conditions of the liver, inflammation of the oral mucosa (stomatitis), and recurrent aphtous stomatitis], a central nervous system inflammatory disease or condition (multiple sclerosis, Alzheimer's disease, and ischemia-reperfusion injury associated with ischemic stroke), a pulmonary inflammatory disease or condition (asthma, adult respiratory distress syndrome, and chronic obstructive pulmonary disease), a (chronic) skin inflammatory disease or condition (psoriasis, allergic lesions, lichen planus, pityriasis rosea, contact dermatitis, atopic dermatitis, and pityriasis rubra pilaris), a disease related to carbohydrate metabolism (diabetes and complications from diabetes) including microvascular and macrovascular disease (atherosclerosis, vascular retinopathies, retinopathy, nephropathy, nephrotic syndrome and neuropathy (polyneuropathy, mononeuropathies and autonomic neuropathy), foot ulcers, joint problems, and increased risk of infection), a disease related to aberrations in adipocyte differentiation or function or smooth muscle cell function (atherosclerosis and obesity), a vascular disease [atheromatous ateriosclerosis, nonatheromatous ateriosclerosis, ischemic heart disease including myocardial infarction and peripheral arterial occlusion, Raynaud's disease and phenomenon, and thromboangiitis obliterans (Buerger's disease)], chronic arthritis, inflammatory bowel diseases, skin dermatoses, diabetes mellitus, SSAO-mediated complication [diabetes (insulin dependent diabetes mellitus (IDDM) and non-insulin dependent diabetes mellitus (NIDDM)) and vascular complication (heart attack, angina, strokes, amputations, blindness and renal failure)], macular edema (diabetic and non-diabetic macular edema), hepatitis and transplantation.

11. The use of claim 10, wherein said VAP-1 associated disease is macular edema.

12. The use of claim 11, wherein said macular edema is diabetic macular edema.

13. The use of claim 11, wherein said macular edema is non-diabetic macular edema.

14. A VAP-1 inhibitor, which comprises the compound of claim 1 or a pharmaceutically acceptable salt thereof.

## Patentansprüche

1. Verbindung der Formel (I), (II), (III) oder (IV): 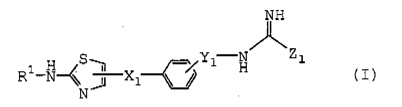 wobei
R¹ Alkylcarbonyl ist;
X₁ eine Bindung oder (C₁-C₆)Alkylen ist;
Y₁ eine Bindung, (C₁-C₆)Alkylen, -CH₂-CO-, -CH₂-CH₂-CO-, -CH₂-CH₂-CO-CH₂- oder -NH-CH₂-CH₂- ist; und
Z₁ -NH₂, -NH((C₁-C₆)Alkyl) oder (C₁-C₆)Alkyl ist;
unter der Voraussetzung, dass
wenn X₁ Ethylen ist, dann Y₁ (C₂-C₆)Alkylen, -CH₂-CO-, -CH₂-CH₂-CO-, -CH₂-CH₂-CO-CH₂- oder -NH-CH₂-CH₂- sein sollte,
wenn X₁ eine Bindung ist, dann Y₁ eine Bindung, Methylen, C₃-C₆ Alkylen, -CH₂-CO-, -CH₂-CH₂-CO-, -CH₂-CH₂-CO-CH₂- oder -NH-CH₂-CH₂- sein sollte, und
wenn R¹ Acetyl ist, X₁ Ethylen ist, Y₁ Ethylen ist und Z₁-NH₂ ist,
dann Y₁ an die *ortho-* oder *meta-P*osition der Phenylgruppe gebunden sein sollte; 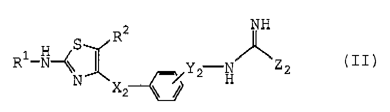 wobei
R¹ Alkylcarbonyl ist;
R² 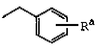 ist,
wobei R^{a} ((C₁-C₆) Alkyl)sulfonyl, Aminosulfonyl oder Di((C₁-C₆)alkyl)aminosulfonyl ist, 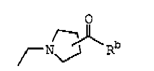 wobei R^{b} Mono- oder Di-((C₁-C₆)alkyl)amino ist, 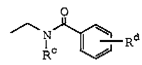 wobei R^{c} (C₁-C₆)Alkyl ist und R^{d} ((C₁-C₆)Alkyl)sulfonyl, Di((C₁-C₆)alkyl)aminocarbonyl, Alkylcarbonyl oder Nitro, oder -CH=CH-CO-Di((C₁-C₆)alkyl)amino ist;
X₂ eine Bindung oder (C₁-C₆)Alkylen ist;
Y₂ eine Bindung, (C₁-C₆) Alkylen, -CH₂-CO- oder -NH-CO-CH₂- ist; und
Z₂ -NH₂ ist;
Vorausgesetzt, dass
wenn R¹ Acetyl ist, X₂ Ethylen ist, Y₂ eine Bindung ist und Z₂ -NH₂ ist, dann R² nicht 3-(Methansulfonyl)benzyl, 4-(Methansulfonyl)benzyl, 4-(Ethansulfonyl)benzyl und 2-(Dimethylaminocarbonyl)pyrrolidin-1-ylmethyl sein sollte; 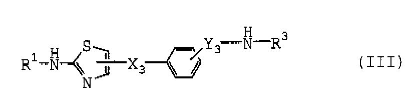 wobei
R¹ Alkylcarbonyl ist; 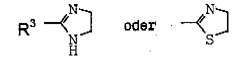 ist;
X₃ (C₁-C₆) Alkylen; und
Y₃ (C₁-C₆) Alkylen ist; 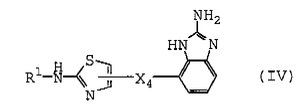 wobei
R¹ Alkylcarbonyl ist; und
X₄ (C₁-C₆)Alkylen ist;
oder ein pharmazeutisch verträgliches Salz davon.

2. Verbindung nach Anspruch 1, wobei R¹ Acetyl ist, oder ein pharmazeutisch verträgliches Salz davon.

3. Verbindung nach Anspruch 1, wobei Z₁ -NH₂ ist, oder ein pharmazeutisch verträgliches Salz davon.

4. Verbindung nach Anspruch 1, wobei die Verbindung ist N-{4-[2-(4-{[Amino(imino)methyl]amino}phenyl)ethyl]-5-[4-(aminosulfonyl)benzyl]-1,3-thiazol-2-yl}acetamid, N-{4-[4-(4-{[Amino(imino)methyl]amino}butyl)phenyl]-1,3-thiazol-2-yl}acetamid, 2-(4-{2-[2-(Acetylamino)-1,3-thiazol-4-yl]ethyl}phenyl)-N-[amino(imino)methyl]acetamid, (3R)-1-({2-(Acetylamino)-4-[2-(4-{[amino(imino)methyl]amino}-phenyl)ethyl]-1,3-thiazol-5-yl}methyl)-N,N-dimethyl-3-pyrrolidincarboxamid, (3S)-1-({2-(Acetylamino)-4-[2-(4-{[amino(imino)methyl]amino}-phenyl)ethyl]-1,3-thiazol-5-yl}methyl)-N,N-dimethyl-3-pyrrolidincarboxamid, N-({2-(Acetylamino)-4-[2-(4-{[amino(imino)methyl]amino}-phenyl)ethyl]-1,3-thiazol-5-yl}methyl)-N-methyl-4-(methylsulfonyl)benzamid, oder N-({2-(acetylamino)-4-[2-(4-{[amino(imino)methyl]amino}-phenyl)ethyl]-1,3-thiazol-5-yl}methyl)-N-methyl-4-nitrobenzamid, oder ein pharmazeutisch verträgliches Salz davon.

5. Verbindung nach Anspruch 1 bis 4 oder pharmazeutisch verträgliches Salz davon zur Verwendung als Medikament.

6. Pharmazeutische Zusammensetzung, welche als aktiven Bestandteil die Verbindung nach Anspruch 1 bis 4 oder ein pharmazeutisch verträgliches Salz davon umfasst.

7. Verwendung der Verbindung nach Anspruch 1 bis 4 oder eines pharmazeutisch verträglichen Salzes davon zur Herstellung eines Medikaments als VAP-1-Inhibitor.

8. Verwendung nach Anspruch 7, wobei die Verbindung N-{4-[2-(4-{[Amino(lmino)methyl]amino}phenyl)ethyl]-5-[4-(aminosulfonyl)benzyl]-1,3-thiazal-2-yl}acetamid, N-{4-[4-(4-{[Amino(imino)methyl]amino}butyl)phenyl]-1,3-thiazol-2-yl}acetamid, 2-(4-{2-[2-(Acetylamino)-1,3-thiazol-4-yl]ethyl}phenyl)-N-[amino(imino)methyl]acetamid, (3R)-1-({2-(Acetylamino)-4-[2-(4-{[amino(imino)methyl]amino}-phenyl)ethyl]-1,3-thiazol-5-yl}methyl)-N,N-dimethyl-3-pyrrolidincarboxamid, (3S)-1-({2-(Acetylamino)-4-[2-(4-{[amino(imino)methyl]amino}-phenyl)ethyl]-1,3-thiazol-5-yl}methyl)-N,N-dimethyl-3-pyrrolidincarboxamid, N-({2-(Acetylamino)-4-[2-(4-{[amino(imino)methyl]amino}-phenyl)ethyl]-1,3-thiazol-5-yl}methyl)-N-methyl-4-(methylsulfonyl)benzamid, oder N-({2-(Acetylamino)-4-[2-(4-{[amino(imino)methyl]amino}-phenyl)ethyl]-1,3-thiazol-5-yl}methyl)-N-methyl-4-nitrobenzamid, oder ein pharmazeutisch verträgliches Salz davon ist.

9. Verwendung der Verbindung nach Anspruch 1 bis 4 oder eines pharmazeutisch verträglichen Salzes davon zur Herstellung eines Medikaments zur Prophylaxe oder Behandlung einer mit VAP-1 zusammenhängenden Erkrankung.

10. Verwendung nach Anspruch 9, wobei die mit VAP-1 verbundene Erkrankung ausgewählt wird aus der Gruppe, die besteht aus Zirrhose, essentieller stabilisierter Hypertension, Diabetes, Arthrose, Endothelschäden (bei Diabetes, Artherosklerose und Hypertension), einer cardiovaskulären Erkrankung, die mit Diabetes und Urämie zusammenhängt, mit Gicht und Arthritis zusammenhängenden Schmerzen, Retinopathie (in Diabetespatienten), entzündlicher (Bindegewebs)-Erkrankung oder -Zustand (rheumatoide Arthritis, ankylosierende Spondylitis, psoriatrische Arthritis und Osteoarthritis oder degenerative Gelenkerkrankung, Reiter -Syndrom, Sjögren- Syndrom, Behçet-Syndrom, rezidivierende Polychondritis, systemischem Lupus erythematosus, discoidem Lupus Erythematosus, systemischer Sclerose, eosinophiler Fasciitis, Polymyositis, Dermatomyositis, Polymyalgia rheumatica, Vasculitis, temporärer Arteritis, Polyarteritis nodosa, Wegener-Granulomatosis, Bindegewebsmischerkrankung und juveniler rheumatoider Arthritis), gastrointestinaler entzündlicher Erkrankung oder-Zustand [Morbus Crohn, ulcerativer Colitis, Reizdarm (spastischer Darm), fibrotische Zustände der Leber, Entzündung der Mundschleimhaut (Stomatitis), und wiederkehrende aphtöse Stomatitis], entzündlicher zentralnervöser Erkrankung oder -Zustand (Multiple Sklerose, Alzheimer-Erkrankung, und Ischämie-Reperfusionserkrankung, die mit ischämischem Schlaganfall zusammenhängt), entzündlicher Lungenerkrankung oder-Zustand (Asthma, ARDS und chronische obstruktive Lungenerkrankung), (chronische) entzündliche Hauterkrankung oder-Zustand (Psoriasis, allergische Läsionen, Lichen planus, Pityriasis rosea, Kontaktdermatitis, atopische Dermatitis, und Pityriasis rubra pilaris), mit Kohlenhydratmetabolismus zusammenhängender Erkrankung (Diabetes and Komplikationen von Diabetes) einschließlich mikrovaskulärer und makrovaskulärer Erkrankungen (Atherosklerose, vaskuläre Retinopathien, Retinopathie, Nephropathie, nephrotisches Syndrom und Neuropathie (Polyneuropathie, Mononeuropathien und autonome Neuropathie), Fußgeschwüre, Gelenkprobleme und erhöhtes Infektionsrisiko), mit Abweichung der Adipocyten Differenzierung oder-funktion oder Glattmuskelzellfunktion zusammenhängende Erkrankung (Atherosklerose und Fettleibigkeit), vaskulärer Erkrankung [atheromatöse Ateriosklerose, nonatheromatöse Ateriosklerose, ischämische Herzerkrankung einschließlich Myocardinfarkt und peripherem Arterienverschluß, Raynaud-Erkrankung und - phänomen, und Thromboangiitis obliterans (Buerger-Erkrankung)], chronischer Arthritis, entzündlicher Darmerkrankung, Hautdermatosen, Diabetes mellitus, SSAO-vermittelter Komplikation [Diabetes (Insulin-abhängiger Diabetes mellitus (IDDM) und nicht-insulin abhängiger Diabetes mellitus (NIDDM)) und vaskuläre Komplikationen (Herzanfall, Angina, Schlaganfall, Amputationen, Erblindung und Nierenversagen)], Makulaödem (diabetisches und nicht-diabetisches Makulaödem), Hepatitis und Transplantation.

11. Verwendung nach Anspruch 10, wobei die mit VAP-1 zusammenhängende Erkrankung Makula-Ödem ist.

12. Verwendung nach Anspruch 11, wobei das Makula-Ödem diabetisches Makular-Ödem ist.

13. Verwendung nach Anspruch 1, wobei das Makula-Ödem ein nicht-diabetisches Makula-Ödem ist.

14. VAP-1 Inhibitor, welcher die Verbindung nach Anspruch 1 oder ein pharmazeutisch verträgliches Salz davon umfasst.

## Revendications

1. composé de la formule (I), (II), (III) ou (IV): 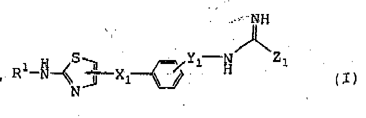 où
R¹ est un groupe alkylcarbonyle ;
X₁ est une liaison ou un groupe alkylène en (C₁-C₆);
Y₁ est une liaison, un groupe alkylène en (C₁-C₆), -CH₂-CO-, -CH₂-CH₂-CO-, -CH₂-CH₂-CO-CH₂- ou -NH-CH₂-CH₂-; et
Z₁ est un groupe -NH₂, -NH(alkyle en (C₁-C₆)) ou alkyle en (C₁-C₆) ;
à la condition que
lorsque X₁ est un groupe éthylène, alors Y₁ doit être un groupe alkylène en (C₁-C₆), -CH₂-CO-, -CH₂-CH₂-CO-, -CH₂-CH₂-CO-CH₂- ou -NH-CH₂-CH₂-,
lorsque X₁ est une liaison, alors Y₁ doit être une liaison, un groupe méthylène, alkylène en (C₁-C₆), -CH₂-CO-, -CH₂-CH₂-CO-, -CH₂-CH₂-CO-CH₂- ou -NH-CH₂-CH₂-, et
lorsque R¹ est un groupe acétyle, X₁ est un groupe éthylène, Y₁ est un groupe éthylène et Z₁ est un groupe -NH₂, alors Y₁ doit être fixé à la position ortho ou méta du groupe phényle ; 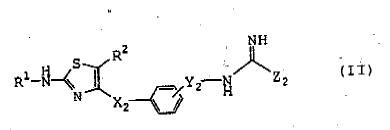 où
R¹ est un groupe alkylcarbonyle ;
R² est 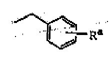 où R^{a} est un groupe (alkyl en (C₁-C₆))sulfonyle, aminosulfonyle ou di (alkyl en (C₁-C₆)aminolulfonyle, 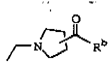 où R^{b} est un groupe mono- ou di-(alkyl en (C₁-C₆))amino, 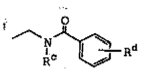 où R^{c} est un groupe alkyle en (C₁-C₆) et R^{d} est un groupe (alkyl en (C₁-C₆))sulfonyle, di(alkyl en (C₁-C₆))aminocarbonyle, alkylcarbonyle ou nitro, ou -CH=CH-CO-di(alkyl en (C₁-C₆))amino;
X₂ est une liaison ou un groupe alkylène en (C₁-C₆) ;
Y₂ est une liaison, un groupe alkylène en (C₁-C₆), -CH₂-CO- ou -NH-CO-CH₂- ; et
Z₂ est -NH₂ ;
à la condition que
lorsque R¹ est un groupe acétyle, X₂ est un groupe éthylène, Y₂ est une liaison et Z₂ est un groupe -NH₂, alors R² ne doit pas être un groupe 3-(méthanesulfonyl)benzyle, 4-(méthanesulfonyl)benzyle, 4-(éthanesulfonyl)benzyle et 2-(diméthylaminocarbonyl) pyrrolidin-1-ylméthyle ; 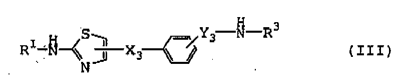 où
R¹ est un groupe alkylcarbonyle ;
R³ est 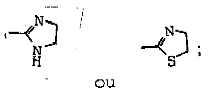 X₃ est alkylène en (C₁-C₆) ; et
Y₃ est alkylène en (C₁-C₆) ; 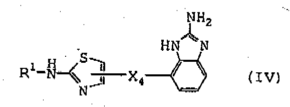 où
R¹ est alkylcarbonyle ; et
X₄ est alkylène en (C₁-C₆) ;
ou un sel pharmaceutiquement acceptable de celui-ci.

2. Composé selon la revendication 1, dans lequel R¹ est un groupe acétyle, ou un sel pharmaceutiquement acceptable de celui-ci.

3. Composé selon la revendication 1, dans lequel Z₁ est un groupe -NH₂, ou un sel pharmaceutiquement acceptable de celui-ci.

4. Composé selon la revendication 1, dans lequel le composé est N-{4-[2-(4-{[amino(imino)méthyl]amino}phényl)éthyl]-5-[4-(aminosulfonyl)benzyl]-1,3-thiazol-2-yl}acétamide, N-{4-[4-(4-{[amino(imino)méthyl]amino}butyl)phényl]-1,3-thiazol-2-yl}acétamide, 2-(4-{2-[2-(acétylamino)-1,3-thiazol-4-yl]éthyl}phényl)-N-[amino(imino)méthyl]acétamide, (3R)-1-({2-(acétylamino)-4-[2-(4-{[amino(imino)méthyl]amino}-phényl)éthyl]-1,3-thiazol-5-yl}méthyl)-N,N-diméthyl-3-pyrrolidinecarboxamide, (3S)-1-({2-(acétylamino)-4-[2-(4-{[amino(imino)méthyl]amino}-phényl)éthyl]-1,3-thiazol-5-yl}méthyl)-N,N-diméthyl-3-pyrrolidinecarboxamide, N-({2-(acétylamino)-4-[2-(4-{[amino(imino)méthyl]amino}-phényl)éthyl]-1,3-thiazol-5-yl}méthyl)-N-méthyl-4-(méthylsulfonyl)benzamide, ou N-({2-(acétylamino)-4-[2-(4-{[amino(imino)méthyl]amino}-phényl)éthyl]-1,3-thiazol-5-yl}méthyl)-N-méthyl-4-nitrobenzamide,
ou un sel pharmaceutiquement acceptable de ceux-ci.

5. Composé selon les revendications 1 à 4 ou sel pharmaceutiquement acceptable de celui-ci destiné à être utilisé comme médicament.

6. Composition pharmaceutique qui comprend, comme ingrédient actif, le composé selon les revendications 1 à 4 ou un sel pharmaceutiquement acceptable de celui-ci.

7. Utilisation du composé selon les revendications 1 à 4 ou d'un sel pharmaceutiquement acceptable de celui-ci pour préparer un médicament comme un inhibiteur de la VAP-1.

8. utilisation selon la revendication 7, dans laquelle le composé est N-{4-[2-(4-{[amino(imino)méthyl]amino}phényl)éthyl]-5-[4-(aminosulfonyl)benzyl]-1,3-thiazol-2-yl}acétamide, N-{4-[4-(4-{[amino(imino)méthyl]amino}butyl)phényl]-1,3-thiazol-2-yl}acétamide, 2-(4-{2-[2-(acétylamino)-1,3-thiazol-4-yl]éthyl}phényl)-N-[amino(imino)méthyl]acétamide, (3R)-1-({2-(acétylamino)-4-[2-(4-{[amino(imino)méthyl]amino}-phényl)éthyl]-1,3-thiazol-5-yl}méthyl)-N,N-diméthyl-3-pyrrolidinecarboxamide, (3S)-1-({2-(acétylamino)-4-[2-(4-{[amino(imino)méthyl]amino}-phényl)éthyl]-1,3-thiazol-5-yl}méthyl)-N,N-diméthyl-3-pyrrolidinecarboxamide, N-({2-(acétylamino)-4-[2-(4-{[amino(imino)méthyl]amino}-phényl)éthyl]-1,3-thiazol-5-yl}méthyl)-N-méthyl-4-(méthylsulfonyl)benzamide, ou N-({2-(acétylamino)-4-[2-(4-{[amino(imino)méthyl]amino}-phényl)éthyl]-1,3-thiazol-5-yl}méthyl)-N-méthyl-4-nitrobenzamide,
ou un sel pharmaceutiquement acceptable de ceux-ci.

9. Utilisation du composé selon les revendications 1 à 4 ou d'un sel pharmaceutiquement acceptable de celui-ci, pour préparer un médicament pour la prophylaxie ou le traitement d'une maladie associée à la VAP-1.

10. Utilisation selon la revendication 9, dans laquelle la maladie associée à la VAP-1 est sélectionnée dans le groupe comprenant la cirrhose, l'hypertension stabilisée essentielle, les diabètes, l'arthrose, l'endommagement endothélial (dans les diabètes, l'athérosclérose et l'hypertension), une maladie cardiovasculaire associée aux diabètes et l'urémie, la douleur associée à la goutte et à l'arthrite, la rétinopathie, (chez les patients diabétiques), une maladie ou un état inflammatoire (du tissu conjonctif) (arthrite rhumatoïde, spondylite ankylosante, arthrite psoriasique et ostéoarthrite ou maladie des articulations dégénérative, syndrome de Reiter, syndrome de Sjögren, syndrome de Behçat, polychondrite récidivante, lupus érythémateux systémique, lupus érythémateux discoïde, sclérose systémique, fasciite éosinophile, polymyosite, dermatomyosite, polymyalgie rhumatismale, vasculite, artérite temporale, périartérite noueuse, granulomatose de Wagener, connectivité mixte, et arthrite rhumatoïde juvénile), une maladie ou un état inflammatoire gastro-intestinale [maladie de Crohn, colite ulcéreuse, syndrome du côlon irritable (côlon spastique), états fibreux du foie, inflammation de la muqueuse orale (stomatite), et stomatite aphteuse récurrente], une maladie ou un état inflammatoire du système nerveux central (sclérose en plaques, maladie d'Alzheimer et ischémie-lésion consécutive à perfusion associée à un accident ischémique cérébral), une maladie ou un état d'inflammation pulmonaire (asthme, syndrome de détresse respiratoire de l'adulte, et maladie pulmonaire obstructive chronique), une maladie ou un état inflammatoire de la peau (chronique) (psoriasis, lésions allergiques, lichen plan, pityriasis rosé, dermatite de contact, dermatite atopique et pityriasis rubra pilaire), une maladie liée au métabolisme des glucides (diabètes et complications des diabètes) incluant la microangiopathie et la macroangiopathie (athérosclérose, rétinopathies vasculaires, rétinopathie, néphropatie, syndrome néphrotique et neuropathie (polyneuropathie, mononeuropathies et neuropathie autonome), ulcères du pied, problèmes d'articulation et risque accru d'infection), une maladie liée aux aberrations dans la différentiation ou la fonction des adipocytes ou la fonction des cellules musculaires lisses (athérosclérose et obésité), une maladie vasculaire [atériosclérose athénomateuse, atériosclérose nonathéromateuse, cardiopathie ischémique incluant l'infarctus du myocarde et l'occlusion artérielle périphérique, la maladie et le phénomène de Raynaud et la thromboangéite oblitérant (maladie de Buerger)], l'arthrite chronique, les maladies intestinales inflammatoires, les dermatoses cutanées, le diabète sucré, la complication médiée par SSAO [diabètes (diabète sucré insuline-dépendant (IDDM) et diabète sucré non insulino-dépendant (NDDM)) et complication vasculaire (attaque cardiaque, angine, accidents cérébrovasculaires, amputations, cécité et maladie rénale)], oedème maculaire (oedème maculaire diabétique et non diabétique), hépatite et transplantation.

11. Utilisation selon la revendication 10, dans laquelle ladite maladie associée à la VAP-1 est un oedème maculaire.

12. Utilisation selon la revendication 11, dans laquelle ledit oedème maculaire est un oedème maculaire diabétique.

13. Utilisation selon la revendication 11, dans laquelle ledit oedème maculaire est un oedème maculaire non diabétique.

14. Inhibiteur de la VAP-1, qui comprend le composé de la revendication 1 ou un sel pharmaceutiquement acceptable de celui-ci.
